# EUROPEAN PATENT APPLICATION

(11) **EP 2 527 334 A1**
(43) Date of publication of application: **28.11.2012**
(21) Application number: 11734755.9
(22) Date of filing: 21.01.2011
(51) Int. Cl.: C07D 307/91, C07D 405/12, C09K 11/06, H01L 51/50

(54) **AROMATIC AMINE DERIVATIVE, AND ORGANIC ELECTROLUMINESCENT ELEMENT COMPRISING SAME**

(30) Priority: 21.01.2010 JP 2010011415; 21.01.2010 JP 2010011414
(71) Applicant: Idemitsu Kosan Co., Ltd., Chiyoda-ku Tokyo 100-8321 (JP)
(72) Inventor: KATO, Tomoki, Sodegaura-shi Chiba 299-0293 (JP)
(74) Representative: Vossius & Partner
(86) International application number: PCT/JP2011/051045
(87) International publication number: WO 2011/090149

(57) **Abstract**

Provided are: an aromatic amine derivative having a terminal substituent selected from a dibenzofuran, a dibenzothiophene, a substituted carbazole, and a substituted fluorene bound to the central skeleton having a specific structure through a nitrogen atom; an organic electroluminescence device, including an organic thin-film layer formed of one or more layers including at least a light emitting layer, the organic thin-film layer being interposed between a cathode and an anode, in which at least one layer of the organic thin-film layer contains the aromatic amine derivative alone or as a component of a mixture, the organic electroluminescence device having a long lifetime and high luminous efficiency; and an aromatic amine derivative for realizing the device.

## Description

### Technical Field

The present invention relates to an aromatic amine derivative and an organic electroluminescence device using the derivative, in particular, an organic electroluminescence device having a long lifetime and high luminous efficiency, and an aromatic amine derivative for realizing the device.

### Background Art

An organic EL device is a spontaneous light emitting device which utilizes such a principle that a fluorescent substance emits light by virtue of recombination energy of holes injected from an anode and electrons injected from a cathode by an application of an electric field. Since an organic EL device of the laminate type capable of being driven under low electric voltage has been reported by C. W. Tang et al. of Eastman Kodak Company (C. W. Tang and S. A. Vanslyke, Applied Physics Letters, Volume 51, Page 913, 1987, or the like), many studies have been conducted for an organic EL device using an organic material as a constituent material. Tang et al. used tris (8-quinolinolato) aluminum for a light emitting layer and a triphenyldiamine derivative for a hole transporting layer. Advantages of the laminate structure reside in the followings: an efficiency of the hole injection into the light emitting layer can be increased; an efficiency of forming exciton which are formed by blocking and recombining electrons injected from the cathode can be increased; and exciton formed within the light emitting layer can be enclosed. As described above, for the structure of the organic EL device, a two-layered structure having a hole transporting (injecting) layer and an electron transporting emitting layer and a three-layered structure having a hole transporting (injecting) layer, a light emitting layer, an electron transporting (injecting) layer, and the like are widely known. In order to increase the efficiency of recombination of injected holes and electrons in the devices of the laminate type, the device structure and the process for forming the device have been studied.

In general, when an organic EL device is driven or stored in an environment of high temperature, there occur adverse effects such as a change in the luminescent color, a decrease in emission efficiency, an increase in driving voltage, and a decrease in a lifetime of light emission.
An aromatic amine derivative having a dibenzofuran skeleton has been proposed for preventing such adverse effects. Patent Literatures 1 to 3 have been reported as compounds each having dibenzofuran in the central skeleton of a diamine compound. Meanwhile, Patent Literatures 4 to 7 have been reported as compounds each having dibenzofuran on a monoamine through an aryl group. However, organic EL devices using those compounds do not show sufficient performance.
In addition, there have been a large number of reports concerning an amine compound in which N-carbazole is bonded to an amine through an aryl group, and examples of the reports include Patent Literatures 8 to 10. However, an organic EL device using such compound does not show sufficient performance.
Further, Patent Literatures 11 and 12 have each reported an amine compound in which 3-carbazole is directly bonded to an amine. However, an organic EL device using such compound does not show sufficient performance. In addition, Patent Literatures 13 and 14 have each reported an amine compound in which 3-carbazole is bonded to an amine through an aryl group. However, an organic EL device using such compound does not show sufficient performance.
As described above, the organic EL device having a high efficiency and a long lifetime has been reported, but it is yet hard to say that the device always shows sufficient performance, so development of the organic EL device having a further excellent performance has been strongly desired.

### Citation List

### Patent Literature

[PTL 1] JP 2005-112765 A
[PTL 2] JP 11-111460 A
[PTL 3] WO 2006/122630 A1
[PTL 4] WO 2006/128800 A1
[PTL 5] JP 2006-151844 A
[PTL 6] JP 2008-021687 A
[PTL 7] WO 2007/125714 A1
[PTL 8] US 6,242,115
[PTL 9] JP 2007-284431 A
[PTL 10] JP 2003-031371 A
[PTL 11] JP 2007-318101 A
[PTL 12] JP 2006-151979 A
[PTL 13] JP 2005-290000 A
[PTL 14] WO 2008/062636 A1

### Summary of Invention

### Technical Problem

The present invention has been made to solve the problems, and an object of the present invention is to provide an organic EL device having a long lifetime and high luminous efficiency, and an aromatic amine derivative for realizing the device.

### Solution to Problem

The inventors of the present invention have made extensive studies with a view toward achieving the object. As a result, the inventors have found that the object can be achieved by using an aromatic amine derivative represented by the following formula (1) as a material for an organic EL device, and thus the present invention has been completed.
That is, the present invention provides the aromatic amine derivative represented by the following formula (1).

A-L-B (1)

In the formula (1), L is represented by the following formula (2).

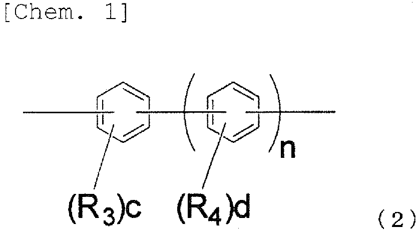

In the formula (2):
n represents an integer of 0 to 3;
R₃ and R₄ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group;
a plurality of R₃'s or R₄'s adjacent or close to each other may be bonded to each other to form a saturated or unsaturated, divalent group that forms a ring;
R₃ and R₄ adjacent to each other may be bonded to each other so that L forms a substituted or unsubstituted fluorenylene group; and
c and d each independently represent an integer of 0 to 4.
In the formula (1), A is represented by the following formula (3).

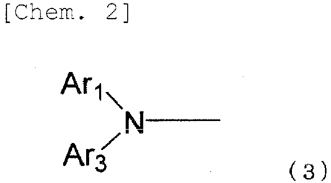

In the formula (3), Ar₁ represents a substituted or unsubstituted aryl group having 6 to 25 ring carbon atoms, or a substituted or unsubstituted heteroaryl group having 5 to 25 ring atoms, and Ar₃ is represented by the following formula (4).

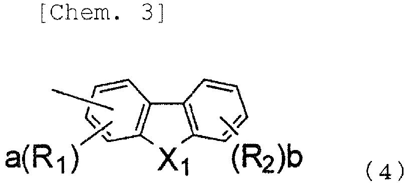

In the formula (4):
X₁ represents O (oxygen atom), S (sulfur atom), NRa, or CRbRc, Ra represents a group selected from the group consisting of an aryl group having 6 to 25 ring carbon atoms and a heteroaryl group having 5 to 25 ring atoms, and Rb or Rc each independently represent a group selected from the group consisting of an aryl group having 6 to 25 ring carbon atoms and a heteroaryl group having 5 to 25 ring atoms;
R₁ and R₂ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group, and a plurality of R₁'s or R₂'s adjacent to each other, or R₁ and R₂ may be bonded to each other to form a saturated or unsaturated, divalent group that forms a ring;
a represents an integer of 0 to 3; and
b represents an integer of 0 to 4.
In the formula (1), B is represented by the following formula (5).

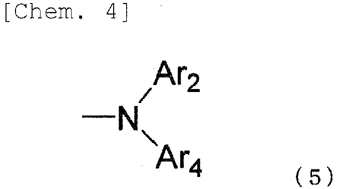

In the formula (5), Ar₂ or Ar₄ represents a substituted or unsubstituted aryl group having 6 to 25 ring carbon atoms, or a substituted or unsubstituted heteroaryl group having 5 to 25 ring atoms.

Further, the present invention provides an organic EL device, including an organic thin-film layer formed of one or more layers including at least a light emitting layer, the organic thin-film layer being interposed between a cathode and an anode, in which at least one layer of the organic thin-film layer contains the aromatic amine derivative alone or as a component of a mixture.

### Advantageous Effects of Invention

An organic EL device using the aromatic amine derivative of the present invention has high luminous efficiency and hardly deteriorates even when used for a long time period, in other words, has a long lifetime.

### Description of Embodiments

The present invention provides an aromatic amine derivative represented by the following formula (1).

A-L-B (1)

In the formula (1), L is represented by the following formula (2).

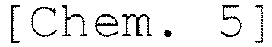

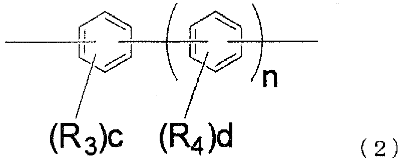

In the formula (2):
n represents an integer of 0 to 3, preferably 0 or 1;
R₃ and R₄ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group;
a plurality of R₃'s or R₄'s adjacent or close to each other may be bonded to each other to form a saturated or unsaturated, divalent group that forms a ring;
R₃ and R₄ adjacent to each other may be bonded to each other so that L forms a substituted or unsubstituted fluorenylene group; and
c and d each independently represent an integer of 0 to 4, preferably 0 to 2.

Examples of the alkyl group represented by each of R₃ and R₄ include a methyl group, an ethyl group, a propyl group, an isopropyl group, a butyl group, an sec-butyl group, a tert-butyl group, a pentyl group, a hexyl group, a heptyl group, an octyl group, a stearyl group, a 2-phenylisopropyl group, a trichloromethyl group, a trifluoromethyl group, a benzyl group, any -phenoxybenzyl group, an α ,α -dimethylbenzyl group, an α, α -methylphenylbenzyl group, an α ,α -ditrifluoromethylbenzyl group, a triphenylmethyl group, and an α -benzyloxybenzyl group.
Examples of the alkenyl group represented by each of R₃ and R₄ include a vinyl group, an allyl group, a1-butenylgroup, a 2-butenyl group, a 3-butenyl group, a 1,3-butanedienyl group, a 1-methylvinyl group, a styryl group, a 2,2-diphenylvinyl group, and a 1,2-diphenylvinyl group.
Examples of the cycloalkyl group represented by each of R₃ and R₄ include a cyclopropyl group, a cyclobutyl group, a cyclopentyl group, a cyclohexyl group, a cycloheptyl group, a cyclooctyl group, a cyclononyl group, a bicycloheptyl group, a bicyclooctyl group, a tricycloheptyl group, and an adamantyl group. Of those, a cyclopentyl group, a cyclohexyl group, a cycloheptyl group, a bicycloheptyl group, a bicyclooctyl group, or an adamantyl group is preferred.
Examples of the alkyl groups of the trialkylsilyl group represented by each of R₃ and R₄ include the same examples as those of the alkyl group.
Examples of the aryl group represented by each of R₃ and R₄ include a phenyl group, a 2-methylphenyl group, a 3-methylphenyl group, a 4-methylphenyl group, a 4-ethylphenyl group, a biphenyl group, a 4-methylbiphenyl group, a 4-ethylbiphenyl group, a 4-cyclohexylbiphenyl group, a terphenyl group, a 3,5-dichlorophenyl group, a naphthyl group, a 5-methylnaphthyl group, an anthryl group, a pyrenyl group, a chrysenyl group, a fluoranthenyl group, and a perylenyl group.
Examples of the aryl groups of the triarylsilyl group represented by each of R₃ and R₄ include the same examples as those of the aryl group.
Examples of the alkyl group and aryl group of the alkylarylsilyl group represented by each of R₃ and R₄ include the same examples as those of the alkyl group and the aryl group.
Examples of the heteroaryl group represented by each of R³ and R^{a} include residues of imidazole, benzimidazole, pyrrole, furan, thiophene, oxadiazoline, indoline, carbazole, pyridine, quinoline, isoquinoline, benzoquinone, pyrrarlozine, imidazolidine, and piperidine.
Examples of the halogen atom represented by each of R₃ and R₄ include fluorine, chlorine, bromine, and iodine.

In addition, examples of the saturated or unsaturated, divalent group that forms a ring as a result of mutual bonding of a plurality of R₃'s or R₄'s adjacent or close to each other, or R₃ and R₄ include a fluorenylene group, a 9, 9-dimethylfluorenylene group, and a phenanthrenylene group.
Specific examples of L include the following structures.

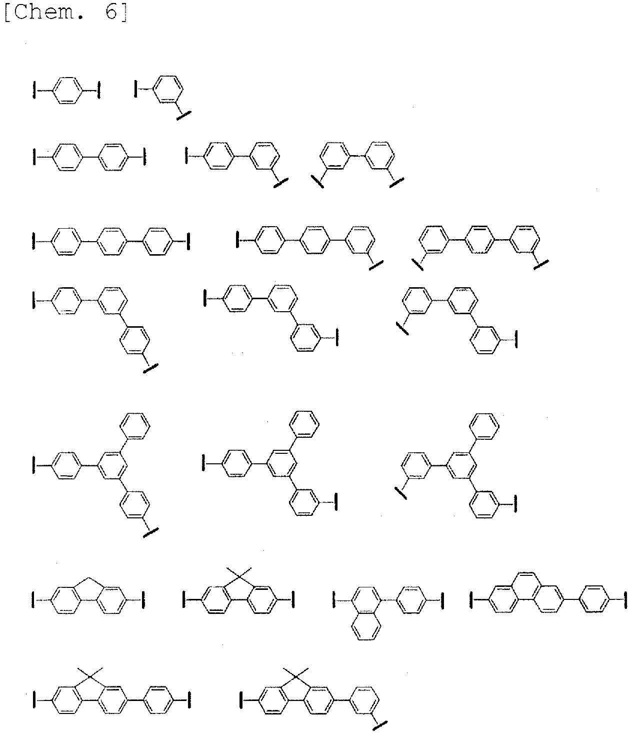

In the formula (1), A is represented by the following formula (3).

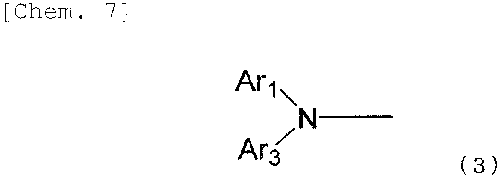

In the formula (3), Ar₁ represents a substituted or unsubstituted aryl group having 6 to 25 ring carbon atoms, or a substituted or unsubstituted heteroaryl group having 5 to 25 ring atoms, and Ar₃ is represented by the following formula (4).
Specific examples of the aryl group and the heteroaryl group each represented by Ar₁ include the same examples as those of the R₃ and the R₄.

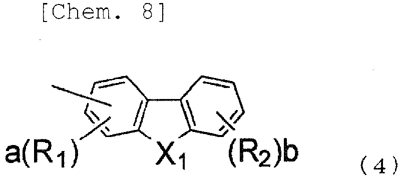

In the formula (4):
X₁ represents O (oxygen atom), S (sulfur atom), NRa, or CRbRc, Ra represents a group selected from the group consisting of an aryl group having 6 to 25 ring carbon atoms and a heteroaryl group having 5 to 25 ring atoms, and Rb or Rc each independently represent a group selected from the group consisting of an aryl group having 6 to 25 ring carbon atoms and a heteroaryl group having 5 to 25 ring atoms.
Examples of the aryl group and the heteroaryl group represented by each of Ra, Rb, and Rc include the same examples as those of R₃ and R₄.
R₁ and R₂ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group, and a plurality of R₁'s or R₂'s adj acent to each other, or R₁ and R₂ may be bonded to each other to form a saturated or unsaturated, divalent group that forms a ring.
Examples of the alkyl group, the alkenyl group, the cycloalkyl group, the trialkylsilyl group, the triarylsilyl group, the alkylarylsilyl group, the aryl group, the heteroaryl group, and the halogen atom represented by R₁ and R₂ include the same examples as those of R₃ and R₄.
In addition, examples of the saturated or unsaturated, divalent group that forms a ring as a result of mutual bonding of a plurality of R₁'s or R₂'s adjacent to each other, or R₁ and R₂ include the same examples as those of the saturated or unsaturated, divalent group that forms a ring as a result of mutual bonding of R₃'s or R₄'s.
a represents an integer of 0 to 3, preferably 0 to 2.
b represents an integer of 0 to 4, preferably 0 to 2.
Specific examples of the formula (4) include the following structures. The examples include structures each obtained by changing S or O in any one of the following structures to the NRa or the CRbRc as well.

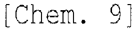

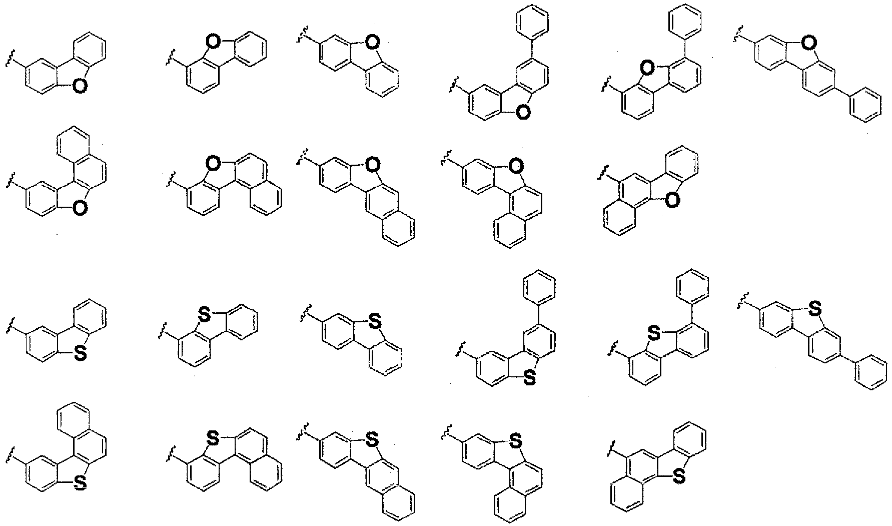

In the formula (1), B is represented by the following formula (5).

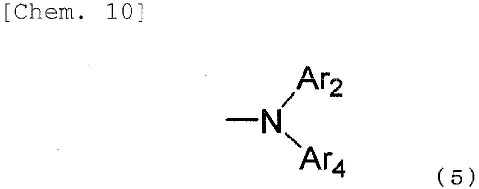

In the formula (5), Ar₂ or Ar₄ represents a substituted or unsubstituted aryl group having 6 to 25 ring carbon atoms, or a substituted or unsubstituted heteroaryl group having 5 to 25 ring atoms.
Specific examples of the aryl group and the heteroaryl group represented by Ar₂ and Ar₄ include the same examples as those of the R₃ and the R₄.

In the formula (1), the Ar₃ is represented by preferably any one of the following formulae (6) to (8), more preferably the formula (6) or (8), particularly preferably the formula (6). 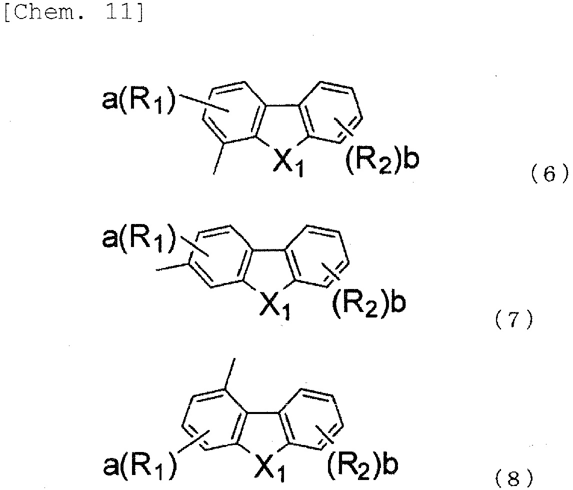 (In the formulae (6) to (8), X₁, R₁, R₂, a, and b each have the same meaning as that used in the formula (4).)

In addition, in the formula (1), the Ar₁ or the Ar₄ is preferably represented by the following formula (9).

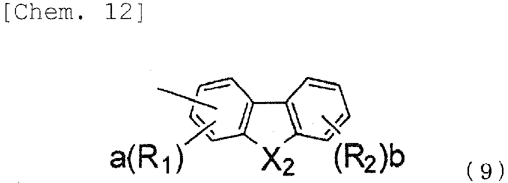

In the formula (9), X₂ represents O (oxygen atom), S (sulfur atom), NRa, or CRbRc. Ra represents a group selected from the group consisting of an aryl group having 6 to 25 ring carbon atoms and a heteroaryl group having 5 to 25 ring atoms. Rb or Rc each independently represent a group selected from the group consisting of an aryl group having 6 to 25 ring carbon atoms and a heteroaryl group having 5 to 25 ring atoms.
Specific examples of the aryl group and the heteroaryl group represented by each of Ra, Rb, and Rc include the same examples as those of the R₃ and the R₉.
R₁ and R₂ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group, and a plurality of R₁'s or R₂'s adjacent to each other, or R₁ and R₂ may be bonded to each other to form a saturated or unsaturated, divalent group that forms a ring.
Examples of the alkyl group, the alkenyl group, the cycloalkyl group, the trialkylsilyl group, the triarylsilyl group, the alkylarylsilyl group, the acryl group, the heteroaryl group, and the halogen atom represented by R₁ and R₂ include the same examples as those of R₃ and R₄.
In addition, examples of the saturatedorunsaturated, divalent group that forms a ring as a result of mutual bonding of a plurality of R₁'s or R₂'s adjacent to each other, or R₁ and R₂ include the same examples as those of the saturated or unsaturated, divalent group that forms a ring as a result of mutual bonding of R₃'s or R₄'s.
a represents an integer of 0 to 3, preferably 0 to 2.
b represents an integer of 0 to 4, preferably 0 to 2.
Specific examples of the formula (9) include the following structures.

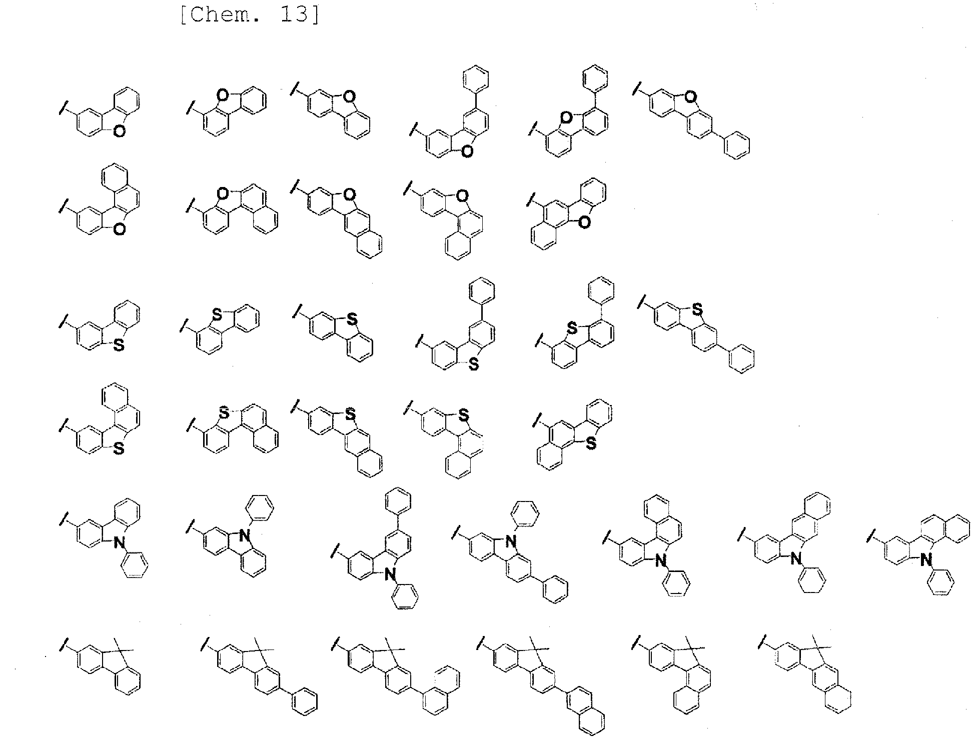

Ar₁ or Ar₄ is preferably represented by any one of the formulae (6) to (8).
When A and B in the formula (1) are identical to each other, an improvement in hole mobility can be expected. When A and B are different from each other, the crystallization of the material is suppressed by the collapse of its symmetry and hence an improvement in the stability of a thin film can be expected.
When the formula (4) and/or the formula (9) are each/is bonded at the 3-position in the formula (1), the hole mobility increases as a result of the expansion of a n-electron conjugated system.
The interaction of the formula (1) with an electron-accepting compound having high planarity typified by each of the following formulae (A) and (B) is improved by a heterocycle having high planarity like the formula (4) or (9).
In addition, when the heterocycle having high planarity like the formula (4) or (9) is directly bonded to a nitrogen atom, an electron density increases. As a result, an ionization potential value reduces, and hence the material can be used not only as a hole transporting material but also as a hole injecting material.
Further, the formula (1) has a diamine structure. As a result, the number of hopping sites increases, and hence a hole injection amount and the hole mobility are improved.

The singlet energy gap and triplet energy gap of the aromatic amine derivative of the present invention can be expanded as a result of the direct bonding of the heterocycle similar to a fluorene structure to the nitrogen atom. Accordingly, when the derivative is used in a layer (hole transporting layer) adjacent to a light emitting layer containing a host material and a dopant material that shows light emission, a reduction in luminous efficiency due to the movement of a carrier from the light emitting layer or to the transfer of each of singlet energy and triplet energy can be suppressed. Further, the derivative has the heterocycle. As a result, its glass transition temperature (Tg) can be increased and hence the stability of an organic thin-film layer can be improved.
In particular, the heterocycle is more preferably bonded to the nitrogen atom at such a position that the conjugated system is additionally contracted (e.g., a position except the 3-position in the case of dibenzofuran) because of the following reason. The singlet energy gap and the triplet energy gap can be additionally expanded. For example, the triplet energy gap can be set to 2.6 eV or more.

In addition, in the formula (1), any one of the Ar₁, the Ar₂, and the Ar₄ is preferably represented by the following formula (10).

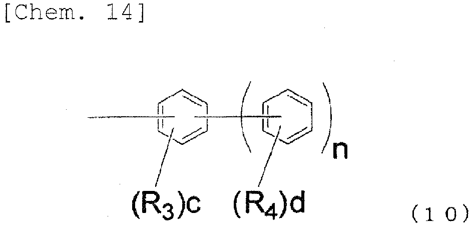

(In the formula (10), R₃, R₄, c, d, and n each have the same meaning as that used in the formula (2).) From the viewpoint of the expansion of the singlet energy gap and the triplet energy gap, n represents preferably 0 to 3, more preferably 0 or 1, particularly preferably 0. In addition, when the bonding position of a benzene ring is a position except a para position, the conjugated system in a molecule can be contracted, and hence the singlet energy gap and the triplet energy gap can be additionally expanded.
Specific examples of the substituent represented by the formula (10) include, but not limited to, the following.

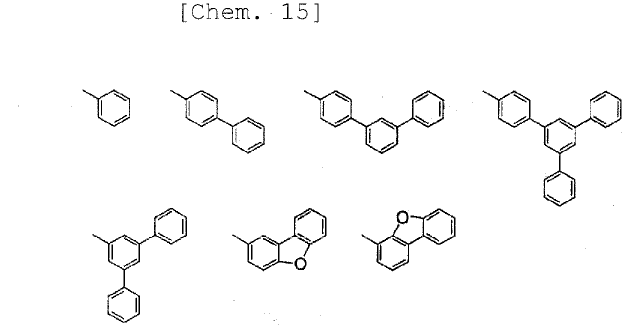

In addition, any one of the Ar₁, the Ar₂, and the Ar₄ preferably represents a phenyl group, a biphenyl group, or an m-terphenyl group.
Specific examples of the aromatic amine derivative represented by the formula (1) of the present invention are shown below. However, the derivative is not limited to these exemplified compounds.

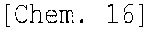

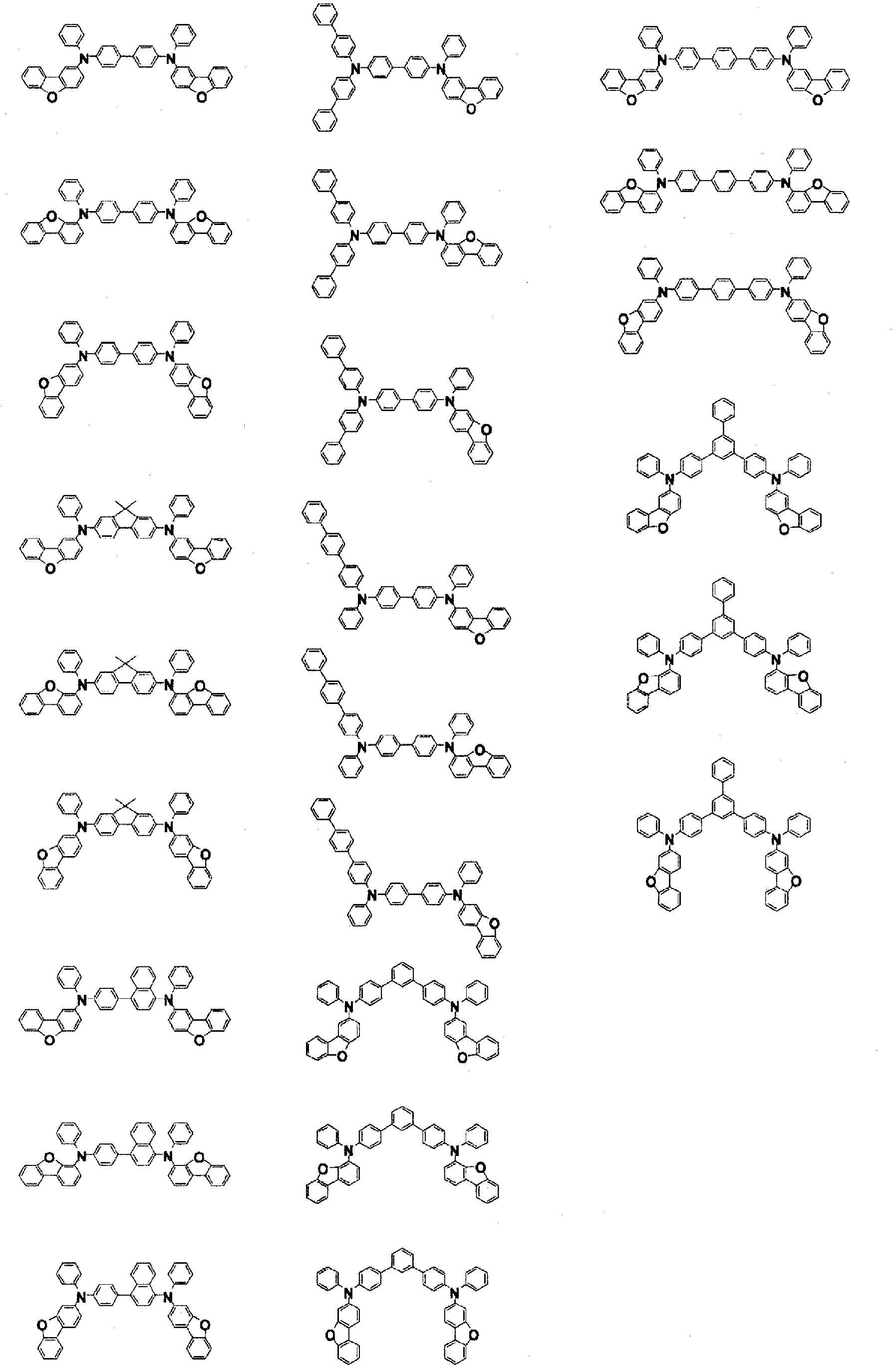

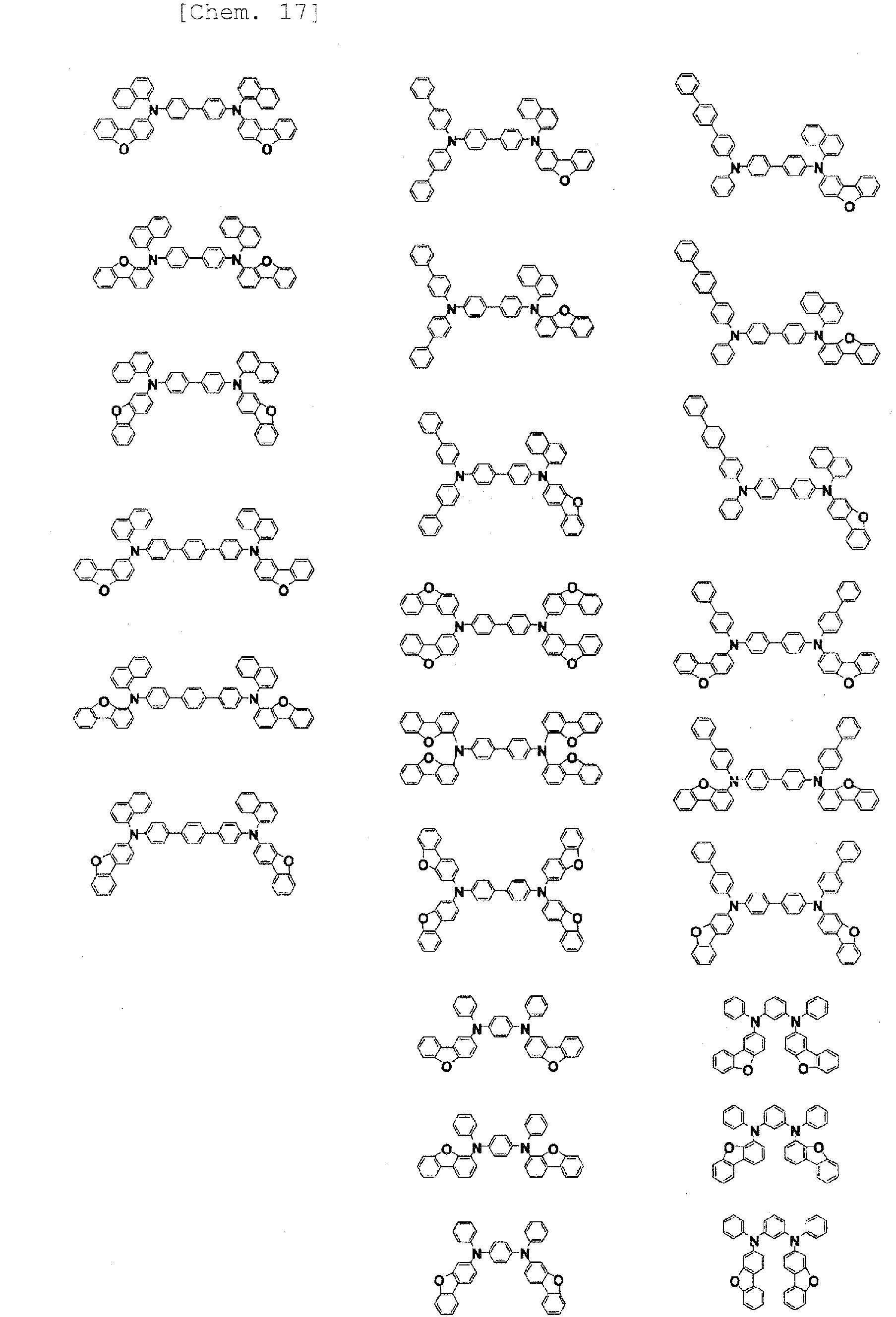

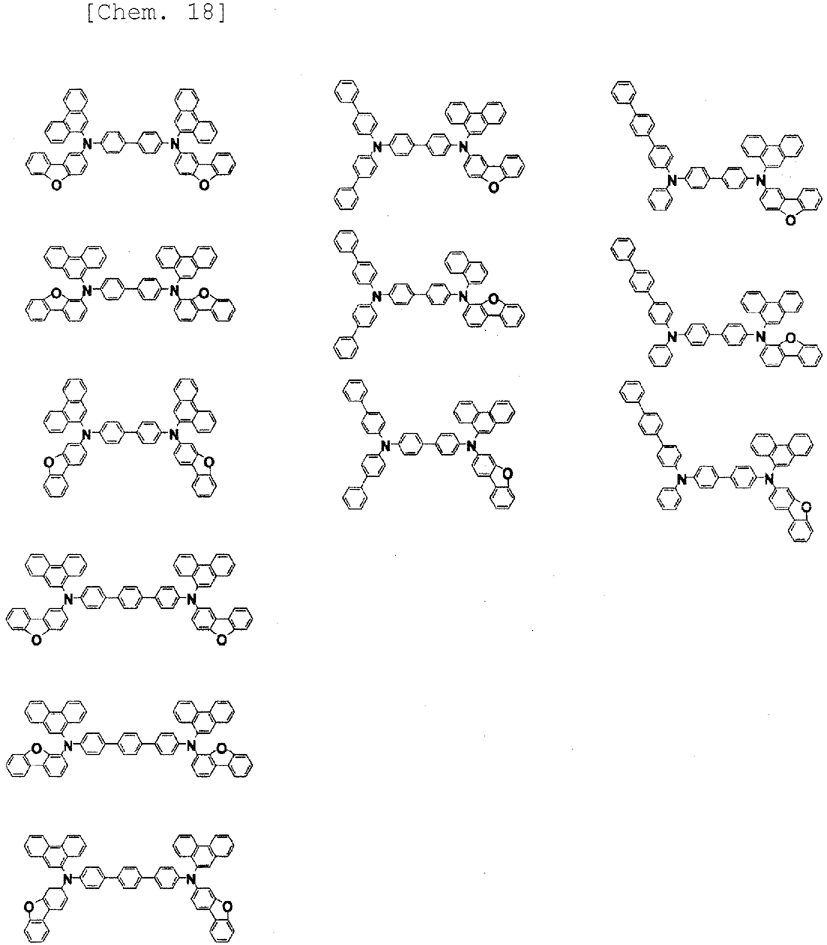

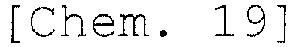

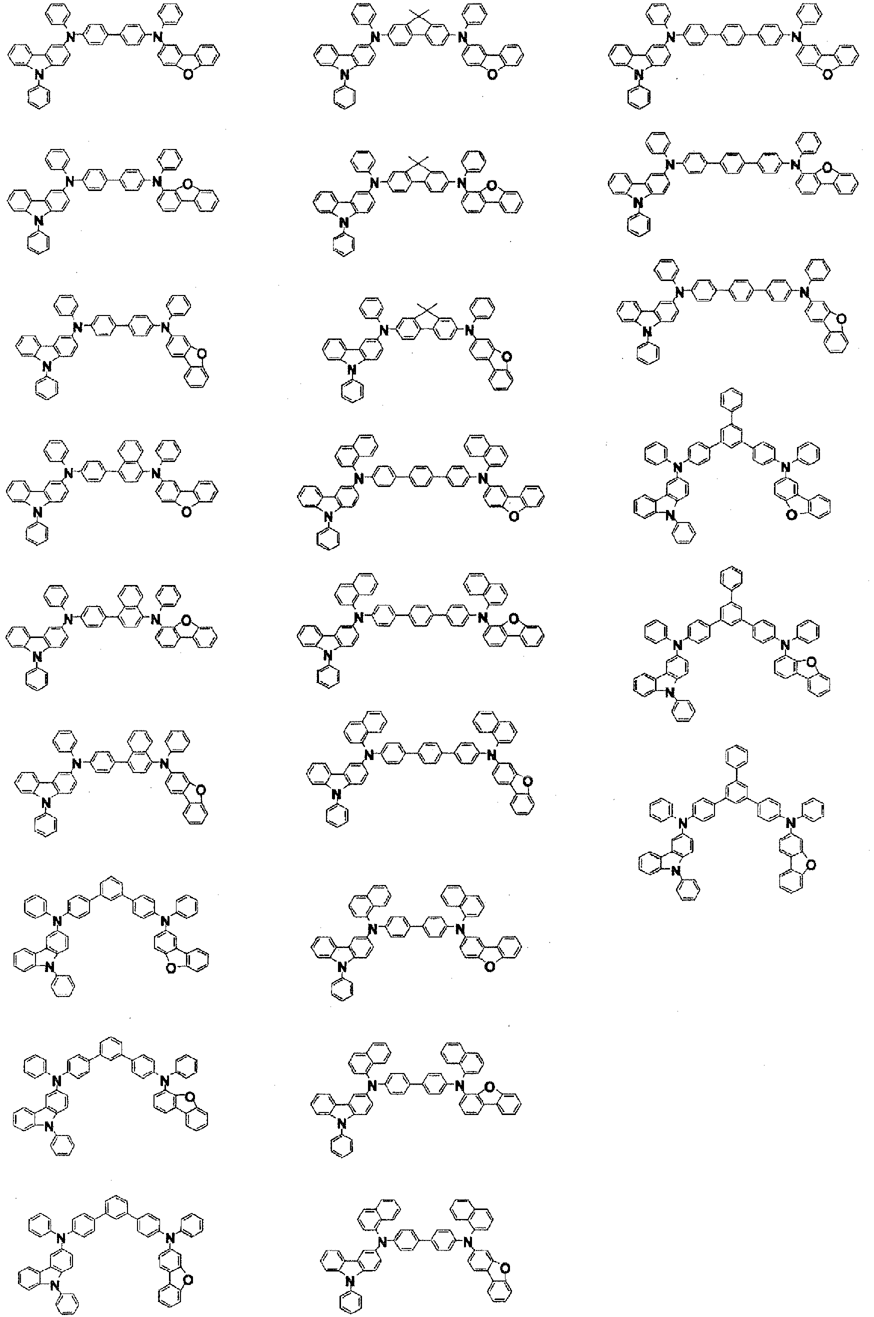

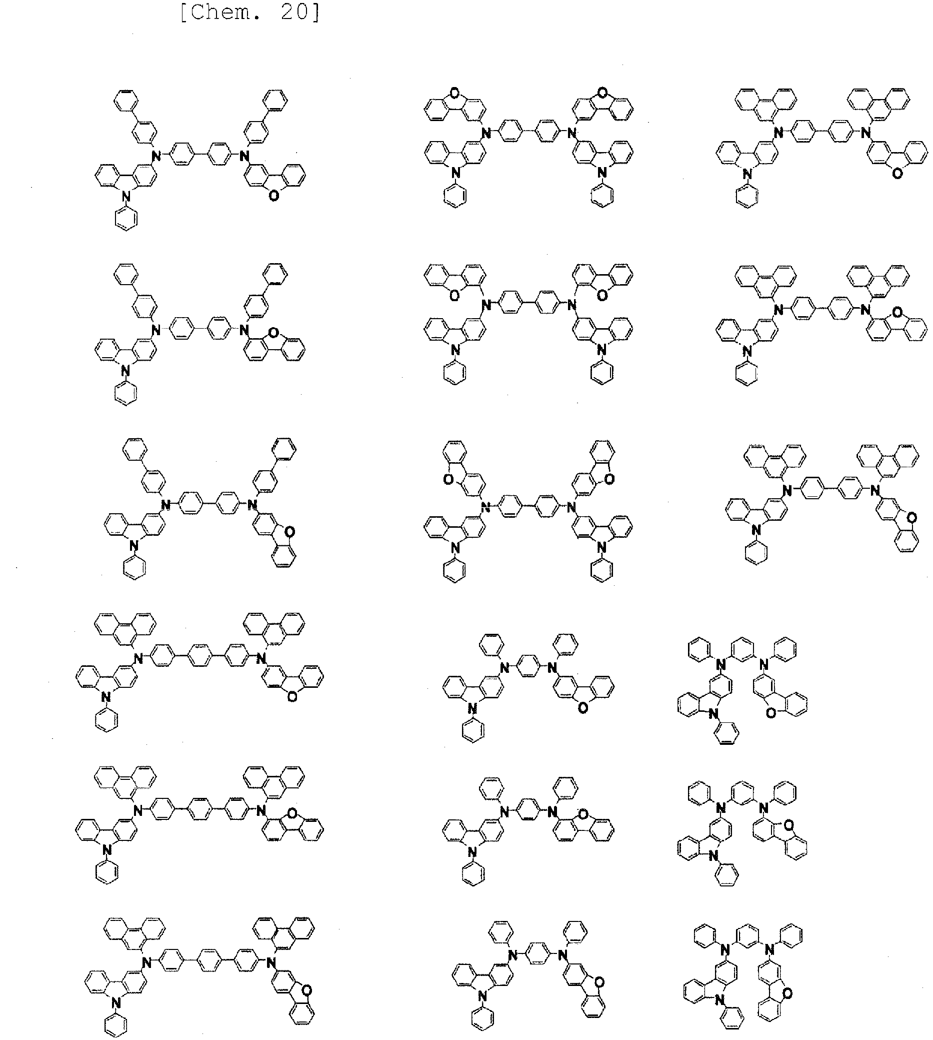

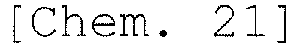

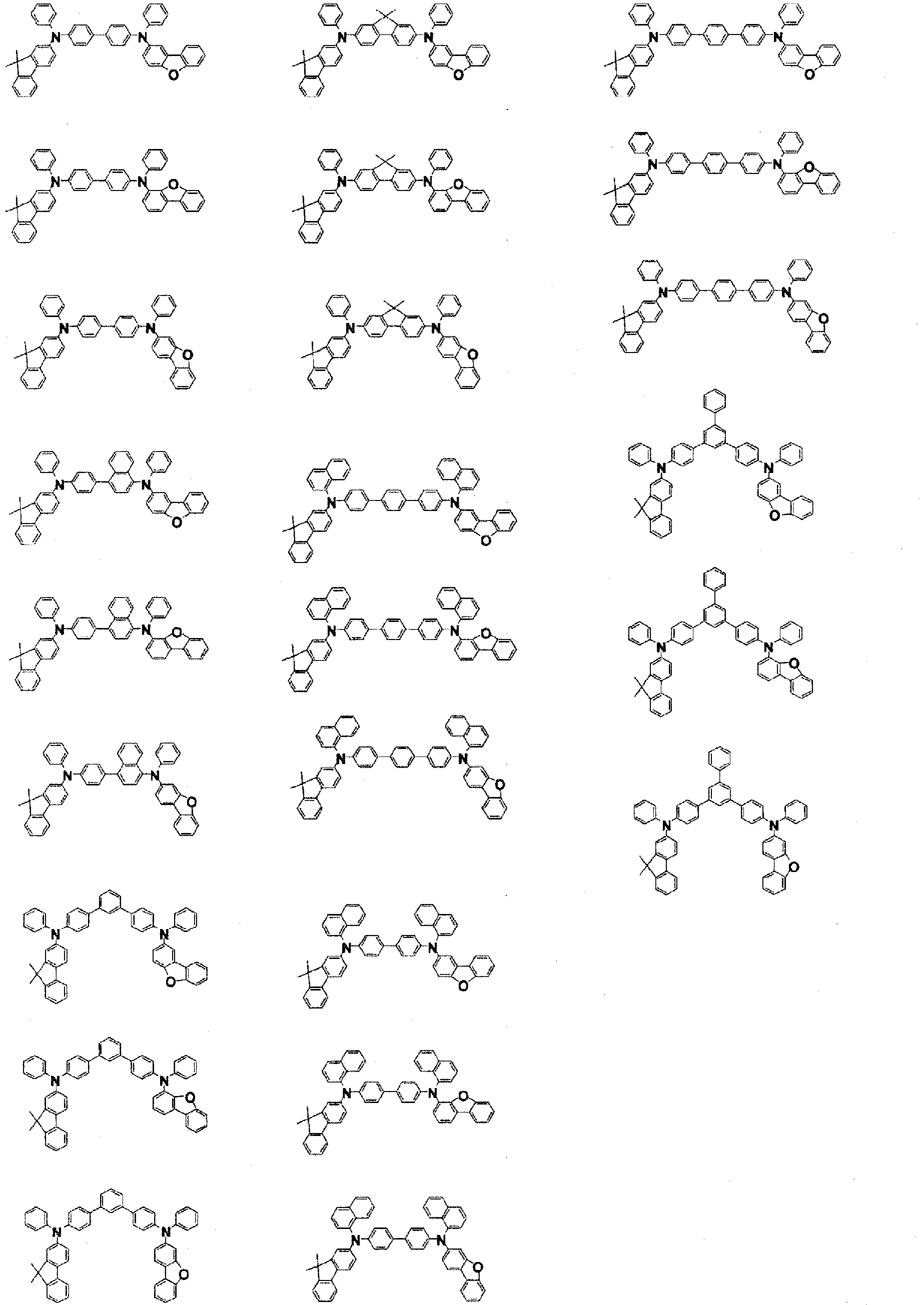

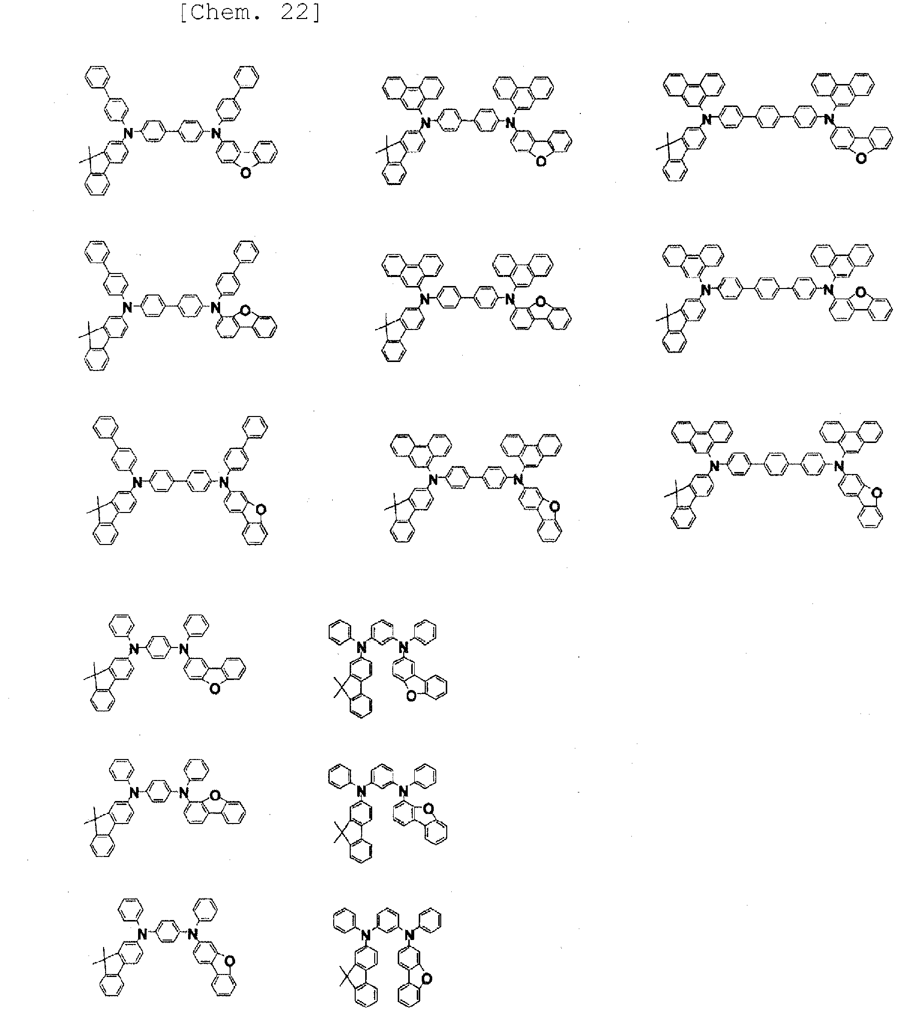

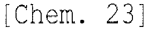

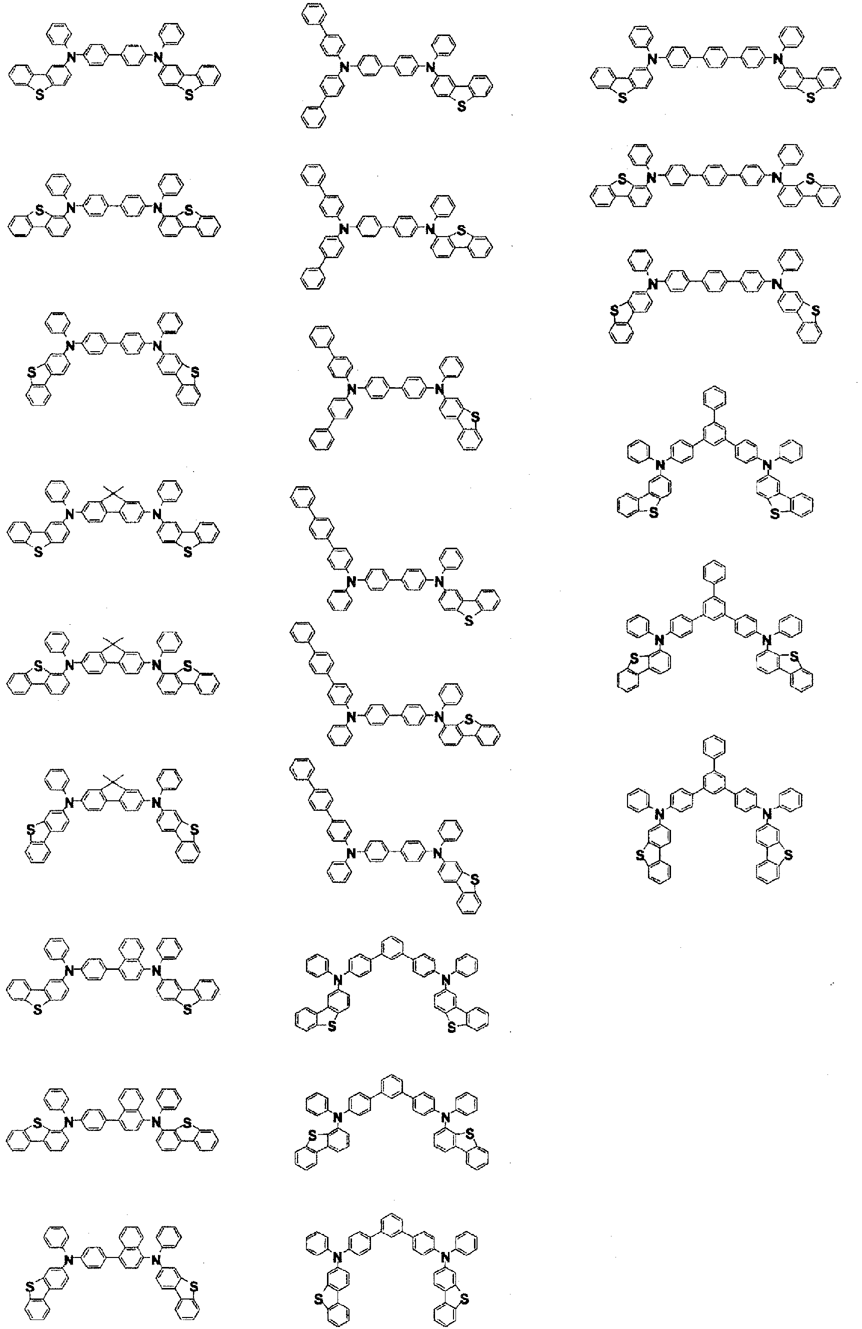

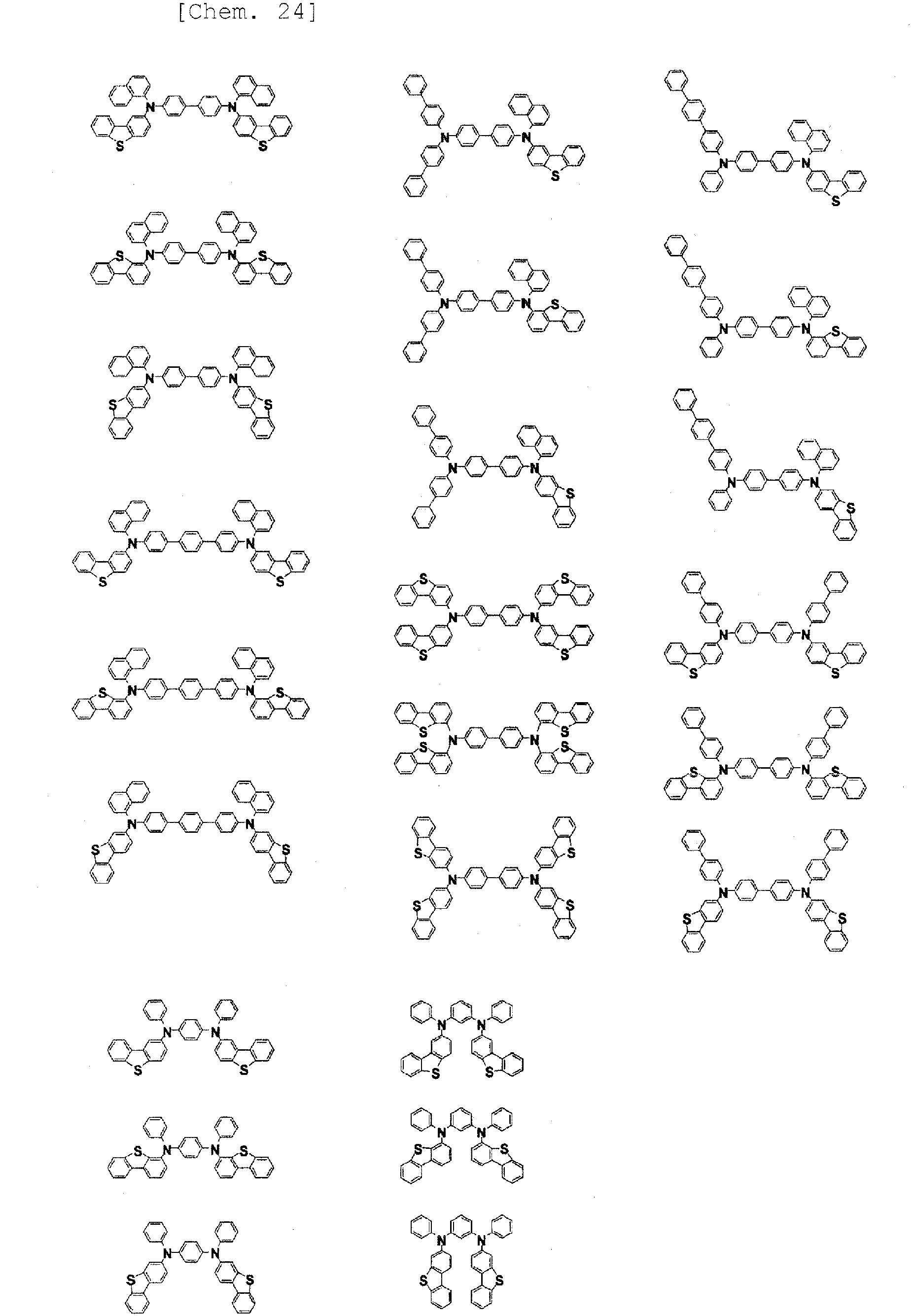

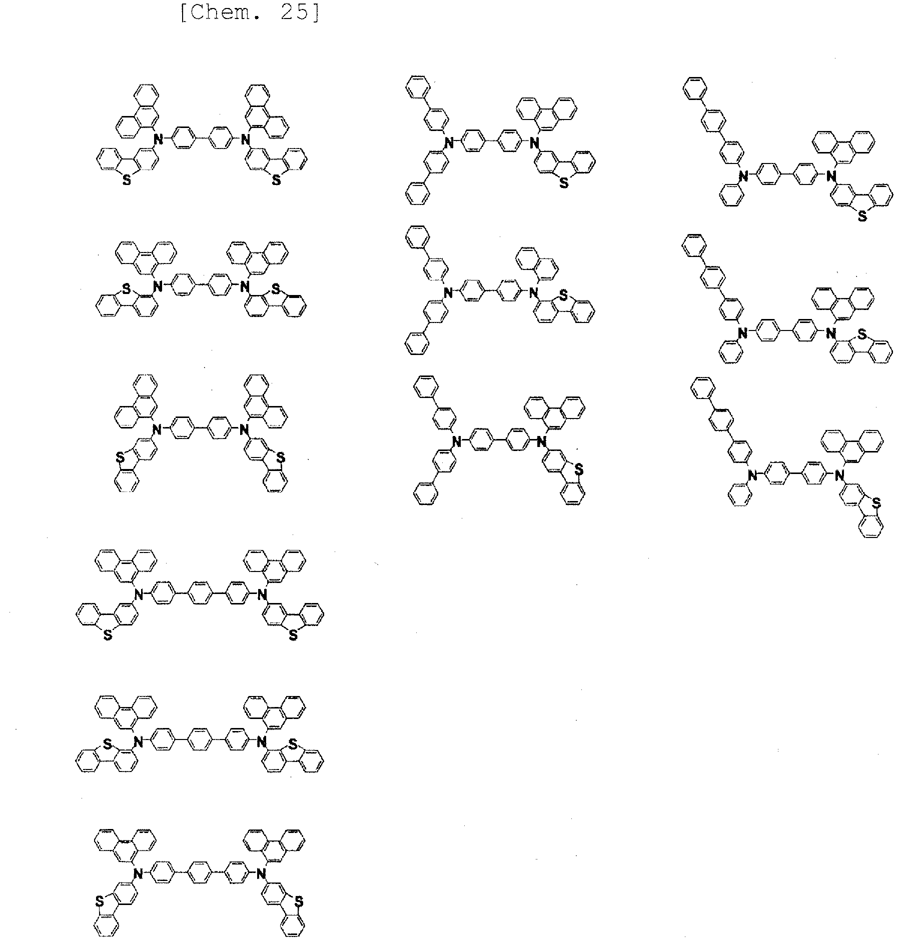

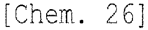

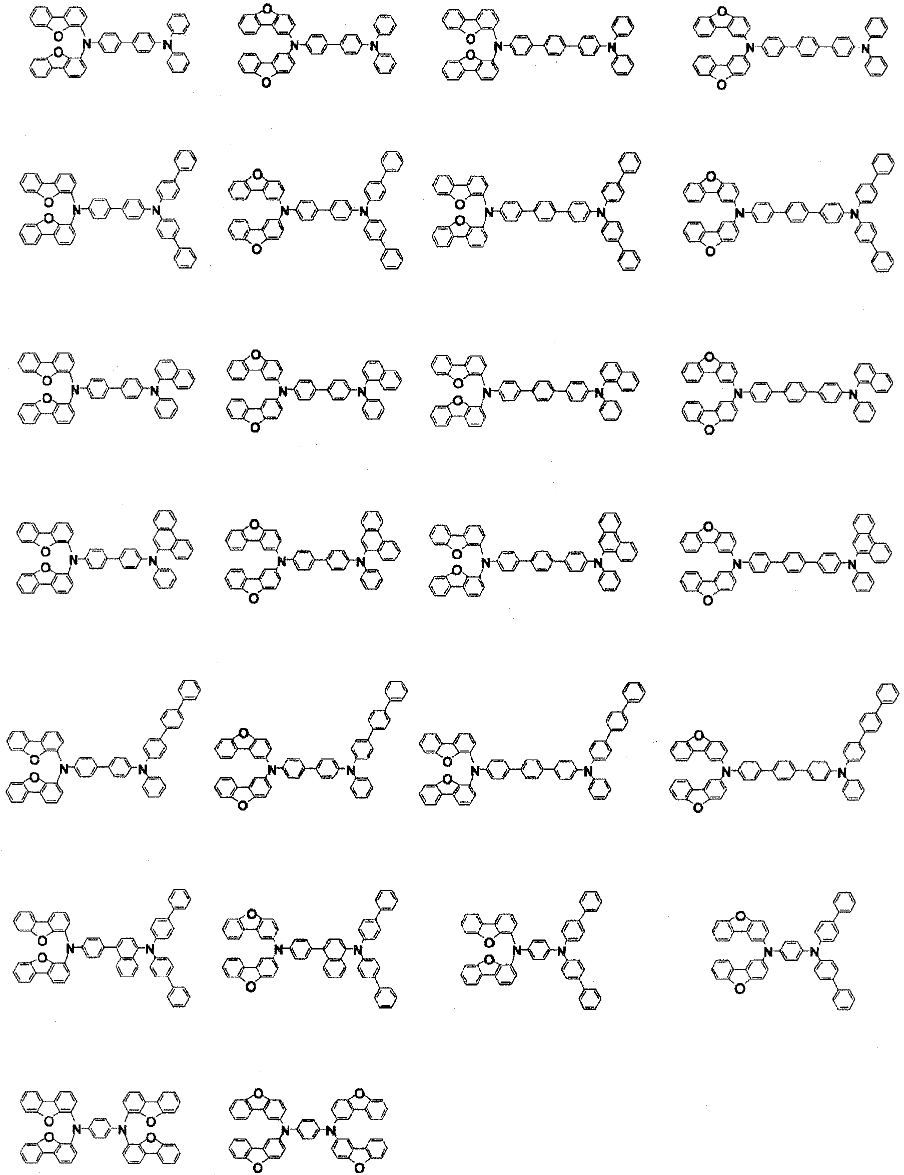

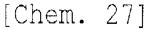

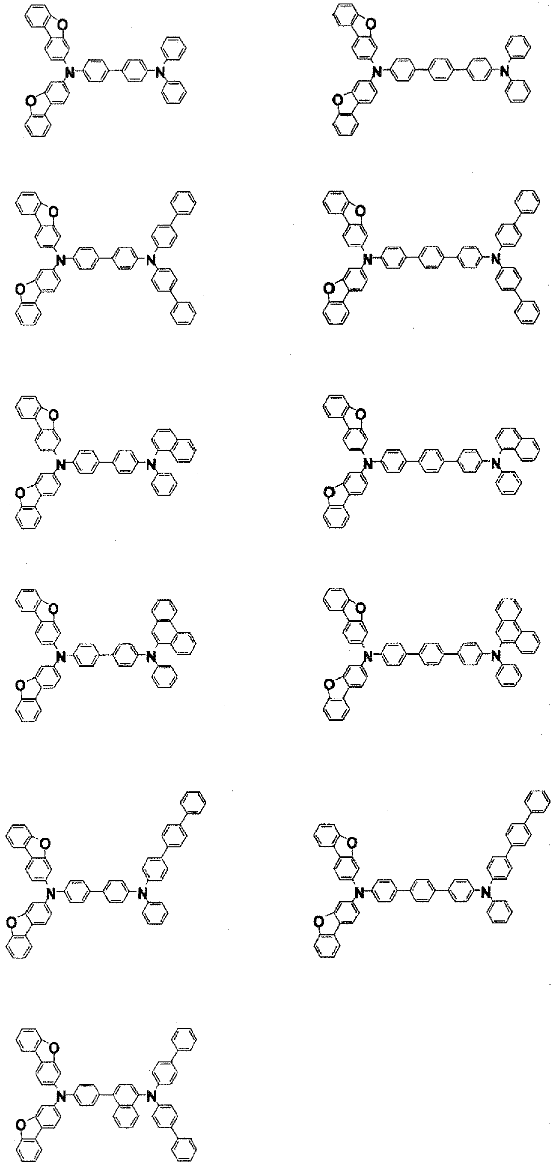

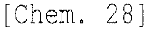

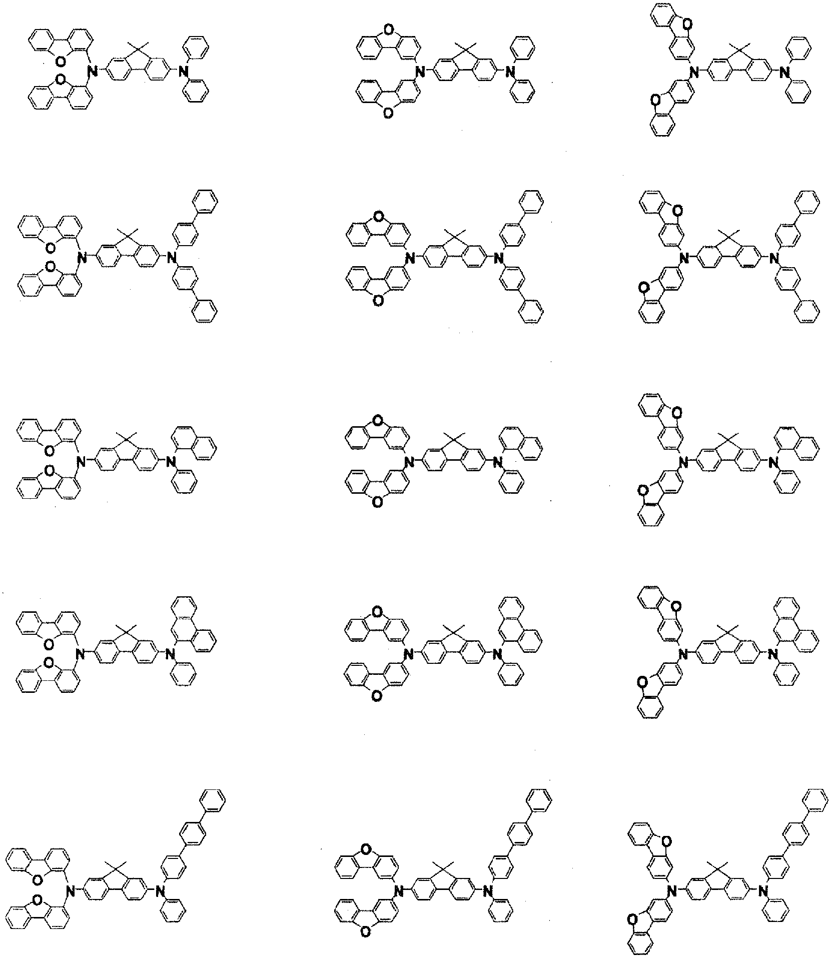

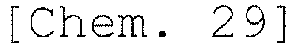

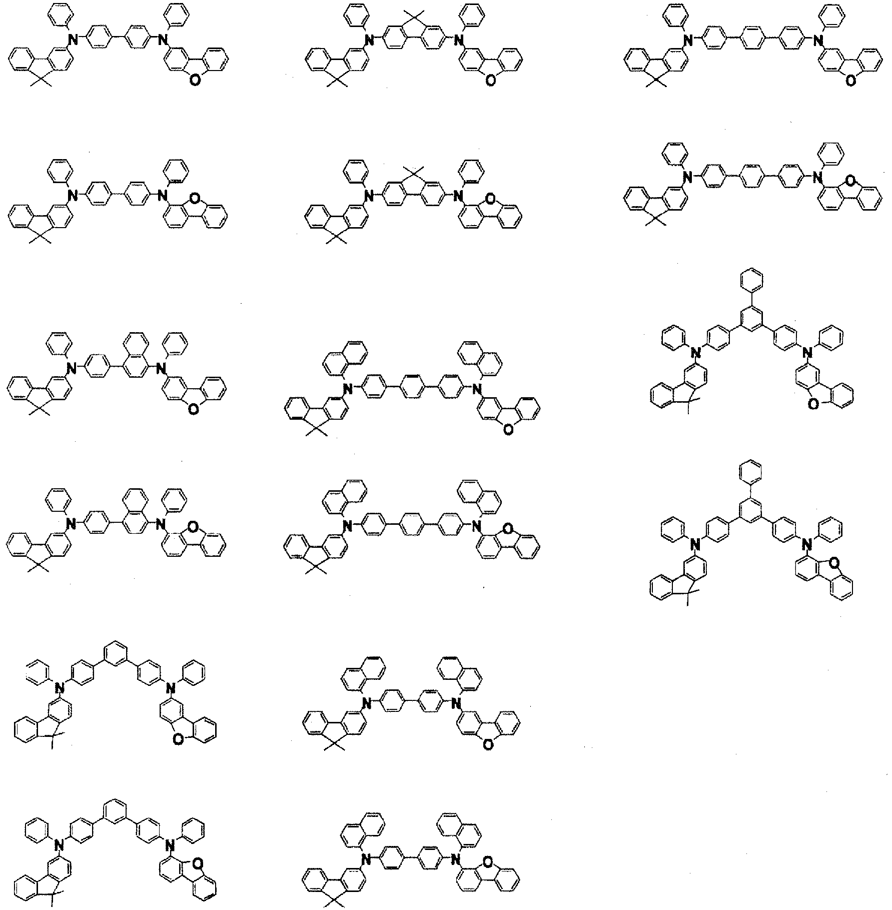

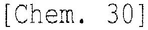

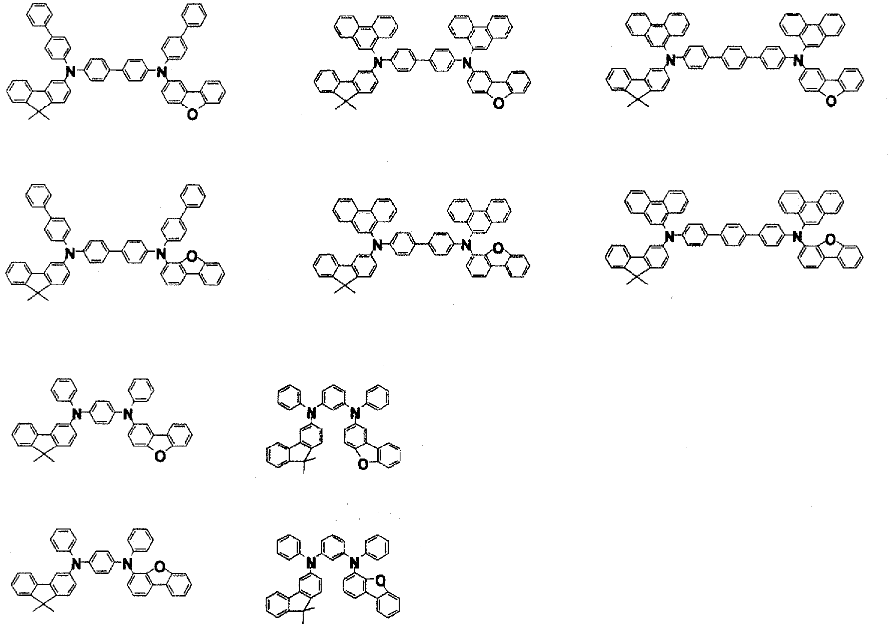

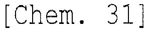

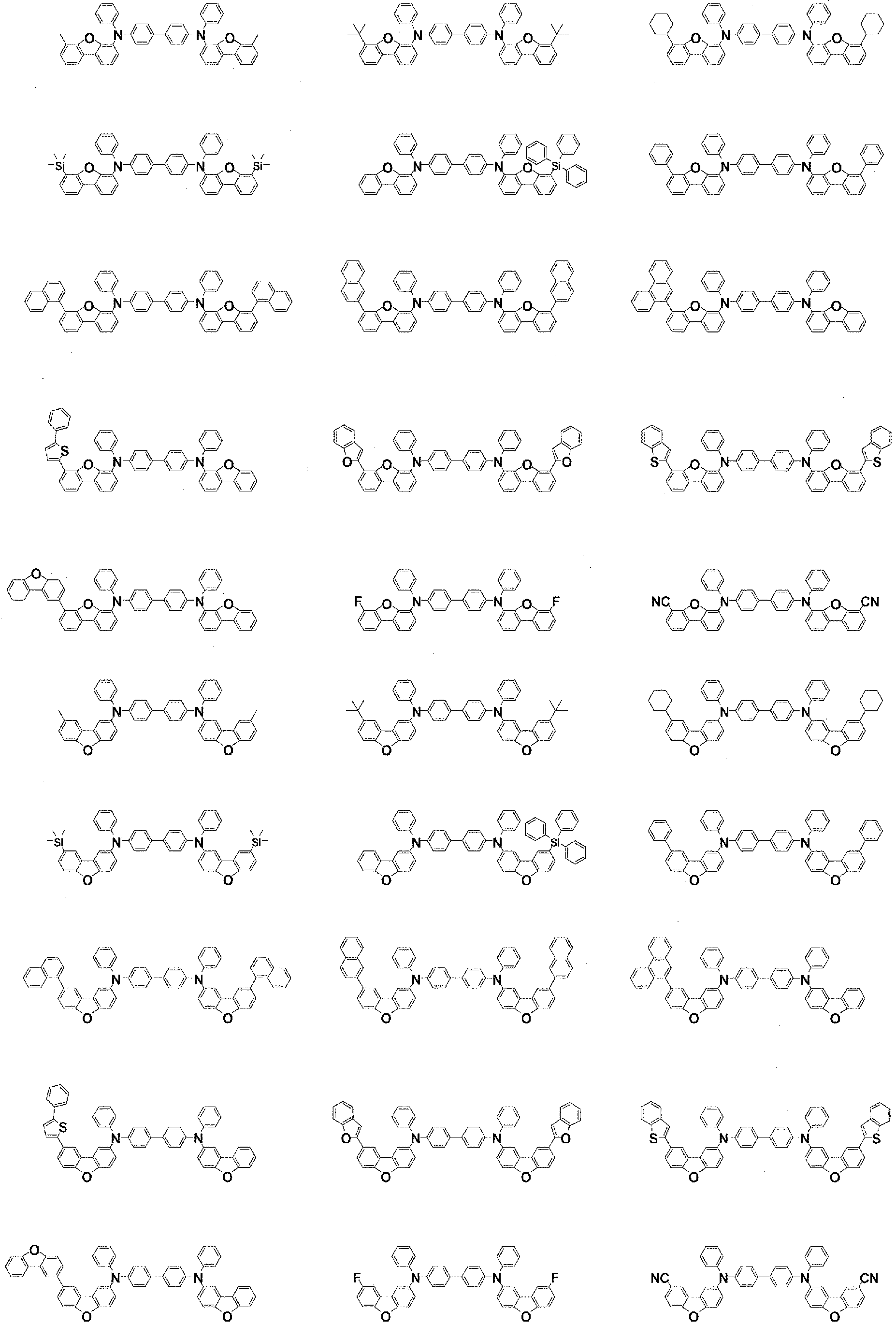

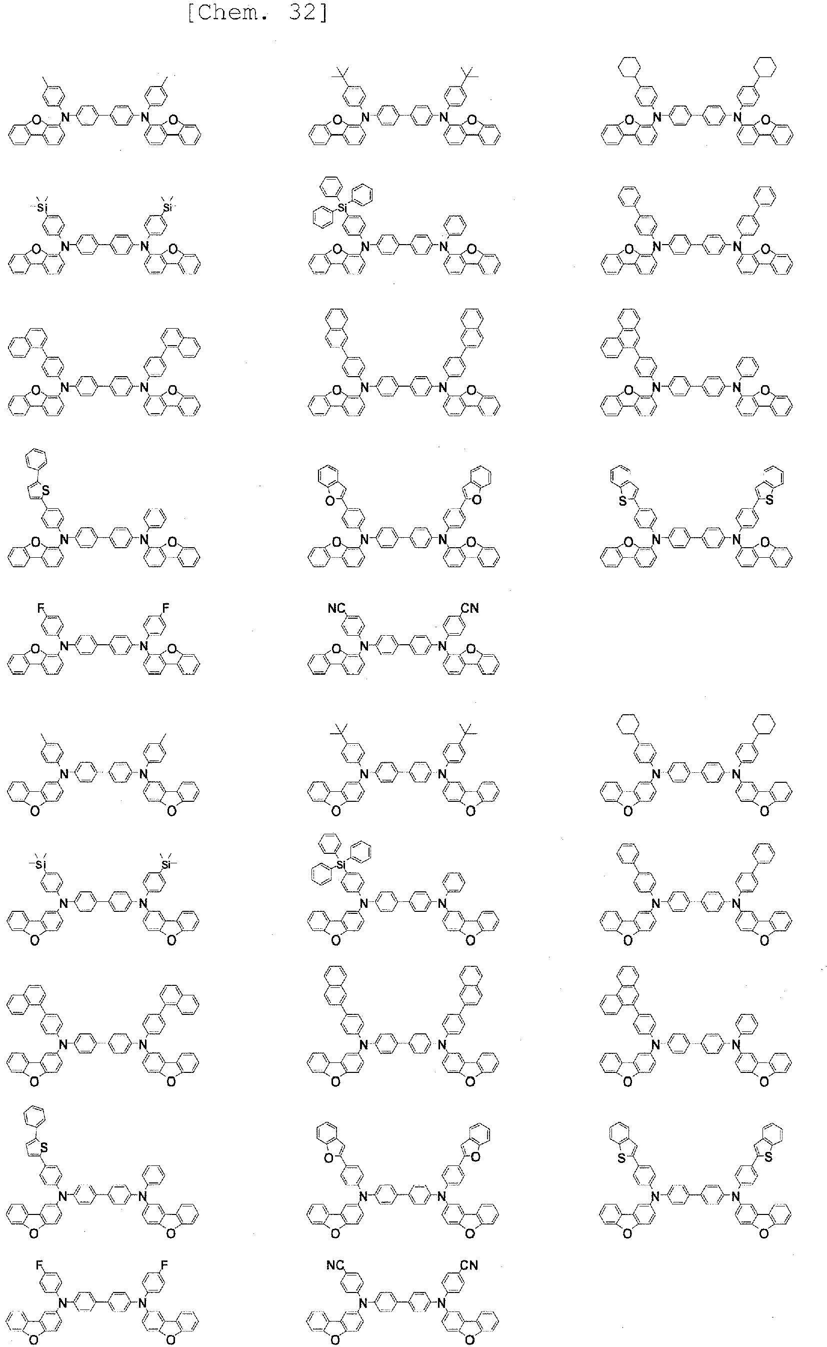

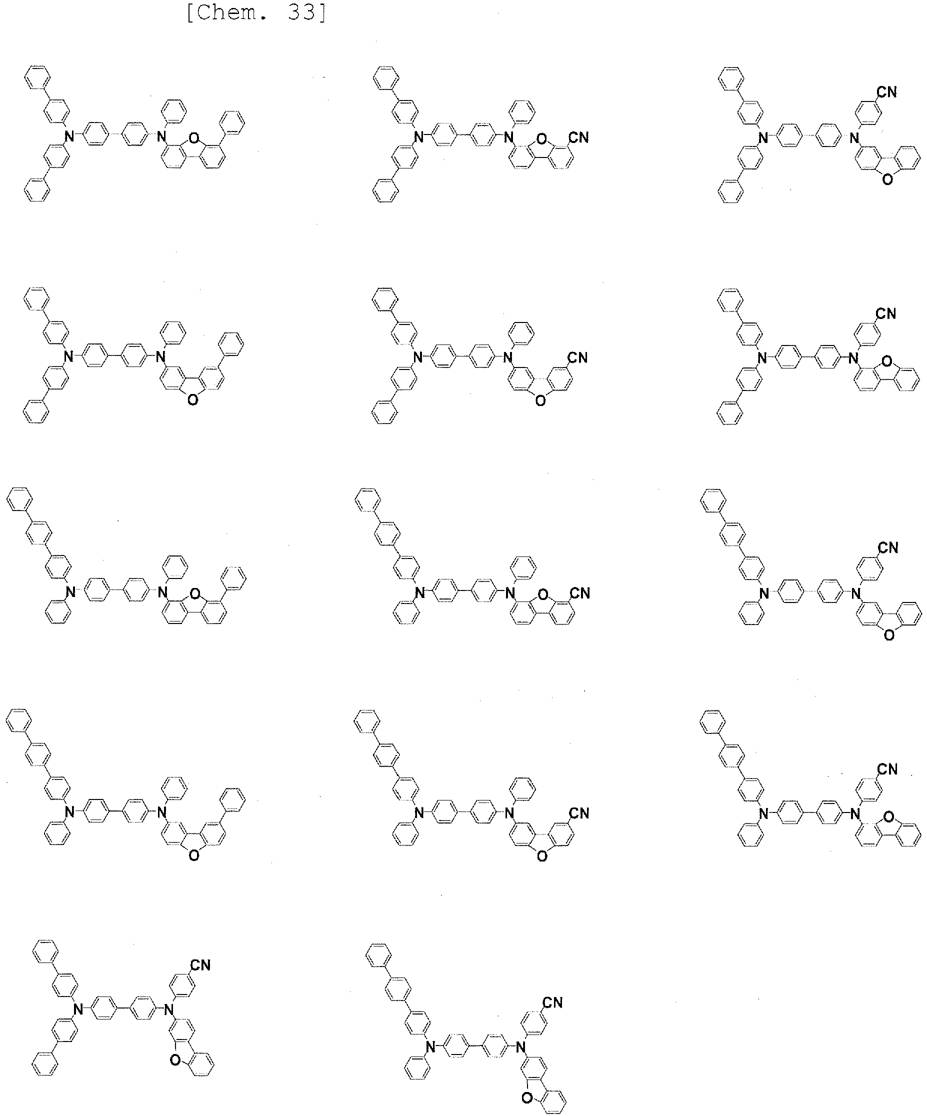

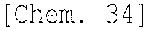

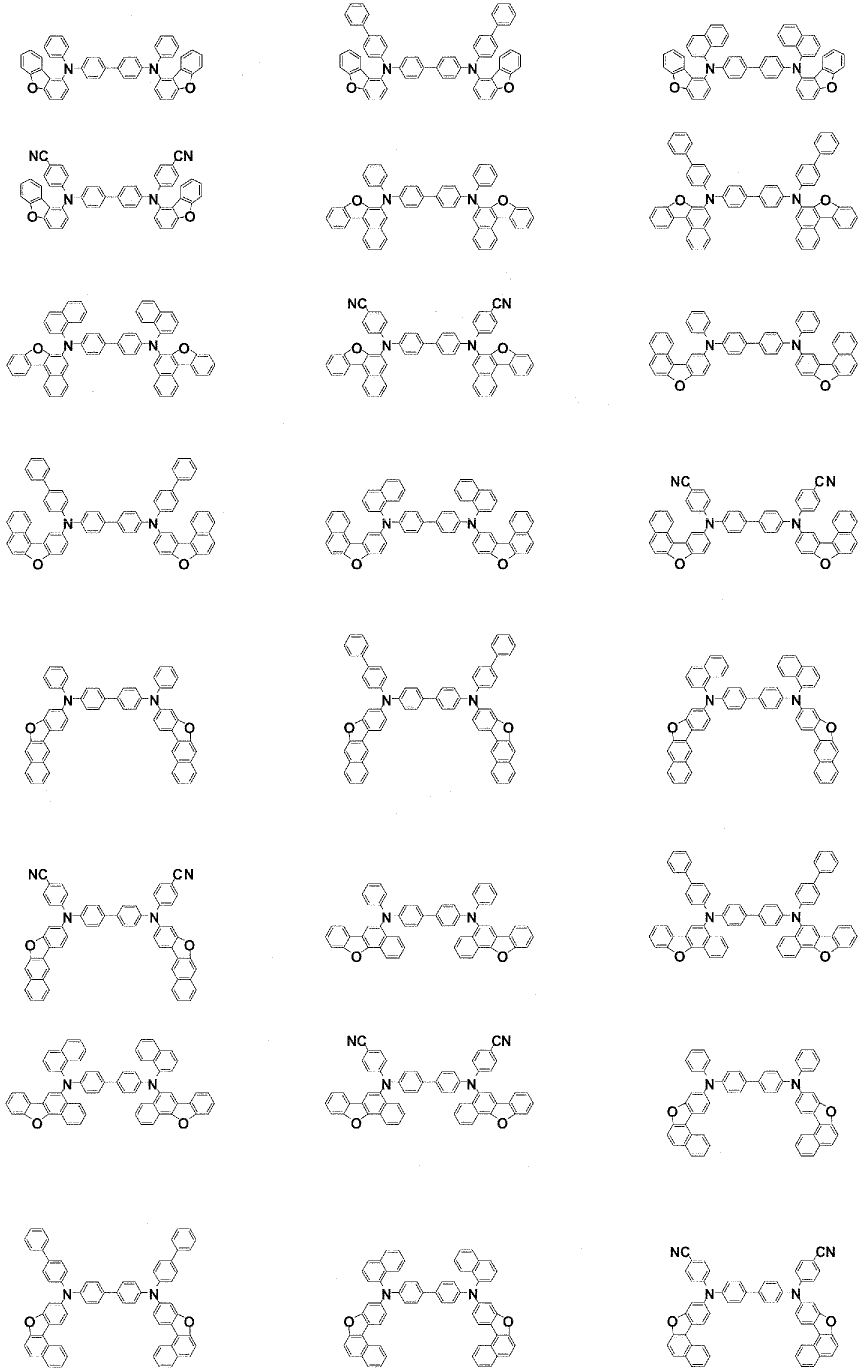

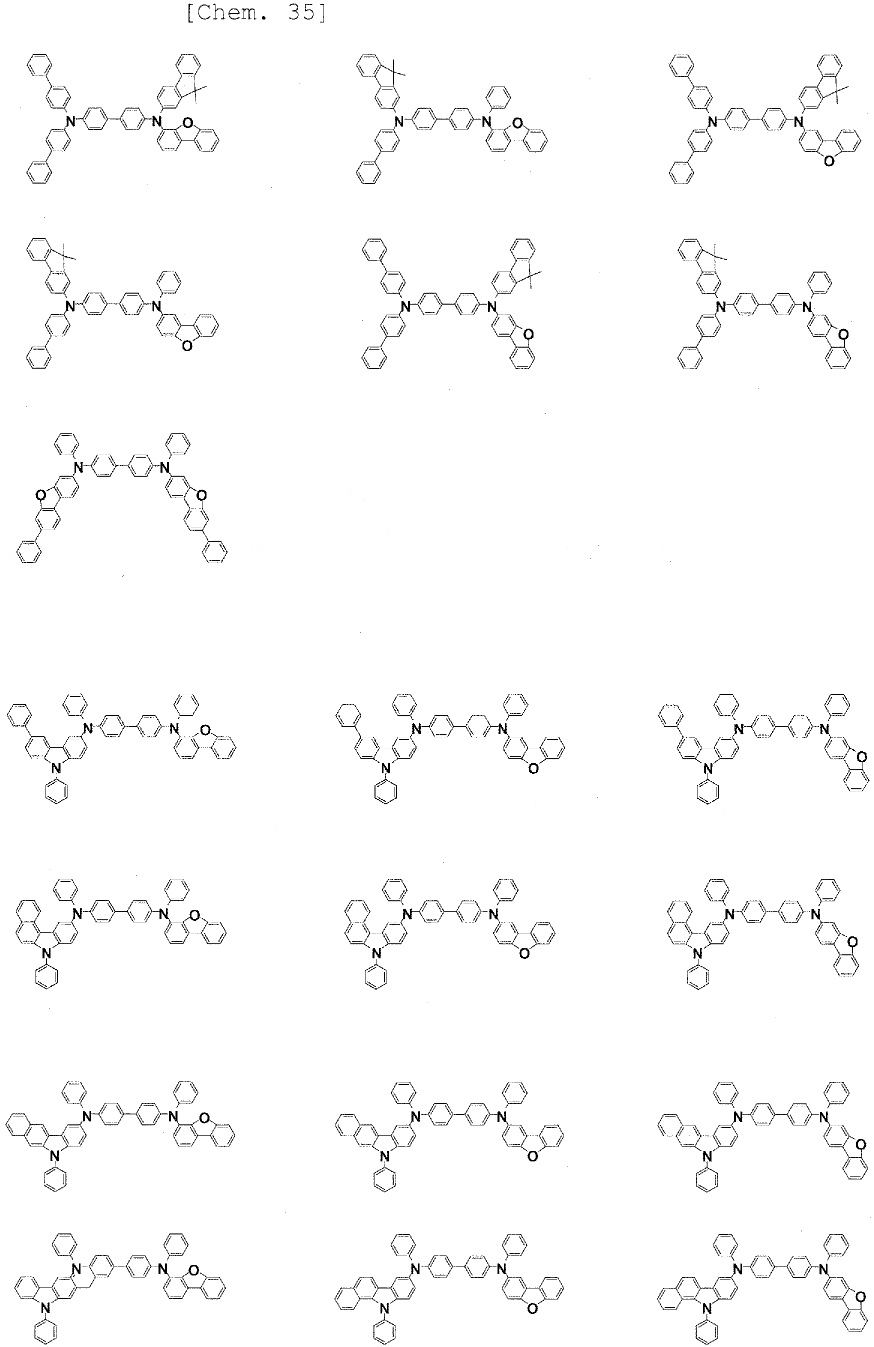

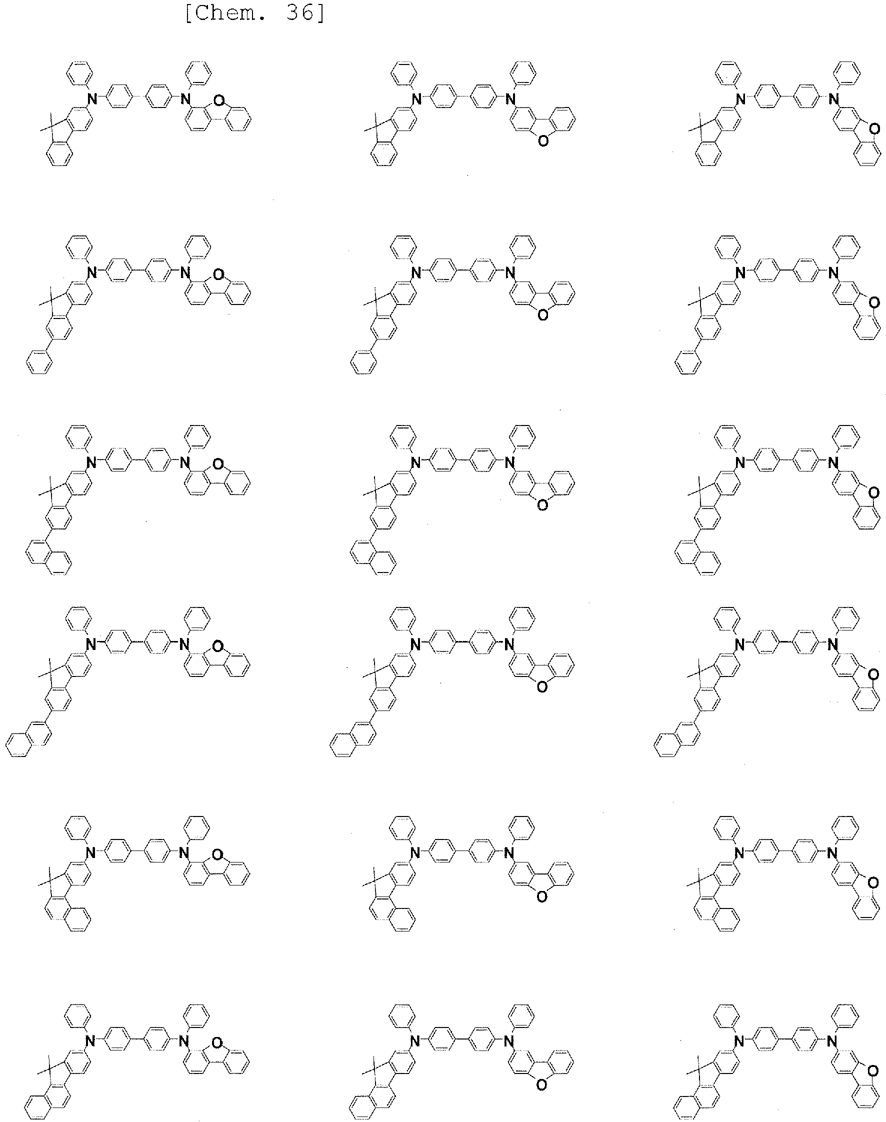

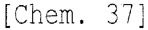

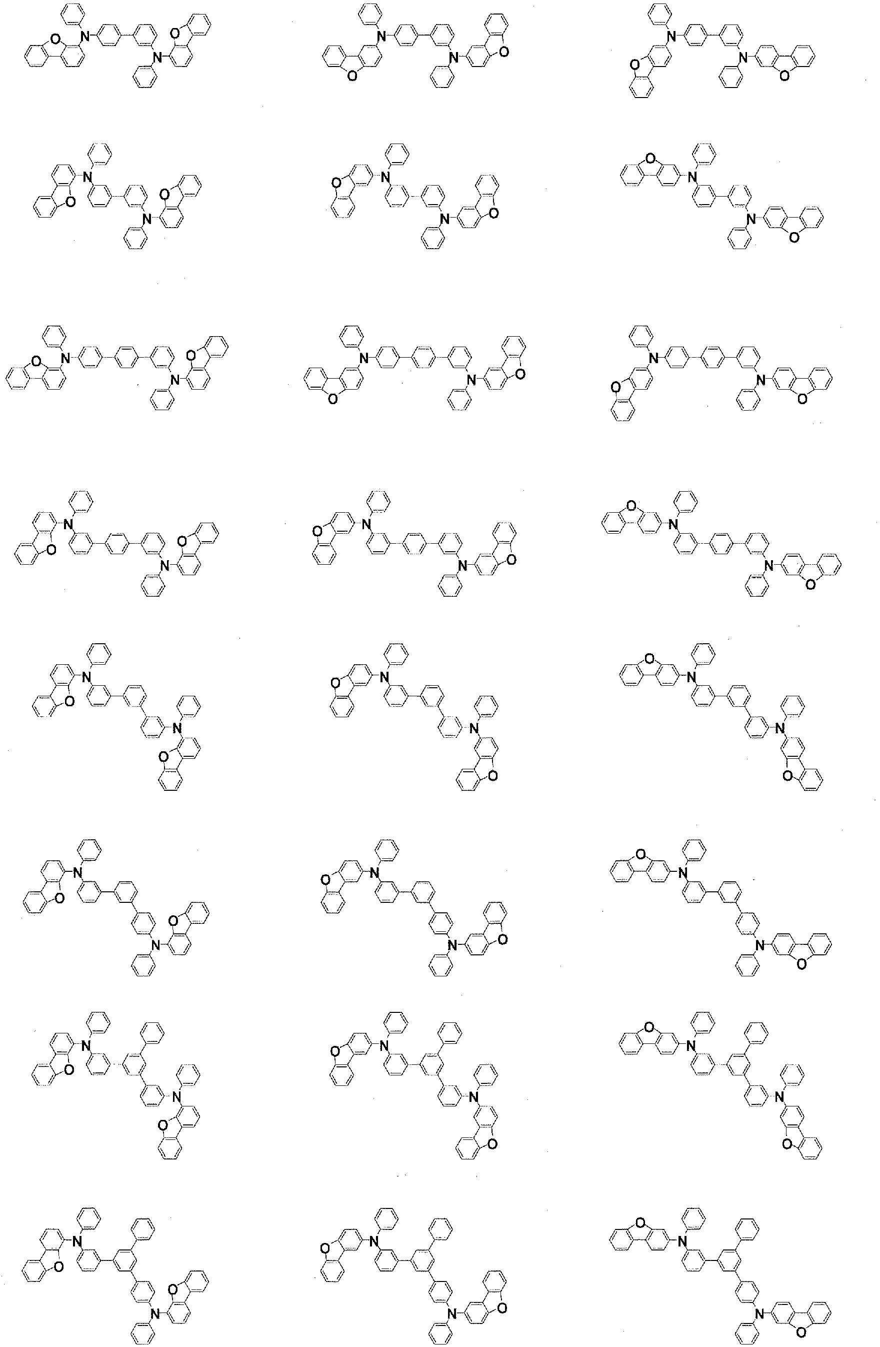

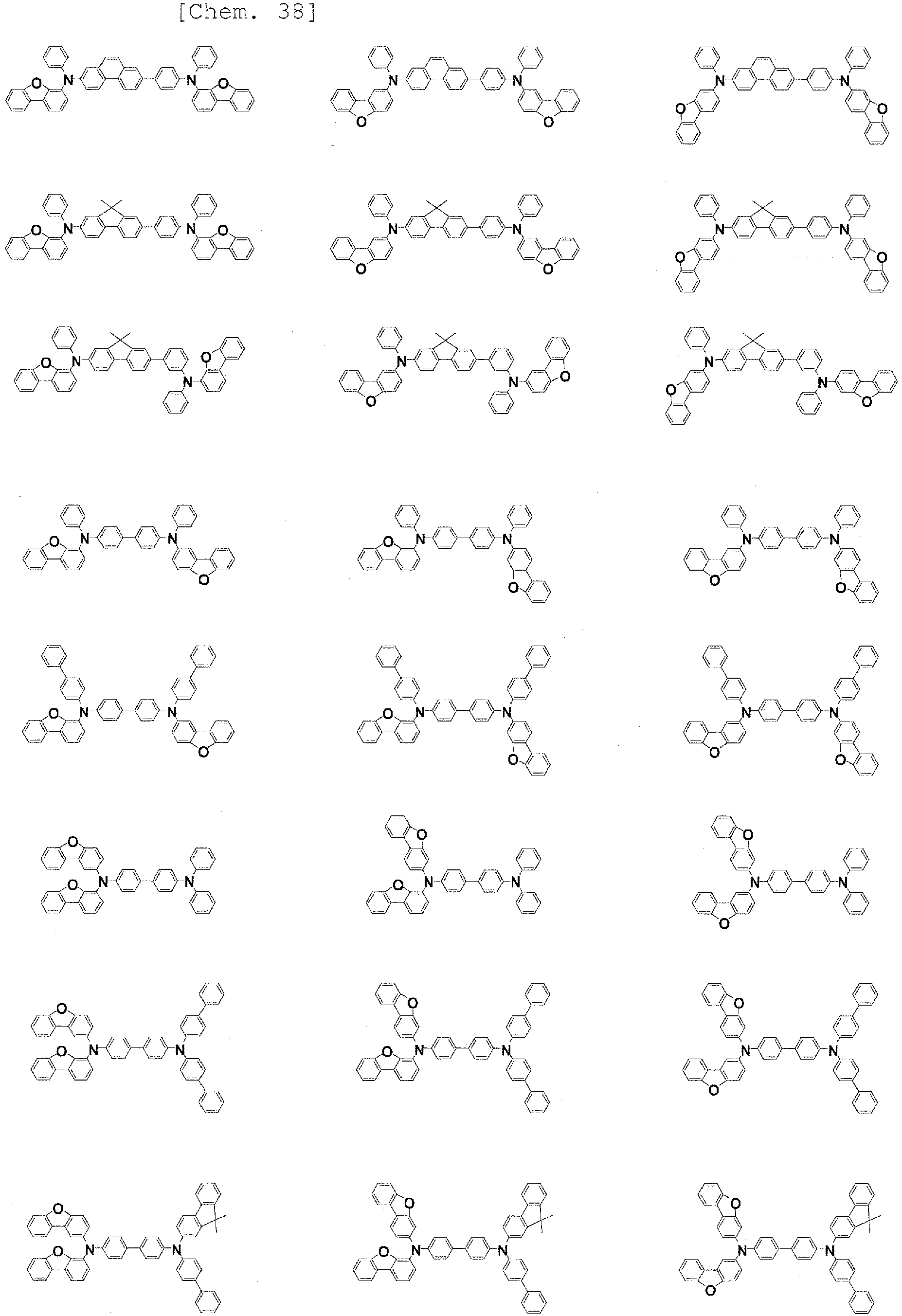

The present invention provides an organic EL device having an organic thin-film layer formed of one or more layers including at least a light emitting layer, the organic thin-film layer being interposed between a cathode and an anode, in which at least one layer of the organic thin-film layer contains the aromatic amine derivative alone or as a component of a mixture.
The structure of the organic EL device of the present invention is described in the following.

### (1) Organic EL device constitution

Typical examples of the constitution of the organic EL device of the present invention include the following:
(1) an anode/light emitting layer/cathode;
(2) an anode/hole injecting layer/light emitting layer/cathode;
(3) an anode/light emitting layer/electron injecting layer/cathode;
(4) an anode/hole injecting layer/light emitting layer/electron injecting layer/cathode;
(5) an anode/organic semiconductor layer/light emitting layer/cathode;
(6) an anode/organic semiconductor layer/electron blocking layer/light emitting layer/cathode;
(7) an anode/organic semiconductor layer/light emitting layer/adhesion improving layer/cathode;
(8) an anode/hole injecting layer/hole transporting layer/light emitting layer/electron injecting layer/cathode;
(9) an anode/insulating layer/light emitting layer/insulating layer/cathode;
(10) an anode/inorganic semiconductor layer/insulating layer/light emitting layer/insulating layer/cathode;
(11) an anode/organic semiconductor layer/insulating layer/light emitting layer/insulating layer/cathode;
(12) an anode/insulating layer/hole injecting layer/hole transporting layer/light emitting layer/insulating layer/cathode; and
(13) an anode/insulating layer/hole injecting layer/hole transporting layer/light emitting layer/electron injecting (transporting) layer/cathode.
Of those, the constitution (8) is preferably used in ordinary cases. However, the constitution is not limited to the foregoing.

The aromatic amine derivative of the present invention is preferably incorporated into the hole transporting layer and/or the hole injecting layer.
In addition, a layer containing an electron-accepting compound is preferably joined to the hole transporting layer and/or the hole injecting layer.
Further, when the layer containing the electron-accepting compound is joined to the layer containing the aromatic amine derivative of the present invention, and the light emitting layer containing a host material and a dopant material that shows light emission is joined on the surface opposite to the layer containing the electron-accepting compound, the simplification of the construction of the device is expected to reduce its driving voltage and production cost.
The electron-accepting compound is preferably a compound having a skeleton having high planarity such as a compound represented by the following formula (A) or (B).

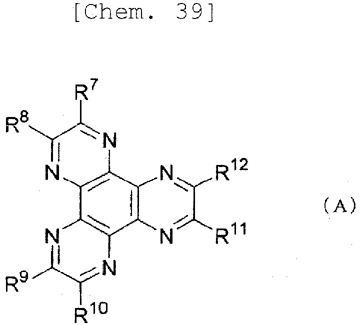

(In the formula (A), R⁷ to R¹² each independently represent a cyano group, -CONH₂, a carboxyl group, or -COOR¹³ (where R¹³ represents an alkyl group having 1 to 20 carbon atoms), or R⁷ and R⁸, R⁹ and R¹⁰, or R¹¹ and R¹² combine with each other to represent a group represented by -CO-O-CO-.)
Examples of the alkyl group represented by R¹³ include a methyl group, an ethyl group, an n-propyl group, an isopropyl group, an n-butyl group, an isobutyl group, a tert-butyl group, a cyclopentyl group, and a cyclohexyl group.

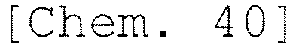

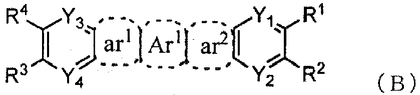

[In the formula (B), Ar¹ represents a fused ring having 6 to 24 ring carbon atoms or a heterocycle having 6 to 24 ring atoms, and ar¹ and ar² may be identical to or different from each other, and are each represented by the following formula (i) or (ii). 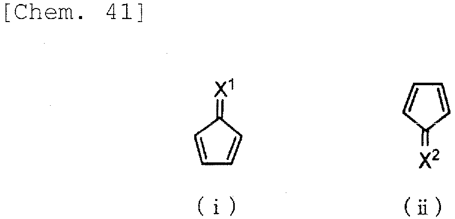 {In the formulae, X¹ and X² may be identical to or different from each other, and each represent any one of the divalent groups represented by the following formulae (a) to (g).

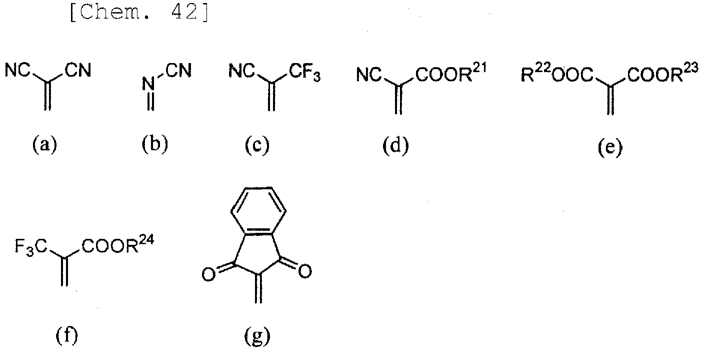

(In the formulae, R²¹ to R²⁴ may be identical to or different from one another, and each represent a hydrogen atom, a substituted or unsubstituted fluoroalkyl group having 1 to 20 carbon atoms, a substituted or unsubstituted alkyl group having 1 to 20 carbon atoms, a substituted or unsubstituted aryl group having 6 to 50 carbon atoms, or a substituted or unsubstituted heterocyclic group having 3 to 50 ring atoms, and R²² and R²³ may be bonded to each other to form a ring.)}
R¹ to R⁴ in the formula (B) may be identical to or different from one another, and each represent a hydrogen atom, a substituted or unsubstituted alkyl group having 1 to 20 carbon atoms, a substituted or unsubstituted aryl group having 6 to 50 carbon atoms, a substituted or unsubstituted heterocyclic group having 3 to 50 ring atoms, a halogen atom, a substituted or unsubstituted fluoroalkyl group having 1 to 20 carbon atoms, a substituted or unsubstituted alkoxy group having 1 to 20 carbon atoms, a substituted or unsubstituted fluoroalkoxy group having 1 to 20 carbon atoms, a substituted or unsubstituted aryloxy group having 6 to 50 carbon atoms, or a cyano group. Substituents adjacent to each other out of R¹ to R⁴ may be bonded to each other to form a ring. Y¹ to Y⁴ may be identical to or different from one another, and each represent -N=, -CH=, or C(R⁵) =, and R⁵ represents a substituted or unsubstituted alkyl group having 1 to 20 carbon atoms, a substituted or unsubstituted aryl group having 6 to 50 carbon atoms, a substituted or unsubstituted heterocyclic group having 3 to 50 ring atoms, a halogen atom, a substituted or unsubstituted fluoroalkyl group having 1 to 20 carbon atoms, a substituted or unsubstituted alkoxy group having 1 to 20 carbon atoms, a substituted or unsubstituted fluoroalkoxy group having 1 to 20 carbon atoms, a substituted or unsubstituted aryloxy group having 6 to 50 carbon atoms, or a cyano group.]

Examples of the respective groups represented by R¹ to R⁵ are as described below.
Examples of the alkyl group include a methyl group, an ethyl group, an n-propyl group, an isopropyl group, an n-butyl group, an isobutyl group, a tert-butyl group, a cyclopentyl group, and a cyclohexyl group.
Examples of the aryl group include a phenyl group, a biphenyl group, a naphthyl group, a fluorophenyl group, and a trifluoromethylphenyl group.
Examples of the heterocyclic group include residues of pyridine, pyrazine, furan, imidazole, benzimidazole, and thiophene.
Examples of the halogen atom include a fluorine atom, a chlorine atom, a bromine atom, and an iodine atom.
Examples of the fluoroalkyl group include a trifluoromethyl group, a pentafluoroethyl group, a perfluorocyclohexyl group, and a perfluoroadamantyl group.
Examples of the alkoxy group and the fluoroalkoxy group include a methoxy group, an ethoxy group, and a trifluoromethoxy group.
Examples of the aryloxy group include a phenyloxy group, a pentaphenyloxy group, and a 4-trifluorophenyloxy group.
In addition, examples of the substituents of those groups include the same examples as those of the halogen atom, the cyano group, the alkyl group, the aryl group, the fluoroalkyl group, and the heterocyclic group listed above.
Substituents adjacent to each other out of R¹ to R⁴ may be bonded to each other to form a ring. Examples of the ring include a benzene ring, a naphthalene ring, a pyrazine ring, a pyridine ring, and a furan ring.

### (2) Light-transmissive substrate

The organic EL device of the present invention is prepared on a light-transmissive substrate. Here, the light-transmissive substrate is the substrate which supports the organic EL device. It is preferred that the light-transmissive substrate have a transmittance of light of 50% or more in the visible region of 400 to 700 nm and be flat and smooth.
Specific examples of the light-transmissive substrate include glass plates and polymer plates . Examples of the glass plate include, in particular, plates formed of soda-lime glass, glass containing barium and strontium, lead glass, aluminosilicate glass, borosilicate glass, barium borosilicate glass, and quartz. Examples of the polymer plate include plates formed of polycarbonate, acrylic, polyethylene terephthalate, polyether sulfide, and polysulfone.

### (3) Anode

The anode of the organic EL device of the present invention has the function of injecting holes into the hole transporting layer or the light emitting layer. It is effective that the anode has a work function of 4.5 eV or more. Specific examples of the material for the anode used in the present invention include indium tin oxide (ITO) alloys, tin oxide (NESA), indium zinc oxide (IZO), gold, silver, platinum, and copper.
The anode can be prepared by forming a thin film of the electrode material described above in accordance with a process such as the vapor deposition process and the sputtering process.
When the light emitted from the light emitting layer is obtained through the anode, it is preferred that the anode have a transmittance of the emitted light higher than 10%. It is also preferred that the sheet resistivity of the anode be several hundred Ω/□ or less. The thickness of the anode is, in general, selected in the range of 10 nm to 1 µ m and preferably in the range of 10 to 200 nm although the preferred range may be different depending on the used material.

### (4) Light emitting layer

The light emitting layer of the organic EL device has a combination of the following functions (1) to (3).
(1) The injecting function: the function of injecting holes from the anode or the hole injecting layer and injecting electrons from the cathode or the electron injecting layer when an electric field is applied.
(2) The transporting function: the function of transporting injected charges (i.e., electrons and holes) by the force of the electric field.
(3) The light emitting function: the function of providing the field for recombination of electrons and holes and leading to the emission of light.
However, the easiness of injection may be different between holes and electrons and the ability of transportation expressed by the mobility may be different between holes and electrons. It is preferred that one of the charges be transferred.
A known method such as a vapor deposition method, a spin coating method, or an LB method is applicable to the formation of the light emitting layer. The light emitting layer is particularly preferably a molecular deposit film. The term "molecular deposit film" as used herein refers to a thin film formed by the deposition of a material compound in a vapor phase state, or a film formed by the solidification of a material compound in a solution state or a liquid phase state. The molecular deposit film can be typically distinguished from a thin film formed by the LB method (molecular accumulation film) on the basis of differences between the films in aggregation structure and higher order structure, and functional differences between the films caused by the foregoing differences.
In addition, as disclosed in JP 57-51781 A, the light emitting layer can also be formed by: dissolving a binder such as a resin and a material compound in a solvent to prepare a solution; and forming a thin film from the prepared solution by the spin coating method or the like.

The aromatic amine derivative of the present invention can be used in the light emitting layer as a light emitting material or a doping material. Other examples of the light emitting material or the doping material include, but not limited to, anthracene, naphthalene, phenanthrene, pyrene, tetracene, coronene, chrysene, fluoresceine, perylene, phthaloperylene, naphthaloperylene, perynone, phthaloperynone, naphthaloperynone, diphenylbutadiene, tetraphenylbutadiene, coumarin, oxadiazole, aldazine, bisbenzoxazoline, bisstyryl, pyrazine, cyclopentadiene, quinoline metal complexes, aminoquinoline metal complexes, benzoquinoline metal complexes, imine, diphenylethylene, vinylanthracene, diaminocarbazole, pyrane, thiopyrane, polymethine, merocyanine, imidazole-chelated oxynoid compounds, quinacridone, rubrene, and fluorescent dyes.

A host material that can be used in a light emitting layer of the organic EL device of the present invention is preferably a compound represented by any one of the following formulae (i) to (ix):
an asymmetric anthracene represented by the following general formula (i):

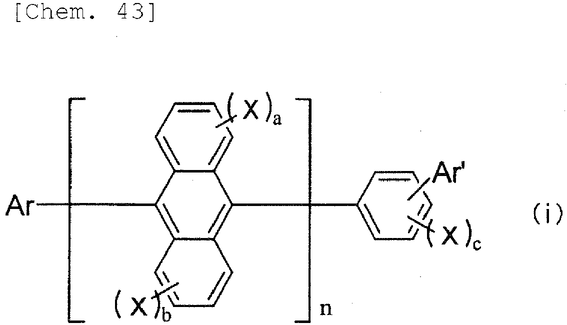

where: Ar represents a substituted or unsubstituted fused aromatic group having 10 to 50 ring carbon atoms;
Ar' represents a substituted or unsubstituted aromatic group having 6 to 50 ring carbon atoms;
X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group, or a hydroxy group;
a, b, and c each represent an integer of 0 to 4; and
n represents an integer of 1 to 3, and when n represents 2 or more, anthracene nuclei in [] may be identical to or different from each other;

an asymmetric monoanthracene derivative represented by the following general formula (ii):

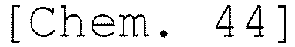

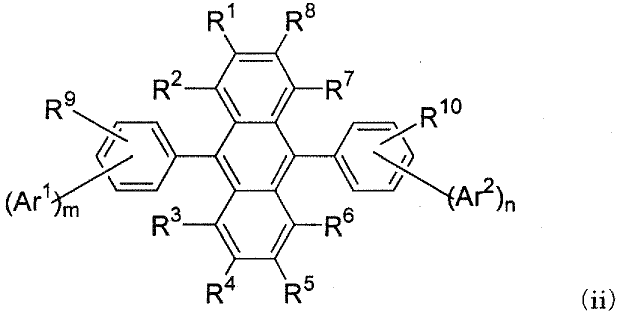

where: Ar¹ and Ar² each independently represent a substituted or unsubstituted aromatic ring group having 6 to 50 ring carbon atoms; m and n each represent an integer of 1 to 4; provided that Ar¹ and Ar² are not identical to each other when m=n=1 and positions at which Ar¹ and Ar² are bound to a benzene ring are bilaterally symmetric, and m and n represent different integers when m or n represents an integer of 2 to 4; and
R¹ to R¹⁰ each independently represents a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxy group;

an asymmetric pyrene derivative represented by the following general formula (iii):

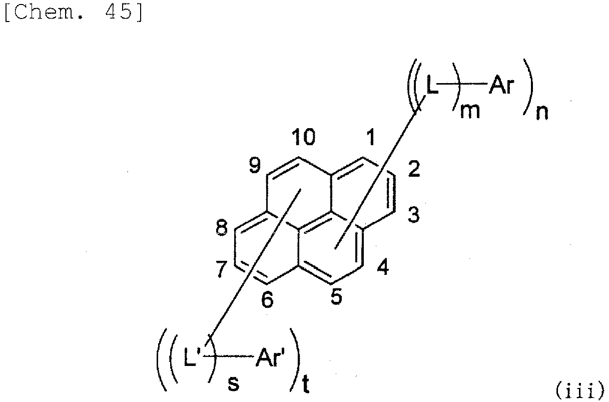

where: Ar and Ar' each represent a substituted or unsubstituted aromatic group having 6 to 50 ring carbon atoms;
L and L' each represent a substituted or unsubstituted phenylene group, a substituted or unsubstituted naphthalenylene group, a substituted or unsubstituted fluorenylene group, or a substituted or unsubstituted dibenzosilolylene group;
m represents an integer of 0 to 2; n represents an integer of 1 to 4; s represents an integer of 0 to 2; t represents an integer of 0 to 4; and
in addition, L or Ar binds to any one of 1- to 5-positions of pyrene, and L' or Ar' binds to any one of 6- to 10-positions of pyrene,
provided that Ar, Ar', L, and L' satisfy the following item (1) or (2) when n + t represents an even number:
(1) Ar≠Ar' and/or L* L' (where the symbol "≠ " means that groups connected with the symbol have different structures); and
(2) when Ar=Ar' and L=L',
   (2-1) m≠ s and/or n≠ t, or
   (2-2) when m=s and n=t,
      (2-2-1) in the case where L and L' (or pyrene) bind (or binds) to different binding positions on Ar and Ar', or (2-2-2) in the case where L and L' (or pyrene) bind (or binds) to the same binding positions on Ar and Ar', the case where the substitution positions of L and L', or of Ar and Ar' in pyrene are 1- and 6-positions, or 2- and 7-positions does not occur;

an asymmetric anthracene derivative represented by the following general formula (iv):

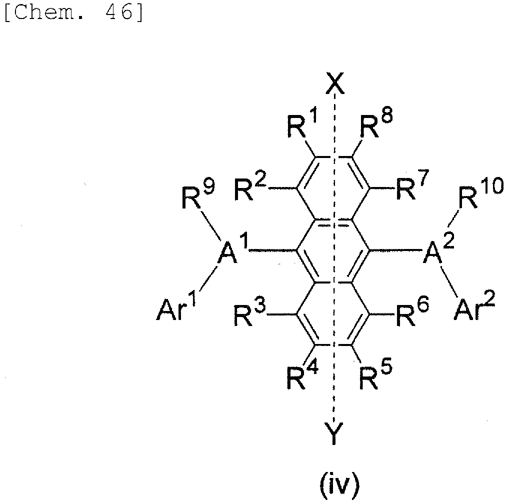

where: A¹ and A² each independently represent a substituted or unsubstituted fused aromatic ring group having 10 to 20 ring carbon atoms;
Ar¹ and Ar² each independently represent a hydrogen atom, or a substituted or unsubstituted aromatic ring group having 6 to 50 ring carbon atoms;
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxy group; and
the number of each of Ar¹, Ar², R⁹, and R¹⁰ may be two or more, and adjacent groups may form a saturated or unsaturated cyclic structure,
provided that the case where groups symmetric with respect to the X-Y axis shown on central anthracene in the general formula (1) bind to 9- and 10-positions of the anthracene does not occur;

an anthracene derivative represented by the following general formula (v):

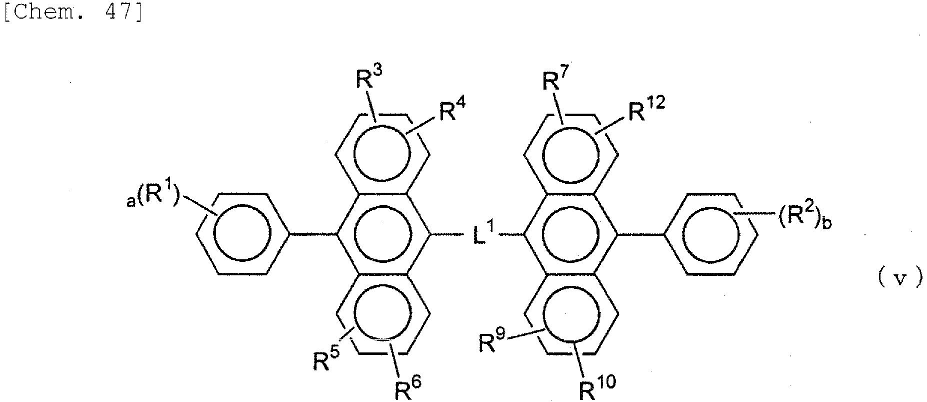

where: R¹ to R¹⁰ each independently represent a hydrogen atom, an alkyl group, a cycloalkyl group, an aryl group which may be substituted, an alkoxyl group, an aryloxy group, an alkylamino group, an alkenyl group, an arylamino group, or a heterocyclic group which may be substituted; a and b each represent an integer of 1 to 5, and, when a or b represents 2 or more, R¹'s or R²'s may be identical to or different from each other, or R¹'s or R²'s may be bonded to each other to form a ring; R³ and R⁴, R⁵ and R⁶, R⁷ and R⁸, or R⁹ and R¹⁰ may be bonded to each other to form a ring; and L¹ represents a single bond, -O-, -S-, -N(R)- (where R represents an alkyl group or an aryl group which may be substituted), an alkylene group, or an arylene group;

an anthracene derivative represented by the following general formula (vi):

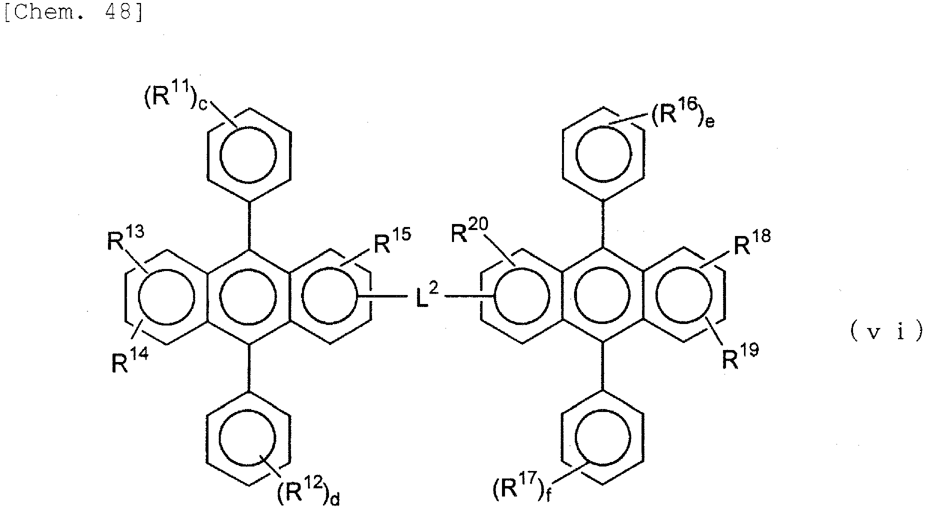

where: R¹¹ to R²⁰ each independently represent a hydrogen atom, an alkyl group, a cycloalkyl group, an aryl group, an alkoxyl group, an aryloxy group, an alkylamino group, an arylamino group, or a heterocyclic group which may be substituted; c, d, e, and f each represent an integer of 1 to 5, and, when any one of c, d, e, and f represents 2 or more, R¹¹'s, R¹²'s, R¹⁶, or R¹⁷'s may be identical to or different from each other, or R¹¹'s, R¹²'s, R¹⁶'s, or R¹⁷'s may be bonded to each other to form a ring; R¹³ and R¹⁴, or R¹⁸ and R¹⁹ may be bonded to each other to form a ring; and L² represents a single bond, -O-, -S-, -N(R)- (where R represents an alkyl group or an aryl group which may be substituted), an alkylene group, or an arylene group;

a spirofluorene derivative represented by the following general formula (via):

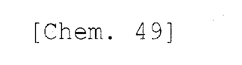

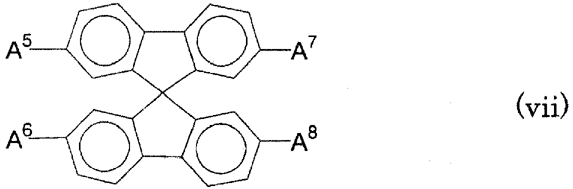

where: A⁵ to A⁸ each independently represent a substituted or unsubstituted biphenylyl group, or a substituted or unsubstituted naphthyl group;

a fused ring-containing compound represented by the following general formula (viii):

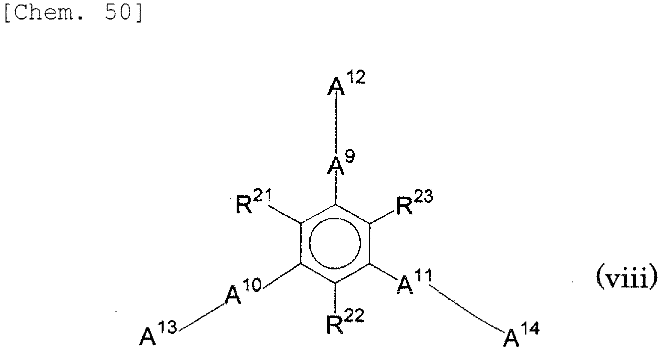

where: A⁹ to A¹⁴ each have the same meaning as that described above; R²¹ to R²³ each independently represent a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms, or a halogen atom; and at least one of A⁹ to A¹⁴ represents a group having three or more fused aromatic rings; and

fluorene compound represented by the following general formula (ix):

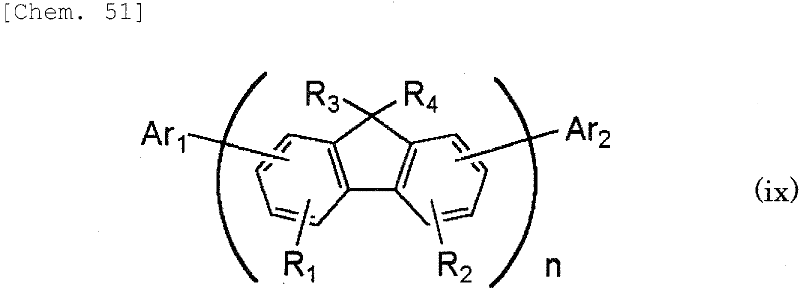

where: R₁ and R₂ each represent a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group, or a halogen atom; R₁'s or R₂'s bonded to different fluorene groups may be identical to or different from each other, and R₁ and R₂ bonded to the same fluorene group may be identical to or different from each other; R₃ and R₄ each represent a hydrogen atom, a substituted or unsubstituted alkyl group, a substituted or unsubstituted aralkyl group, a substituted or unsubstituted aryl group, or a substituted or unsubstituted heterocyclic group; R₃'s or R₄'s bonded to different fluorene groups may be identical to or different from each other, and R₃ and R₄ bonded to the same fluorene group may be identical to or different from each other; Ar₁ and Ar₂ each represent a substituted or unsubstituted fused polycyclic aromatic group having three or more benzene rings in total, or a substituted or unsubstituted fused polycyclic heterocyclic group that has three or more rings each of which is a benzene ring or a heterocyclic ring in total and that is bonded to a fluorene group by carbon, and Ar₁ and Ar₂ may be identical to or different from each other; and n represents an integer of 1 to 10.

Of the above-mentioned host materials, an anthracene derivative is preferred, a monoanthracene derivative is more preferred, and an asymmetric anthracene is particularly preferred.
In addition, a phosphorescent compound can also be used as a light emitting material of a dopant. A compound containing a carbazole ring in a host material is preferred as the phosphorescent compound. The dopant is a compound capable of emitting light from a triplet exciton, and is not particularly limited as long as light is emitted from a triplet exciton, a metal complex containing at least one metal selected from the group consisting of Ir, Ru, Pd, Pt, Os, and Re is preferred, and a porphyrin metal complex or an orthometalated metal complex is preferred.
Any one of various ligands can be used for forming an orthometalated metal complex. Examples of a preferred ligand include 2-phenylpyridine derivatives, 7,8-benzoquinoline derivatives, 2-(2-thienyl)pyridine derivatives, 2-(1-naphthyl)pyridine derivatives, and 2-phenylquinoline derivatives. Each of those derivatives may have a substituent as required. A fluoride of any one of those derivatives, or one obtained by introducing a trifluoromethyl group into any one of those derivatives is a particularly preferred blue-based dopant. The metal complex may further include a ligand other than the above-mentioned ligands such as acetylacetonato or picric acid as an auxiliary ligand.
The content of the phosphorescent dopant in the light emitting layer is not particularly limited, and can be appropriately selected in accordance with a purpose. The content is, for example, 0.1 to 70 mass%, and is preferably 1 to 30 mass%. When the content of the phosphorescent compound is less than 0.1 mass%, the intensity of emitted light is weak, and an effect of the incorporation of the compound is not sufficiently exerted. When the content exceeds 70 mass%, a phenomenon referred to as concentration quenching becomes remarkable, and device performance reduces.
A host suitable for the phosphorescent compound formed of a compound containing a carbazole ring is a compound having a function of causing the phosphorescent compound to emit light as a result of the occurrence of energy transfer from its excited state to the phosphorescent compound. The host compound is not particularly limited as long as the compound can transfer exciton energy to the phosphorescent compound, and can be appropriately selected depending on purposes. The compound may have, for example, an arbitrary heterocycle as well as the carbazole ring.

Specific examples of the host compound include carbazole derivatives, triazole derivatives, oxazole derivatives, oxadiazole derivatives, imidazole derivatives, polyarylalkane derivatives, pyrazoline derivatives, pyrazolone derivatives, phenylene diamine derivatives, arylamine derivatives, amino substituted chalcone derivatives, styrylanthracene derivatives, fluorenone derivatives, hydrazonederivatives, stilbene derivatives, silazane derivatives, aromatic tertiary amine compounds, styrylamine compounds, aromatic dimethylidene-based compounds, porphyrin-based compounds, anthraquinodimethane derivatives, anthrone derivatives, diphenylquinone derivatives, thiopyranedioxide derivatives, carbodiimide derivatives, fluorenilidene methane derivatives, distyryl pyrazine derivatives, heterocyclic tetracarboxylic anhydrides such as naphthaleneperylene, phthalocyanine derivatives, various metal complex polysilane-based compounds typified by a metal complex of an 8-quinolinol derivative or a metal complex having metal phthalocyanine, benzooxazole, or benzothiazole as a ligand, poly(N-vinylcarbazole) derivatives, aniline-based copolymers, conductive high molecular weight oligomers such as a thiophene oligomer and polythiophene, polymer compounds such as polythiophene derivatives, polyphenylene derivatives, polyphenylene vinylene derivatives, and polyfluorene derivatives. One of the host materials may be used alone, or two or more of them may be used in combination.
Specific examples thereof include the compounds as described below.

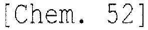

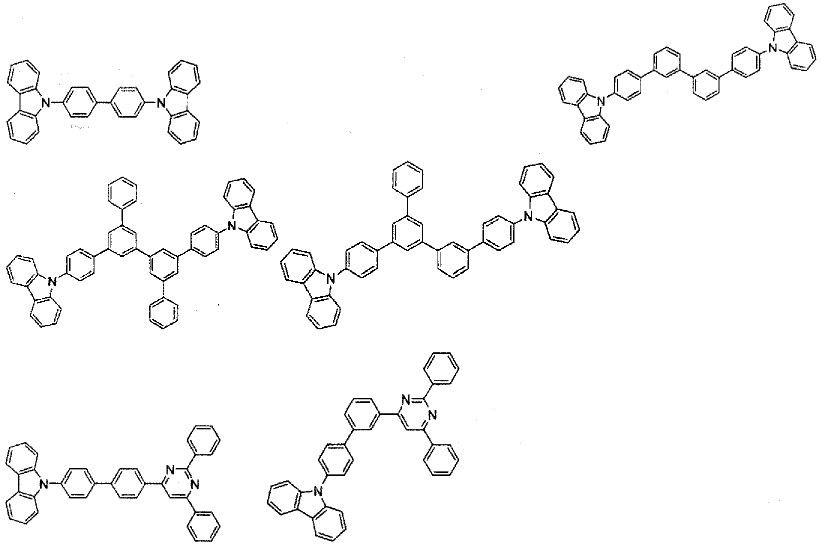

In addition, the light emitting layer may contain a hole transporting material, an electron transporting material, or a polymer binder as required.
Further, the light emitting layer has a thickness of preferably 5 to 50 nm, more preferably 7 to 50 nm, most preferably 10 to 50 nm. When the thickness is less than 5 nm, there is a possibility that the light emitting layer becomes difficult to form and hence chromaticity adjustment becomes difficult. When the thickness exceeds 50 nm, the driving voltage may increase.

Further, when the light emitting layer containing the host material and the dopant material that shows light emission is joined to the layer containing the aromatic amine derivative of the present invention, the simplification of the construction of the device is expected to reduce its driving voltage and production cost.
The dopant material in this case is preferably a metal complex compound containing a metal selected from Ir, Pt, Os, Cu, Ru, Re, and Au, and is preferably a metal complex compound having a partial structure represented by any one of the following formulae (21) to (29) or a tautomer thereof.

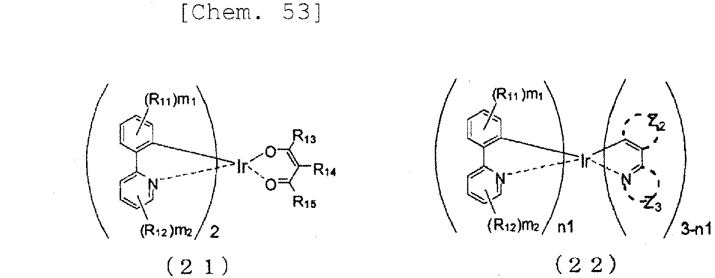

(In the formulae:
R₁₁ and R₁₂ each independently represent an alkyl group having 1 to 20 carbon atoms, an alkenyl group having 1 to 20 carbon atoms, an aryl group having 6 to 25 ring carbon atoms, a heteroaryl group having 5 to 25 ring atoms, a halogen atom, or a cyano group, and a plurality of R₁₁'s or R₁₂'s adjacent to each other may each independently form a saturated or unsaturated, divalent group;
R₁₃ to R₁₅ each independently represent a hydrogen atom or an alkyl group having 1 to 20 carbon atoms;
Z₂ represents an atomic group that forms an aryl ring having 6 to 25 ring carbon atoms or a heteroaryl ring having 5 to 25 ring atoms, and Z₃ represents an atomic group that forms a nitrogen-containing heteroaryl ring having 5 to 25 ring atoms;
m₁ and m₂ each independently represent an integer of 0 to 4; and
n₁ represents an integer of 1 to 3.)

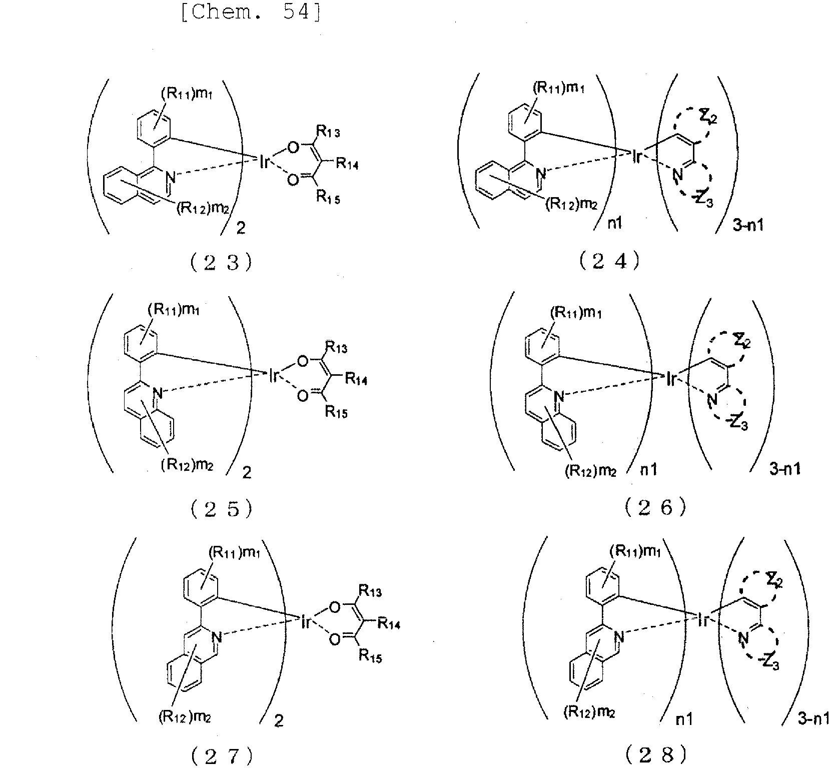

(In the formulae (23) to (28), R₁₁ to R₁₅, Z₂, Z₃, m₁, m₂, and n₁ each have the same meaning as that used in each of the formulae (21) and (22).)

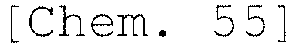

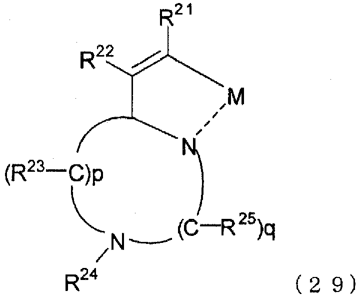

(In the formula:
R²¹ to R²⁵ each independently represent a hydrogen atom, a cyano group, a nitro group, a halogen atom, a substituted or unsubstituted alkyl group having 1 to 20 carbon atoms, a substituted or unsubstituted amino group, a substituted or unsubstituted alkoxyl group having 1 to 20 carbon atoms, a substituted or unsubstituted alkylsilyl group having 1 to 20 carbon atoms, a substituted or unsubstituted acyl group having 1 to 20 carbon atoms, or a substituted or unsubstituted aromatic group having 1 to 30 carbon atoms, and R²¹ and R²², R²³ and R²⁴, or R²⁴ and R²⁵ may be bonded to each other to form a ring structure;
p and q each represent an integer of 0 to 3, p+q represents 2 or 3, when p represents an integer of 2 or more, a plurality of R²³'s may be bonded to each other to form a ring structure, and when q represents an integer of 2 or more, a plurality of R²⁵'s may be bonded to each other to form a ring structure; and
M represents a metal atom selected from iridium (Ir), rhodium (Rh), platinum (Pt), and palladium (Pd).)
Specific examples of such dopantmaterial include the following compounds as well as PQIr (iridium (III) bis (2-phenyl quinolyl-N,C^{2'}) acetylacetonate) and Ir(ppy)₃ (fac-tris(2-phenylpyridine) iridium).

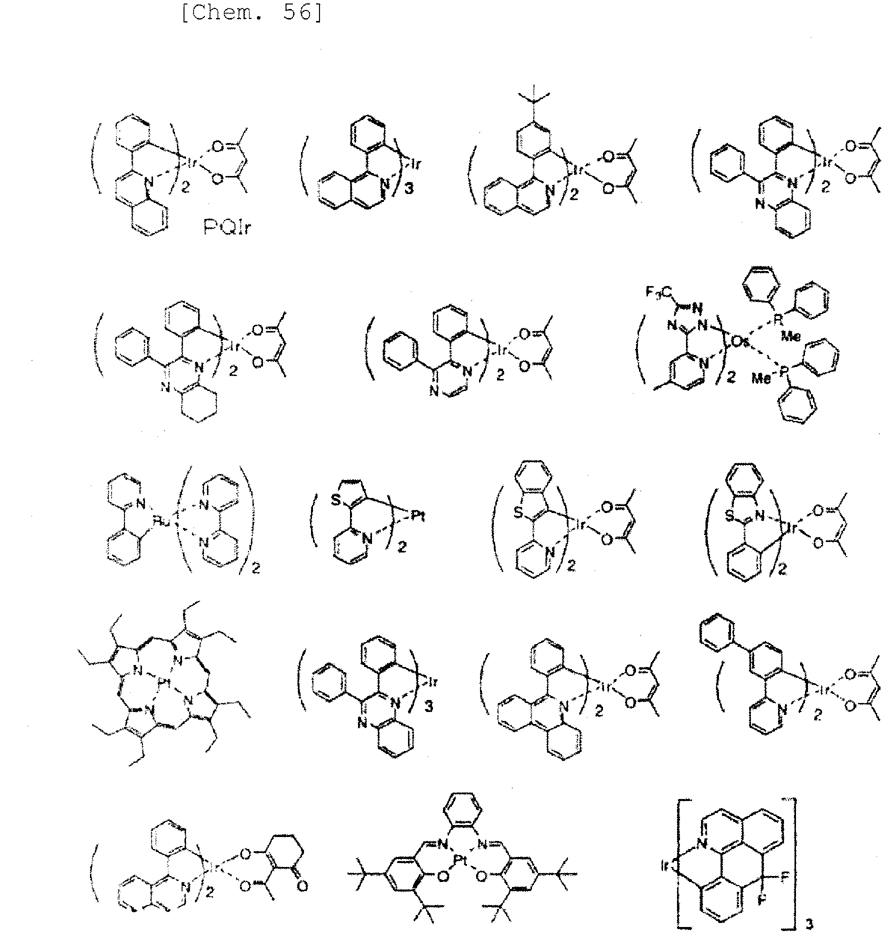

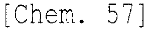

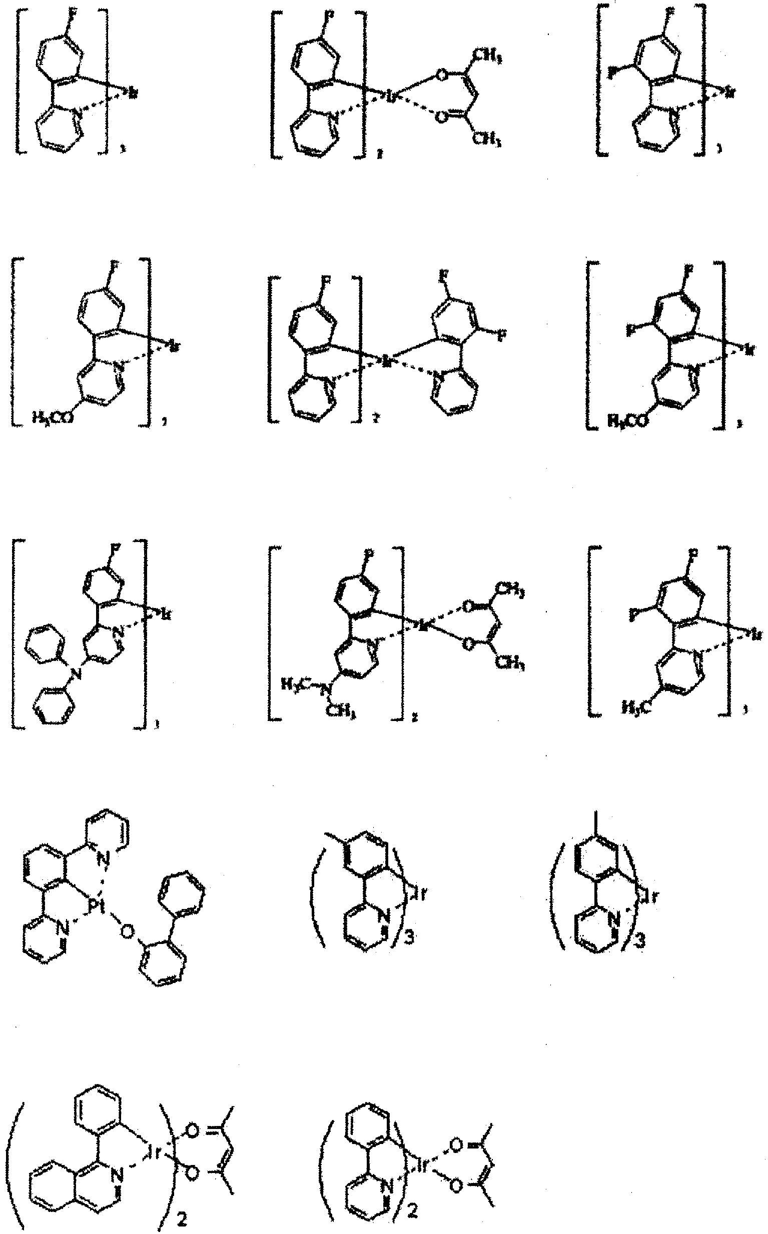

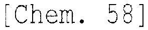

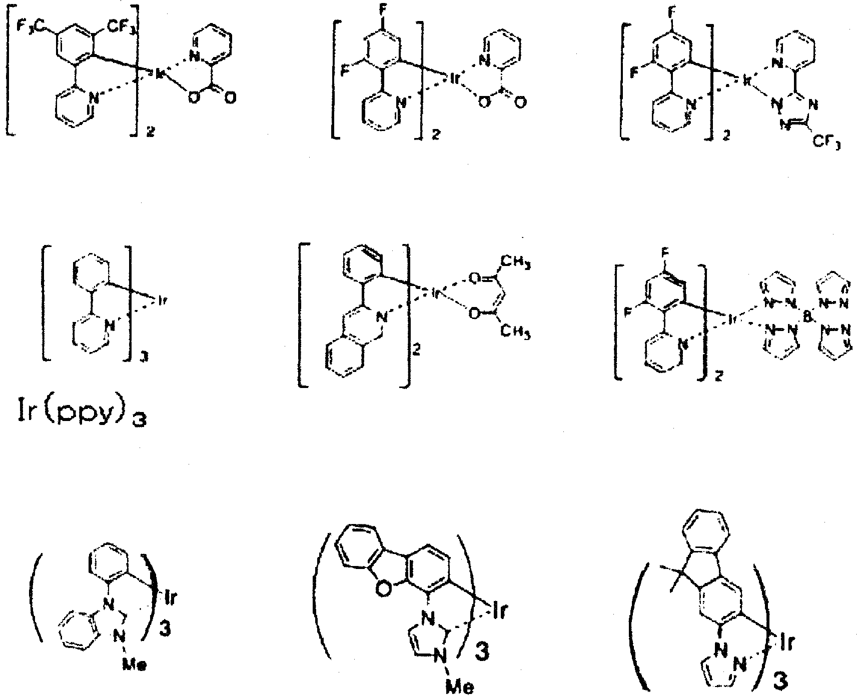

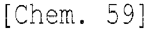

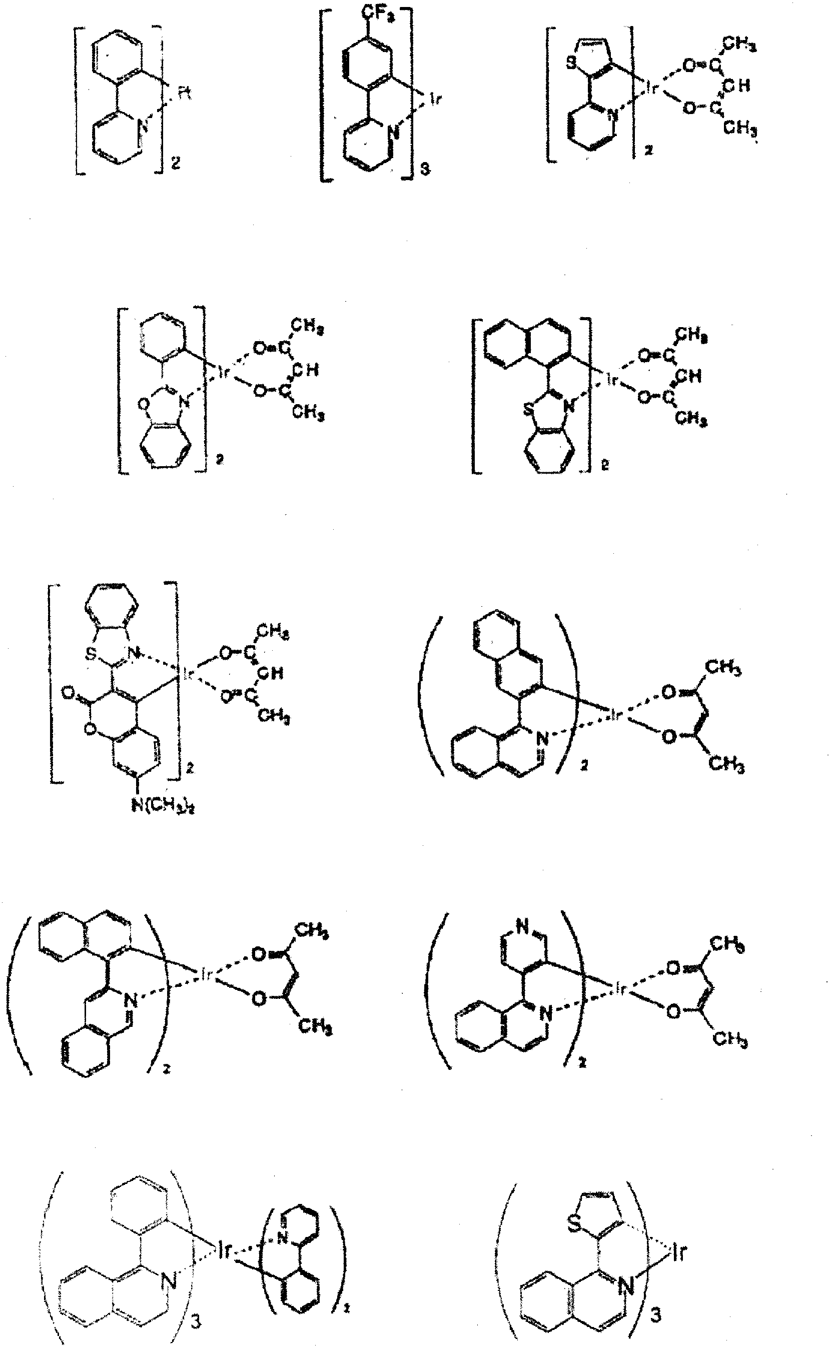

In the present invention, the wavelength at which the phosphorescent dopant shows the maximum emission luminance is preferably 470 nm or more and 700 nm or less, more preferably 480 nm or more and 700 nm or less, particularly preferably 500 nm or more and 650 nm or less.
A high-efficiency organic EL device can be obtained by doping the host of the present invention with the phosphorescent dopant having such luminous wavelength to construct the phosphorescent light emitting layer.

In addition, the host material in this case is preferably a compound having a substituted or unsubstituted, polycyclic, fused aromatic skeleton portion, and the polycyclic, fused aromatic skeleton portion is preferably selected from the group consisting of substituted or unsubstituted phenanthrenediyl, chrysenediyl, fluoranthenediyl, and triphenylenediyl groups.
The polycyclic, fused aromatic skeleton portion is preferably a compound represented by any one of the following formulae (12) to (15).

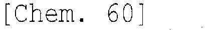

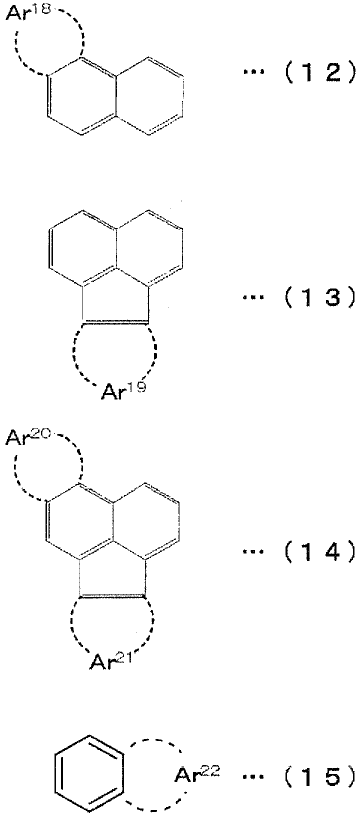

(Inthe formulae, Ar¹⁸ to Ar²² each represent a substituted or unsubstituted fused ring structure having 4 to 10 ring carbon atoms.)
Thepolycyclic, fused aromatic skeleton portion of the compound having the polycyclic, fused aromatic skeleton portion is preferably substituted with a group having phenanthrene, chrysene, fluoranthene, or triphenylene.

Examples of the compound represented by the formula (12) include substituted or unsubstituted phenanthrene and chrysene.
Examples of the compound represented by the formula (13) include substituted or unsubstituted acenaphthylene, acenaphthene and fluoranthene.
The compound represented by the formula (14) is, for example, a substituted or unsubstituted benzofluoranthene.
The compound represented by the formula (15) is, for example, a substituted or unsubstituted naphthalene.

In the present invention, the polycyclic, fused aromatic skeleton portion is preferably a phenanthrene simple substance represented by the following formula (50) or a derivative thereof.

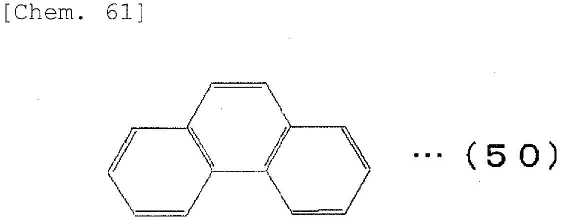

A substituent for the phenanthrene derivative is, for example, an alkyl group, a cycloalkyl group, an aralkyl group, an alkenyl group, a cycloalkenyl group, an alkynyl group, a hydroxyl group, a mercapto group, an alkoxy group, an alkylthio group, an arylether group, an arylthioether group, an aryl group, a heterocyclic group, a halogen, a haloalkane, a haloalkene, a haloalkyne, a cyano group, an aldehyde group, a carbonyl group, a carboxyl group, an ester group, an amino group, a nitro group, a silyl group, or a siloxanyl group.
Such phenanthrene derivative is, for example, a derivative represented by the following formula (50A).

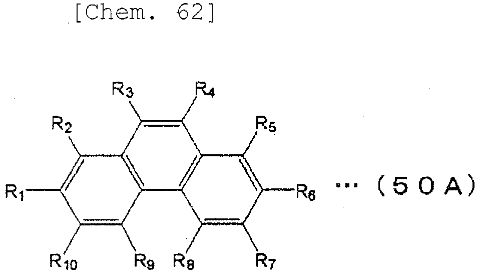

In the formula (50A), R₁ to R₁₀ each independently represent a hydrogen atom, or a substituent constructed of one, or a combination of two or more, of a substituted or unsubstituted aryl group having 5 to 30 ring carbon atoms, a branched or linear alkyl group having 1 to 30 carbon atoms, and a substituted or unsubstituted cycloalkyl group having 3 to 20 carbon atoms.
Specific examples of the phenanthrene derivative represented by the formula (50) include the following.

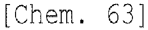

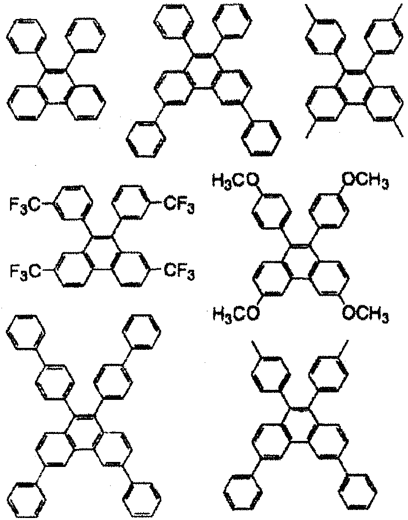

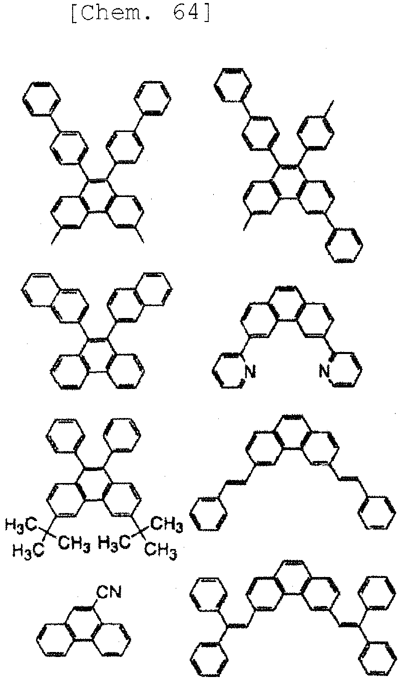

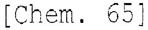

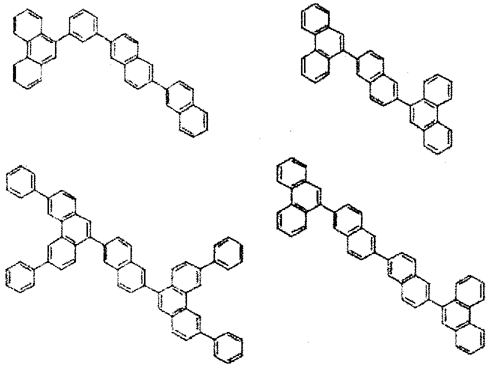

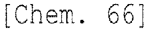

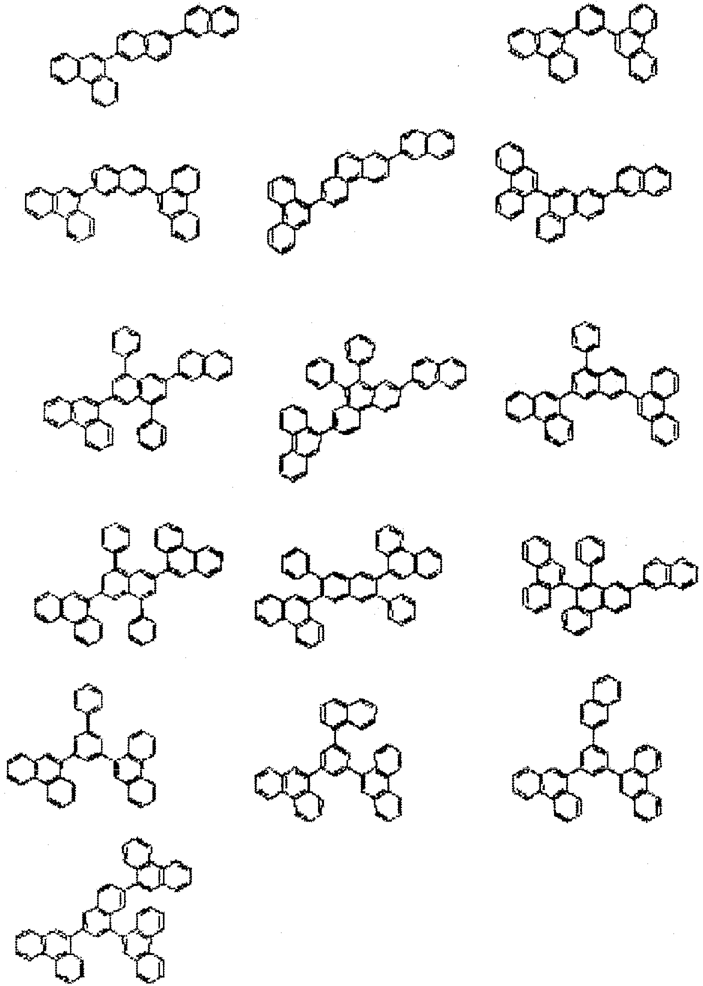

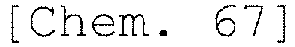

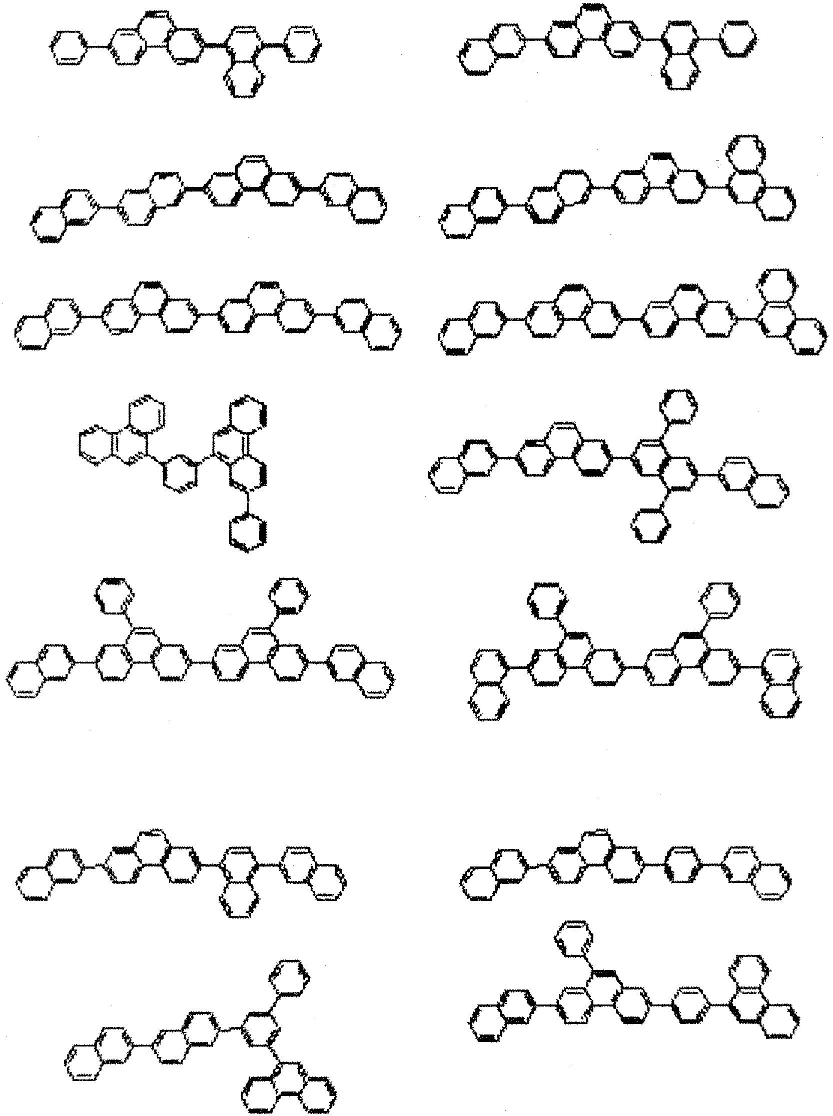

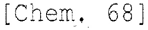

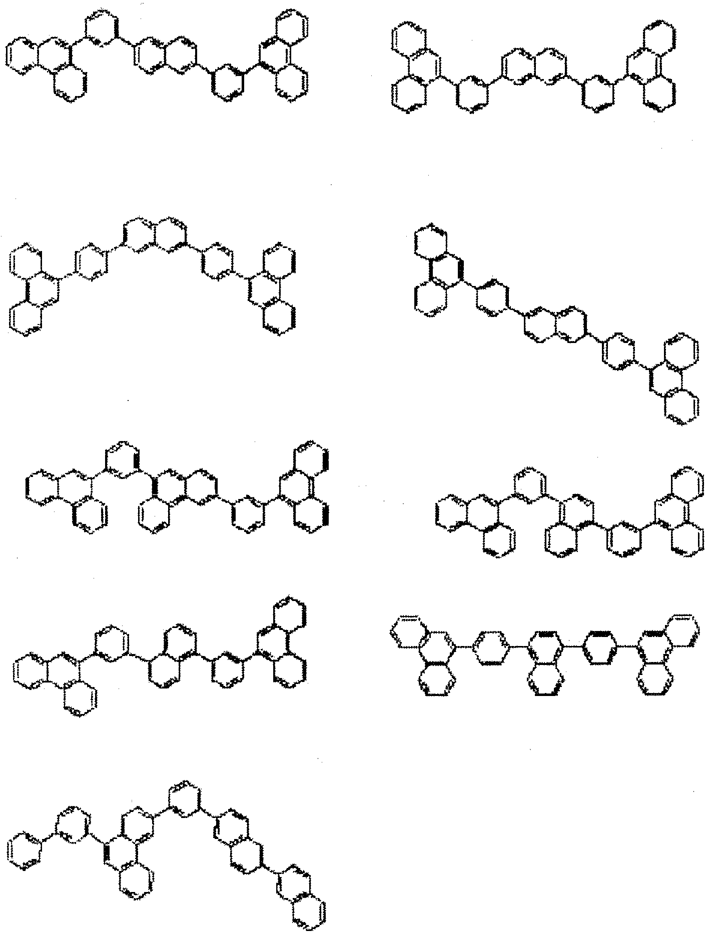

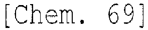

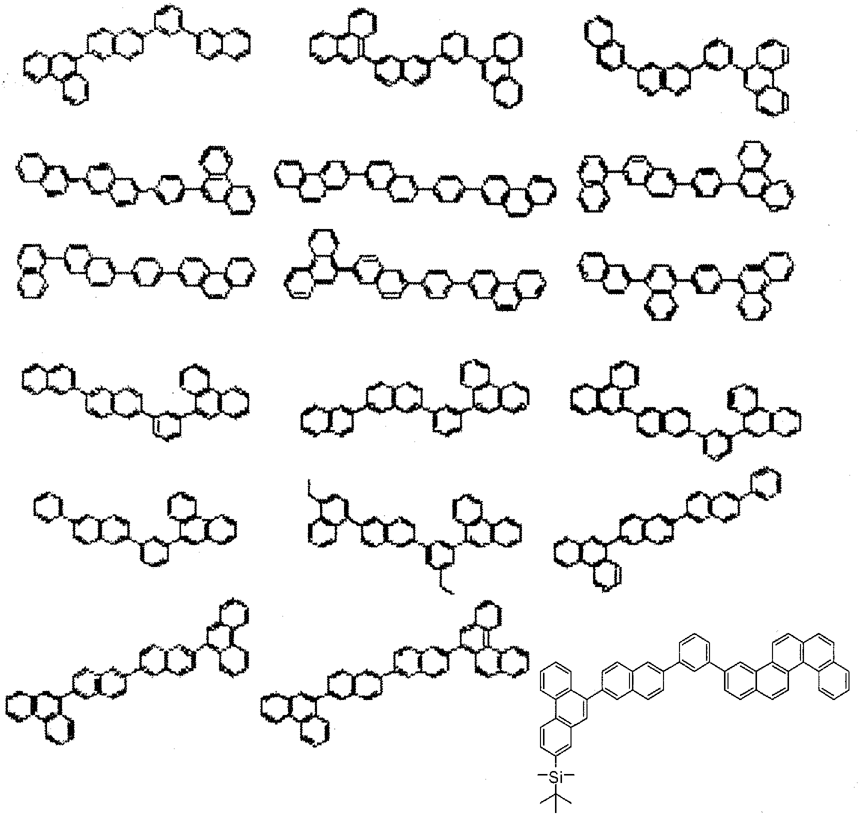

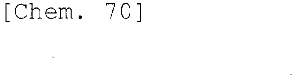

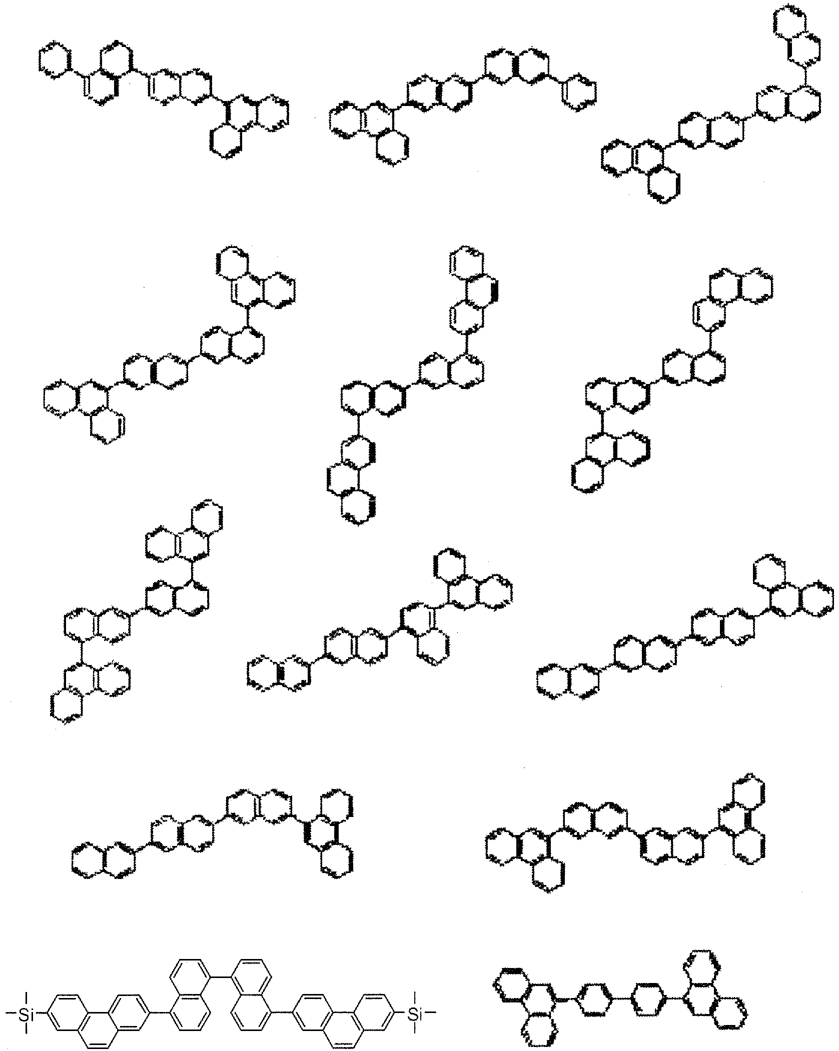

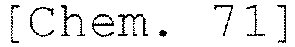

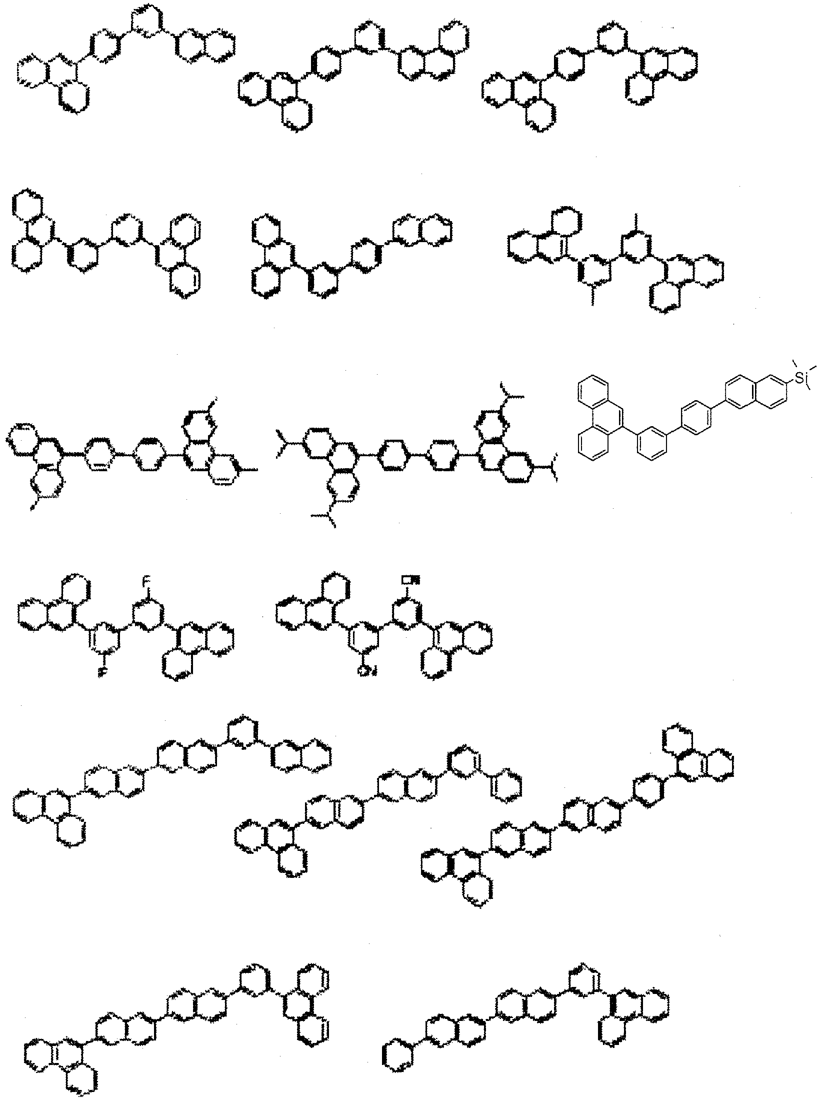

Inthepresentinvention,thepolycyclic,fused aromatic skeleton portion is preferably a chrysene simple substance represented by the following formula (51) or a derivative thereof.

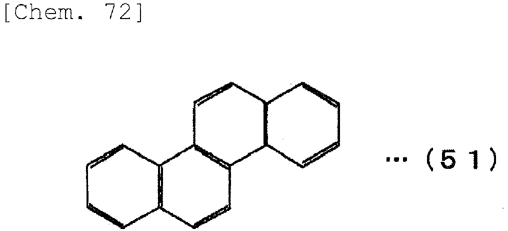

Such chrysene derivative is, for example, a derivative represented by the following formula (51A).

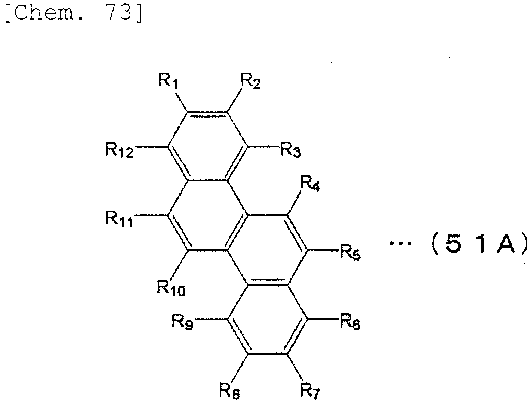

In the formula (51A), R₁ to R₁₂ each independently represent a hydrogen atom, or a substituent constructed of one, or a combination of two or more, of a substituted or unsubstituted aryl group having 5 to 30 ring carbon atoms, a branched or linear alkyl group having 1 to 30 carbon atoms, and a substituted or unsubstituted cycloalkyl group having 3 to 20 carbon atoms.
Specific examples of the chrysene derivative represented by the formula (51) include the following.

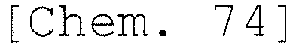

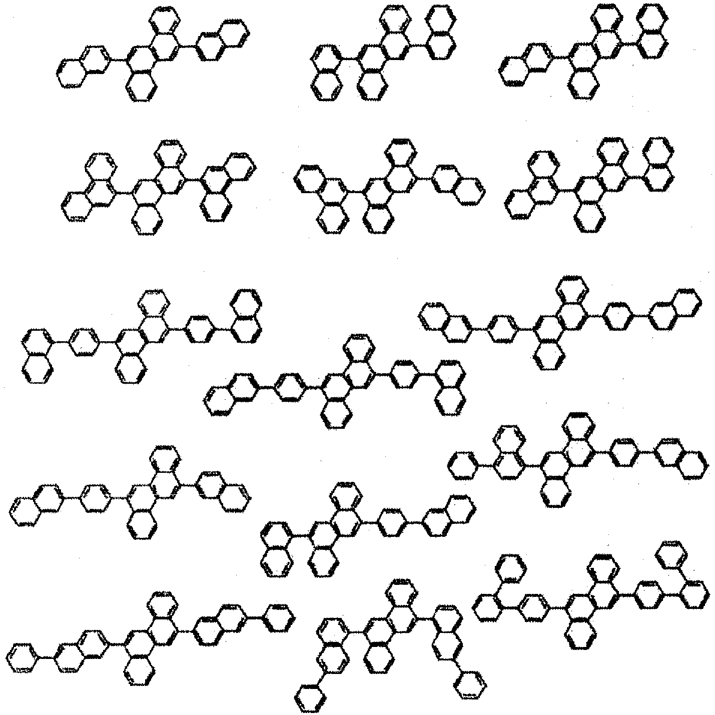

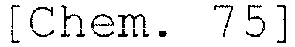

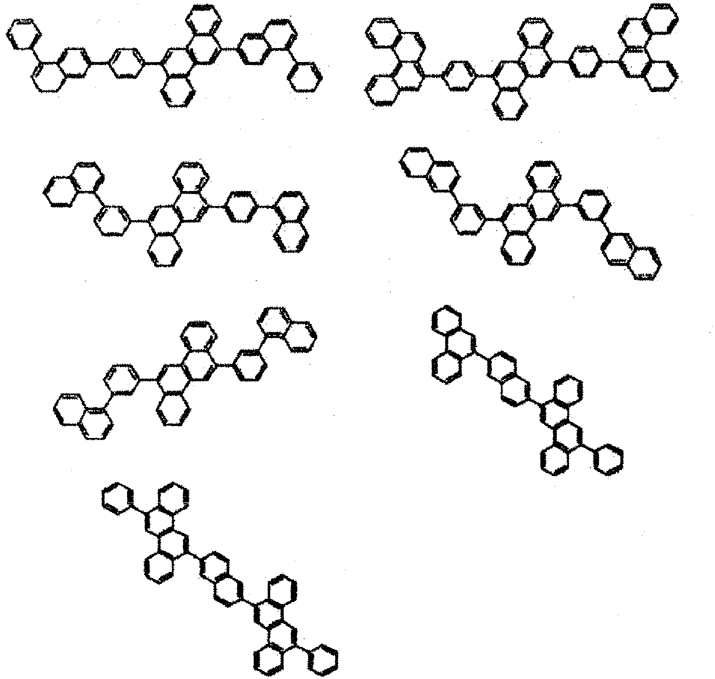

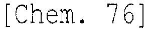

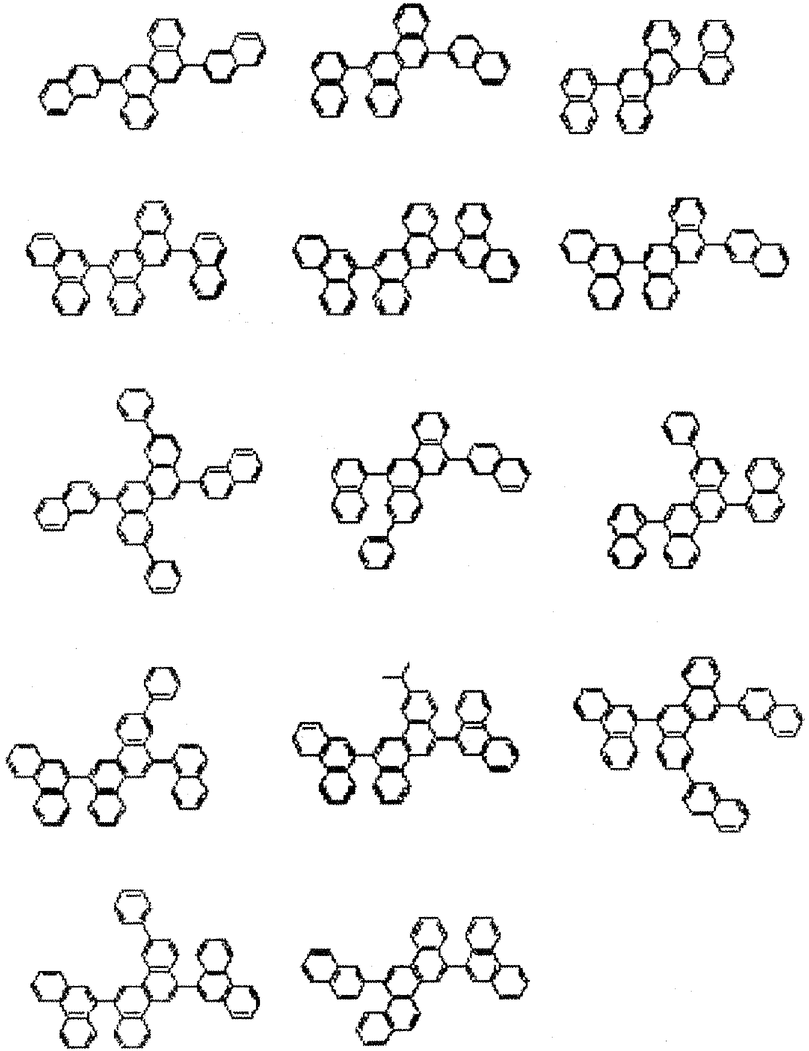

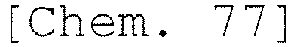

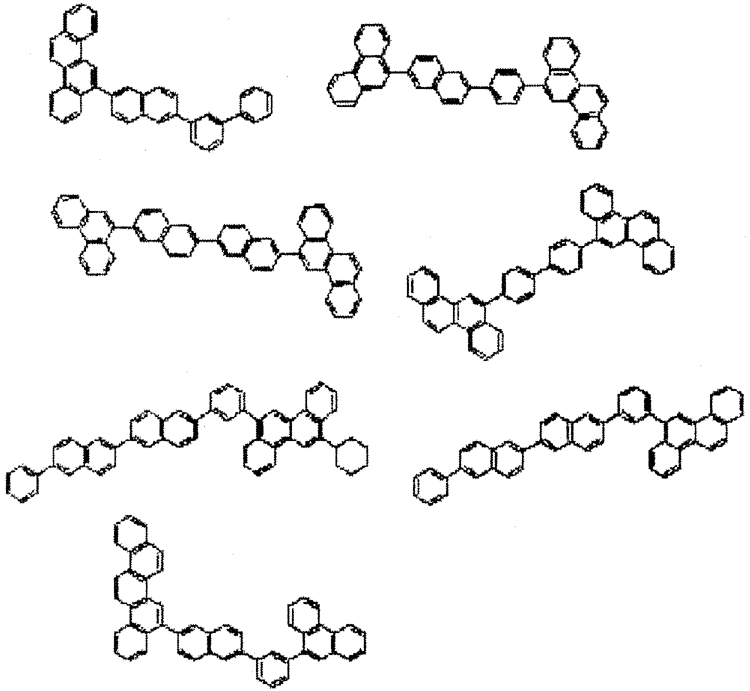

In the present invention, the polycyclic, fused aromatic skeleton portion is preferably a simple substance of a compound (benzo[c]phenanthrene) represented by the following formula (52) or a derivative thereof.

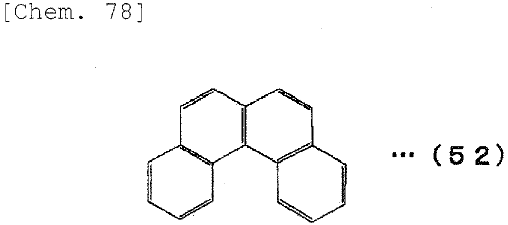

Such benzo[c]phenanthrene derivative is, for example, a derivative represented by the following formula (52A).

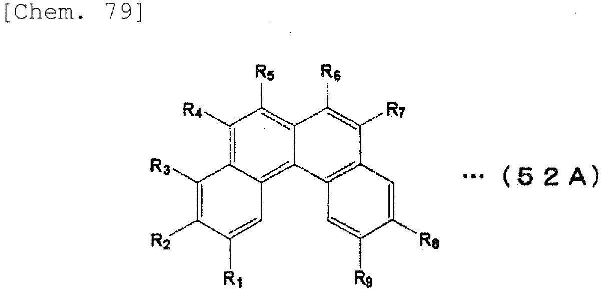

In the formula (52A), R₁ to R₉ each independently represent a hydrogen atom, or a substituent constructed of one, or a combination of two or more, of a substituted or unsubstituted aryl group having 5 to 30 ring carbon atoms, a branched or linear alkyl group having 1 to 30 carbon atoms, and a substituted or unsubstituted cycloalkyl group having 3 to 20 carbon atoms.
Specific examples of the benzo[c]phenanthrene derivative represented by the formula (52) include the following.

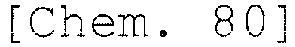

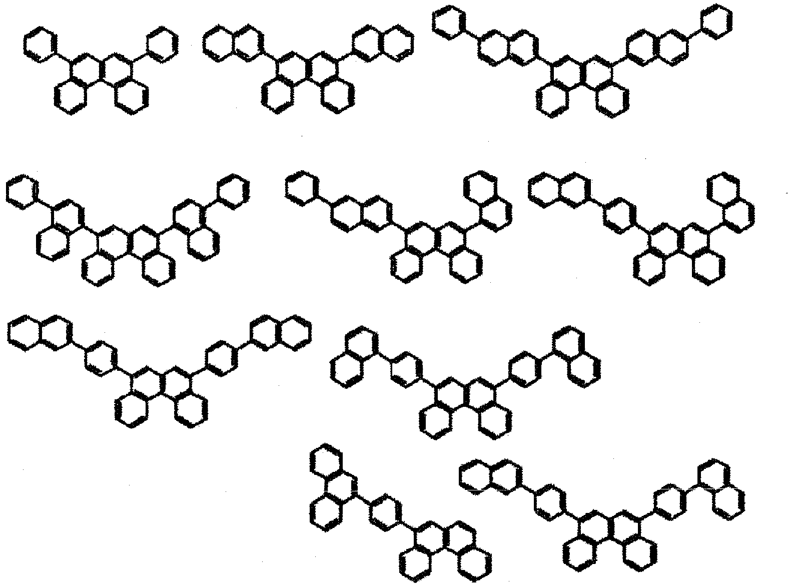

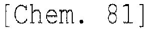

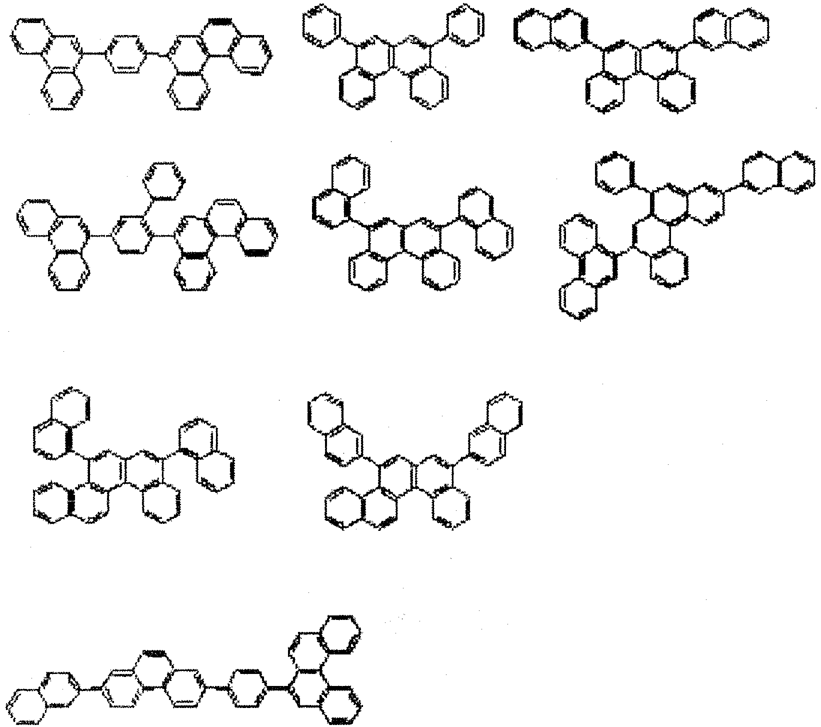

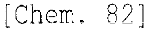

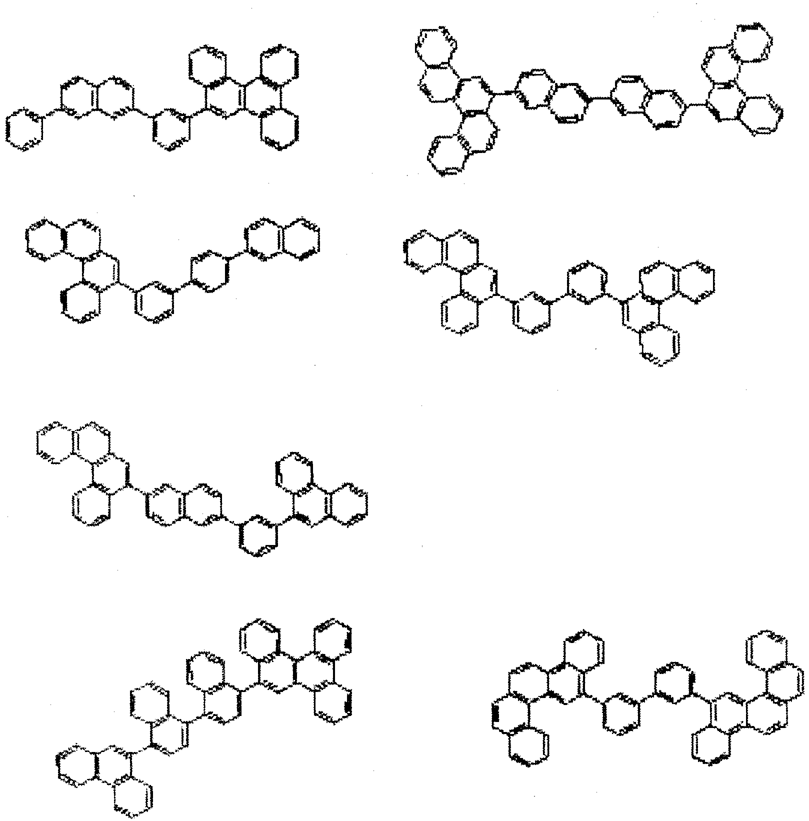

Inthepresentinvention,thepolycyclic,fused aromatic skeleton portion is preferably a simple substance of a compound (benzo[c]chrysene) represented by the following formula (53) or a derivative thereof.

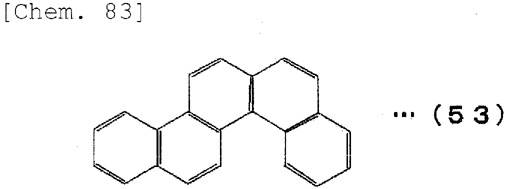

Such benzo[c]chrysene derivative is, for example, a derivative represented by the following formula (53A).

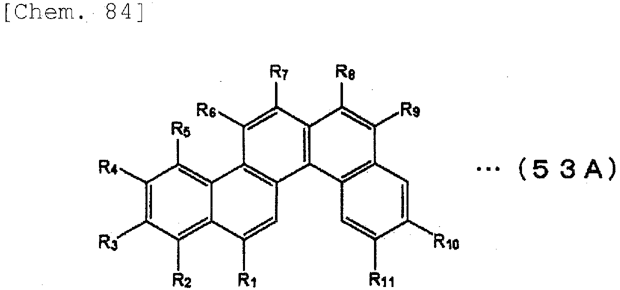

In the formula (53A), R₁ to R₁₁ each independently represent a hydrogen atom, or a substituent constructed of one, or a combination of two or more, of a substituted or unsubstituted aryl group having 5 to 30 ring carbon atoms, a branched or linear alkyl group having 1 to 30 carbon atoms, and a substituted or unsubstituted cycloalkyl group having 3 to 20 carbon atoms.
Specific examples of the benzo[c]chrysene derivative represented by the formula (53) include the following.

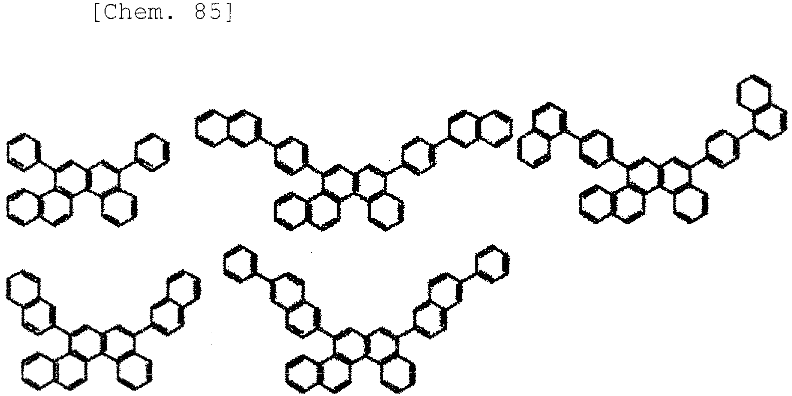

In the present invention, the polycyclic, fused aromatic skeleton portion is preferably a simple substance of a compound (dibenzo[c,g]phenanthrene) represented by the following formula (54) or a derivative thereof.

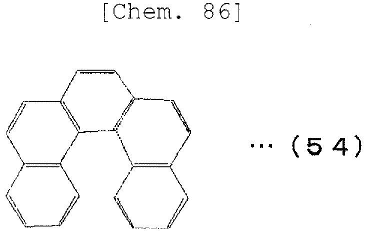

Examples of the derivative of such compound include the following.

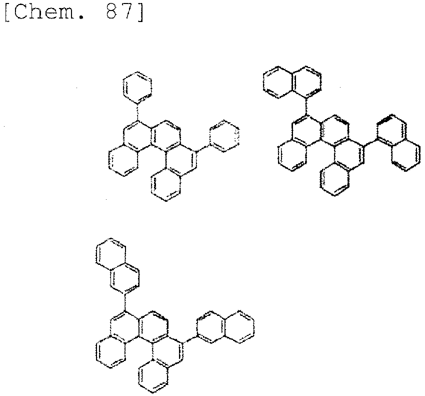

In the present invention, the polycyclic, fused aromatic skeleton portion is preferably a fluorolanthene simple substance represented by the following formula (55) or a derivative thereof.

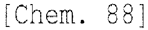

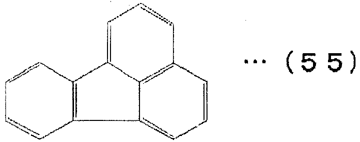

Such fluorolanthene derivative is, for example, a derivative represented by the following formula (55A).

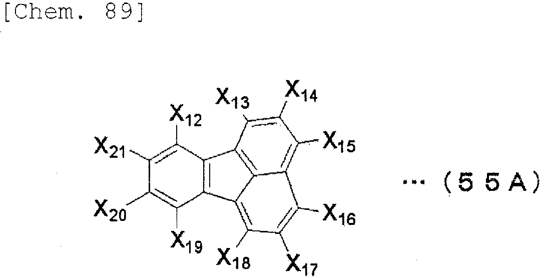

In the formula (55A), X₁₂ to X₂₁ each represent a hydrogen atom, a halogen atom, a linear, branched, or cyclic alkyl group, a linear, branched, or cyclic alkoxy group, or a substituted or unsubstituted aryl group.
It should be noted that the term "aryl group" refers to a carbocyclic aromatic group such as a phenyl group or a naphthyl group, or a heterocyclic aromatic group such as a furyl group, a thienyl group, or a pyridyl group.

X₁₂ to X₂₁ each represent preferably a hydrogen atom, a halogen atom (such as a fluorine atom, a chlorine atom, or a bromine atom), a linear, branched, or cyclic alkyl group having 1 to 16 carbon atoms (such as a methyl group, an ethyl group, an n-propyl group, an isopropyl group, an n-butyl group, an isobutyl group, an sec-butyl group, a tert-butyl group, an n-pentyl group, an isopentyl group, a neopentyl group, a tert-pentyl group, a cyclopentyl group, an n-hexyl group, a 3,3-dimethylbutyl group, a cyclohexyl group, an n-heptyl group, a cyclohexylmethyl group, an n-octyl group, a tert-octyl group, a 2-ethylhexyl group, an n-nonyl group, an n-decyl group, an n-dodecyl group, an n-tetradecyl group, or an n-hexadecyl group), a linear, branched, or cyclic alkoxy group having 1 to 16 carbon atoms (such as a methoxy group, an ethoxy group, an n-propoxy group, an isopropoxy group, an n-butoxy group, an isobutoxy group, an sec-butoxy group, an n-pentyloxy group, a neopentyloxy group, a cyclopentyloxy group, an n-hexyloxy group, a 3,3-dimethylbutyloxy group, a cyclohexyloxy group, an n-heptyloxy group, an n-octyloxy group, a 2-ethylhexyloxy group, an n-nonyloxy group, an n-decyloxy group, an n-dodecyloxy group, an n-tetradecyloxy group, or an n-hexadecyloxy group), or a substituted or unsubstituted aryl group having 4 to 16 carbon atoms (such as a phenyl group, a 2-methylphenyl group, a 3-methylphenyl group, a 4-methylphenyl group, a 4-ethylphenyl group, a 4-n-propylphenyl group, a 4-isopropylphenyl group, a 4-n-butylphenyl group, a 4-tert-butylphenyl group, a 4-isopentylphenyl group, a 4-tert-pentylphenyl group, a 4-n-hexylphenyl group, a 4-cyclohexylphenyl group, a 4-n-octylphenyl group, a 4-n-decylphenyl group, a 2,3-dimethylphenyl group, a 2,4-dimethylphenyl group, a 2,5-dimethylphenyl group, a 3,4-dimethylphenyl group, a 5-indanyl group, a 1,2,3,4-tetrahydro-5-naphthyl group, a 1,2,3,4-tetrahydro-6-naphthyl group, a 2-methoxyphenyl group, a 3-methoxyphenyl group, a 4-methoxyphenyl group, a 3-ethoxyphenyl group, a 4-ethoxyphenyl group, a 4-n-propoxyphenyl group, a 4-isopropoxyphenyl group, a 4-n-butoxyphenyl group, a 4-n-pentyloxyphenyl group, a 4-n-hexyloxyphenyl group, a 4-cyclohexyloxyphenyl group, a 4-n-heptyloxyphenyl group, a 4-n-octyloxyphenyl group, a 4-n-decyloxyphenyl group, a 2,3-dimethoxyphenyl group, a 2,5-dimethoxyphenyl group, a 3,4-dimethoxyphenyl group, a 2-methoxy-5-methylphenyl group, a 3-methyl-4-methoxyphenyl group, a 2-fluorophenyl group, a 3-fluorophenyl group, a 4-fluorophenyl group, a 2-chlorophenyl group, a 3-chlorophenyl group, a 4-chlorophenyl group, a 4-bromophenyl grou^{p},a4-trifluoromethylphenylgroup,a3,4-dichlorophenylgroup, a 2-methyl-4-chlorophenyl group, a 2-chloro-4-methylphenyl group, a 3-chloro-4-methylphenyl group, a 2-chloro-4-methoxyphenyl group, a 4-phenylphenyl group, a 3-phenylphenyl group, a 4-(4'-methylphehyl)phenyl group, a 4-(4'-methoxyphehyl) phenyl group, a 1-naphthyl group, a 2-naphthyl group, a 4-ethoxy-1-naphthyl group, a 6-methoxy-2-naphthyl group, a 7-ethoxy-2-naphthyl group, a 2-furyl group, a 2-thienyl group, a 3-thienyl group, a 2-pyridyl group, a 3-pyridyl group, or a 4-pyridyl group), more preferably a hydrogen atom, a fluorine atom, a chlorine atom, an alkyl group having 1 to 10 carbon atoms, an alkoxy group having 1 to 10 carbon atoms, or an aryl group having 6 to 12 carbon atoms, still more preferably a hydrogen atom, a fluorine atom, a chlorine atom, an alkyl group having 1 to 6 carbon atoms, an alkoxy group having 1 to 6 carbon atoms, or a carbocyclic aromatic group having 6 to 10 carbon atoms.

Specific examples of the fluoranthene, derivative represented by the formula (55) include the following.

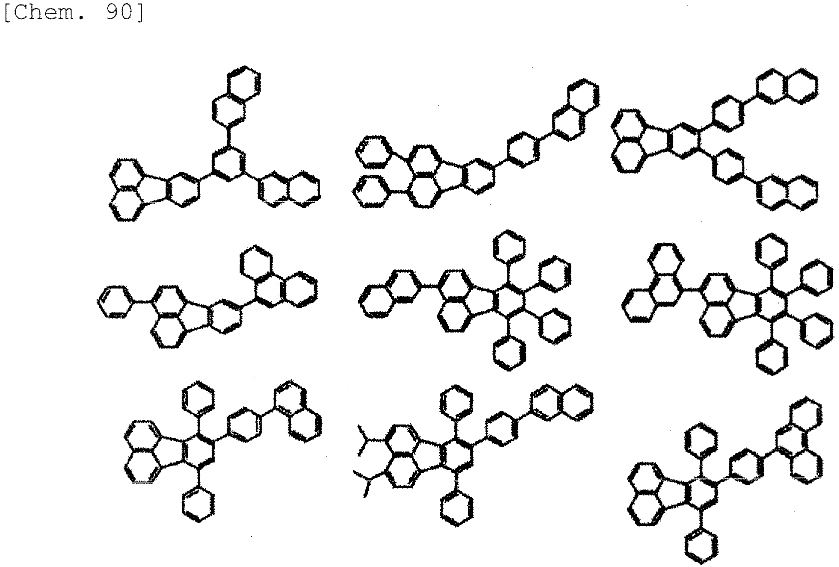

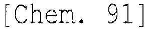

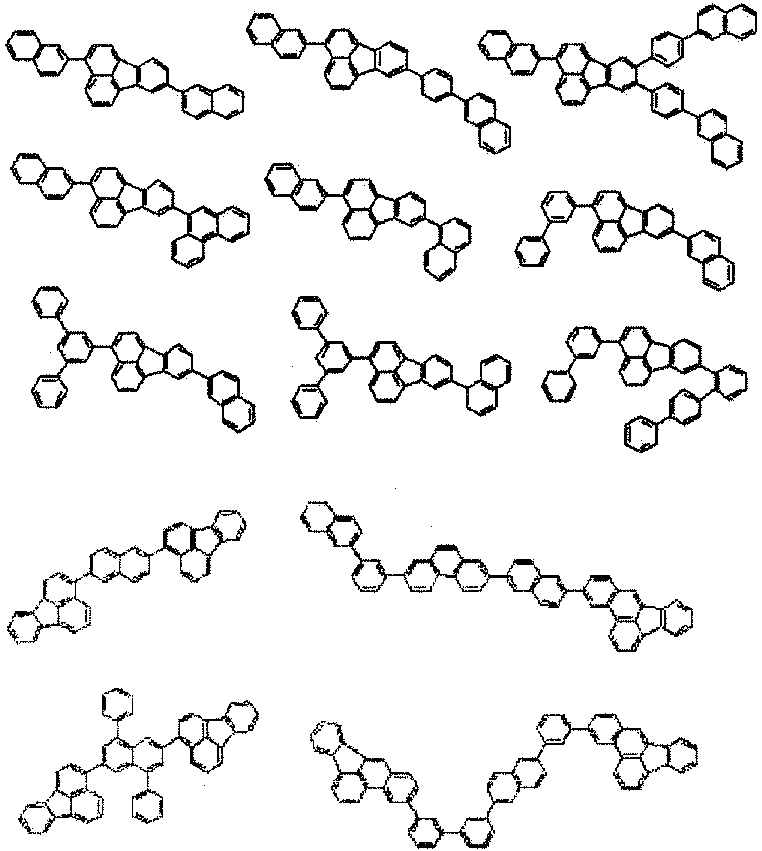

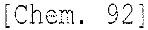

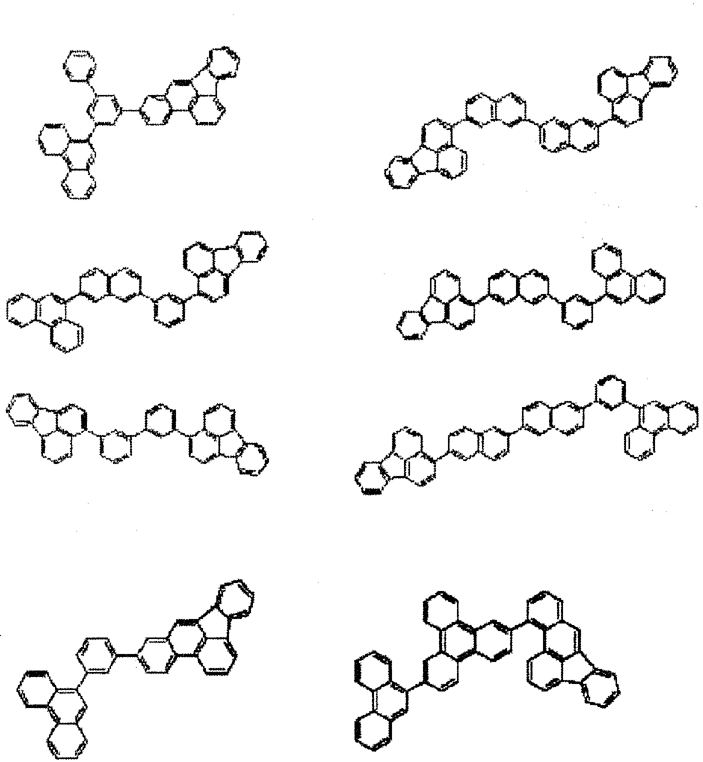

Examples of the substituted or unsubstituted benzofluoranthene include a benzo[b]fluoranthene simple substance represented by the following formula (551) or a derivative thereof, and a benzo[k]fluoranthene simple substance represented by the following formula (552) or a derivative thereof.

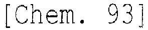

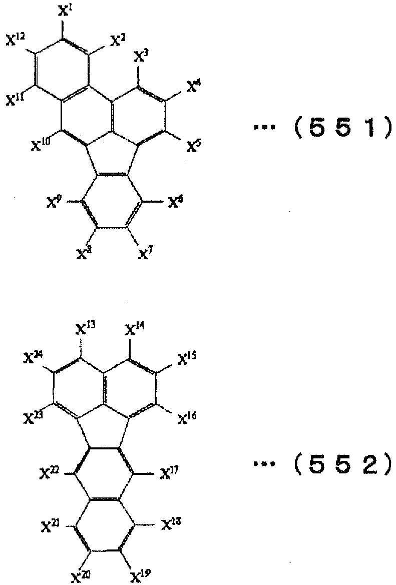

In the formulae (551) and (552), X¹ to X²⁴ each represent a hydrogen atom, a halogen atom, a linear, branched, or cyclic alkyl group, a linear, branched, or cyclic alkoxy group, or a substituted or unsubstituted aryl group.
It should be noted that the term "aryl group" refers to a carbocyclic aromatic group such as a phenyl group or a naphthyl group, or a heterocyclic aromatic group such as a furyl group, a thienyl group, or a pyridyl group.

X¹ to X²⁴ each represent preferably a hydrogen atom, a halogen atom (such as a fluorine atom, a chlorine atom, or a bromine atom), a linear, branched, or cyclic alkyl group having 1 to 16 carbon atoms (such as a methyl group, an ethyl group, an n-propyl group, an isopropyl group, an n-butyl group, an isobutyl group, an sec-butyl group, a tert-butyl group, an n-pentyl group, an isopentyl group, a neopentyl group, a tert-pentyl group, a cyclopentyl group, an n-hexyl group, a 3,3-dimethylbutyl group, a cyclohexyl group, an n-heptyl group, a cyclohexylmethyl group, an n-octyl group, a tert-octyl group, a 2-ethylhexyl group, an n-nonyl group, an n-decyl group, an n-dodecyl group, an n-tetradecyl group, or an n-hexadecyl group), a linear, branched, or cyclic alkoxy group having 1 to 16 carbon atoms (such as a methoxy group, an ethoxy group, an n-propoxy group, an isopropoxy group, an n-butoxy group, an isobutoxy group, an sec-butoxy group, an n-pentyloxy group, a neopentyloxy group, a cyclopentyloxy group, an n-hexyloxy group, a 3,3-dimethylbutyloxy group, a cyclohexyloxy group, an n-heptyloxy group, an n-octyloxy group, a 2-ethylhexyloxy group, an n-nonyloxy group, an n-decyloxy group, an n-dodecyloxy group, an n-tetradecyloxy group, or an n-hexadecyloxy group), or a substituted or unsubstituted aryl group having 4 to 16 carbon atoms (such as a phenyl group, a 2-methylphenyl group, a 3-methylphenyl group, a 4-methylphenyl group, a 4-ethylphenyl group, a 4-n-propylphenyl group, a 4-isopropylphenyl group, a 4-n-butylphenyl group, a 4-tert-butylphenyl group, a 4-isopentylphenyl group, a 4-tert-pentylphenyl group, a 4-n-hexylphenyl group, a 4-cyclohexylphenyl group, a 4-n-octylphenyl group, a 4-n-decylphenyl group, a 2,3-dimethylphenyl group, a 2,4-dimethylphenyl group, a 2,5-dimethylphenyl group, a 3,4-dimethylphenyl group, a 5-indanyl group, a 1,2,3,4-tetrahydro-5-naphthyl group, a 1,2,3,4-tetrahydro-6-naphthyl group, a 2-methoxyphenyl group, a 3-methoxyphenyl group, a 4-methoxyphenyl group, a 3-ethoxyphenyl group, a 4-ethoxyphenyl group, a 4-n-propoxyphenyl group, a 4-isopropoxyphenyl group, a 4-n-butoxyphenyl group, a 4-n-pentyloxyphenyl group, a 4-n-hexyloxyphenyl group, a 4-cyclohexyloxyphenyl group, a 4-n-heptyloxyphenyl group, a 4-n-octyloxyphenyl group, a 4-n-decyloxyphenyl group, a 2,3-dimethoxyphenyl group, a 2,5-dimethoxyphenyl group, a 3,4-dimethoxyphenyl group, a 2-methoxy-5-methylphenyl group, a 3-methyl-4-methoxyphenyl group, a 2-fluorophenyl group, a 3-fluorophenyl group, a 4-fluorophenyl group, a 2-chlorophenyl group, a 3-chlorophenyl group, a 4-chlorophenyl group, a 4-bromophenyl group, a 4-trifluoromethylphenyl group, a 3,4-dichlorophenyl group, a 2-methyl-4-chlorophenyl group, a 2-chloro-4-methylphenyl group, a 3-chloro-4-methylphenyl group, a 2-chloro-4-methoxyphenyl group, a 4-phenylphenyl group, a 3-phenylphenyl group, a 4-(4'-methylphehyl)phenyl group, a 4-(4'-methoxyphehyl)phenyl group, a 1-naphthyl group, a2-naphthylgroup, a 4-ethoxy-1-naphthyl group, a 6-methoxy-2-naphthyl group, a 7-ethoxy-2-naphthyl group, a 2-furyl group, a 2-thienyl group, a 3-thienyl group, a 2-pyridyl group, a 3-pyridyl group, or a 4-pyridyl group), more preferably a hydrogen atom, a fluorine atom, a chlorine atom, an alkyl group having 1 to 10 carbon atoms, an alkoxy group having 1 to 10 carbon atoms, or an aryl group having 6 to 12 carbon atoms, still more preferably a hydrogen atom, a fluorine atom, a chlorine atom, an alkyl group having 1 to 6 carbon atoms, an alkoxy group having 1 to 6 carbon atoms, or a carbocyclic aromatic group having 6 to 10 carbon atoms.

Examples of the benzo[b]fluoranthene derivative represented by the formula (551) include the following.

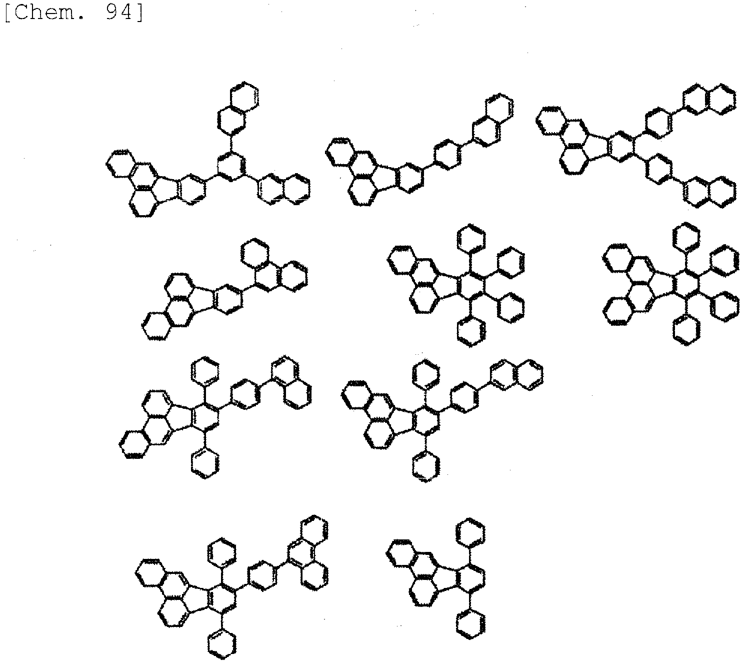

Examples of the benzo[k]fluoranthene derivative represented by the formula (552) include the following.

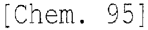

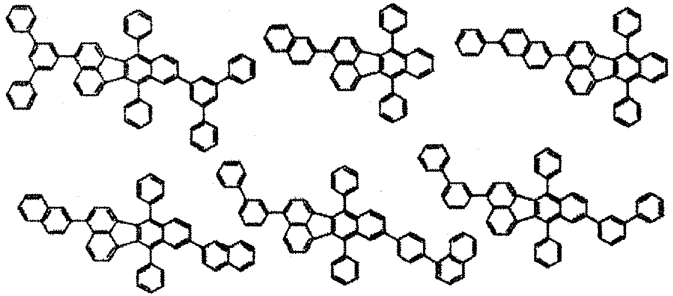

In the present invention, the polycyclic, fused aromatic skeleton portion is preferably a triphenylene simple substance represented by the following formula (56) or a derivative thereof.

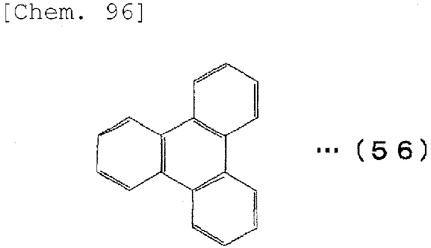

Such triphenylene derivative is, for example, a derivative represented by the following formula (56A).

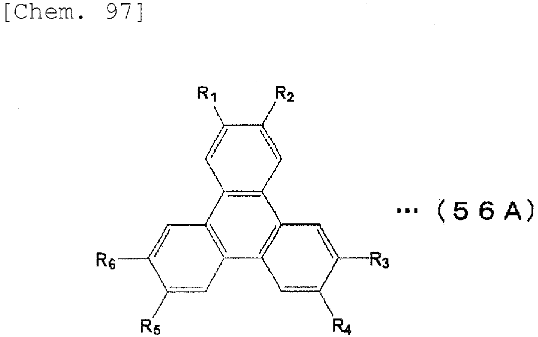

In the formula (56A), R₁ to R₆ each independently represent a hydrogen atom, or a substituent constructed of one, or a combination of two or more, of a substituted or unsubstituted aryl group having 5 to 30 ring carbon atoms, a branched or linear alkyl group having 1 to 30 carbon atoms, and a substituted or unsubstituted cycloalkyl group having 3 to 20 carbon atoms.
Specific examples of the triphenylene derivative represented by the formula (56) include the following.

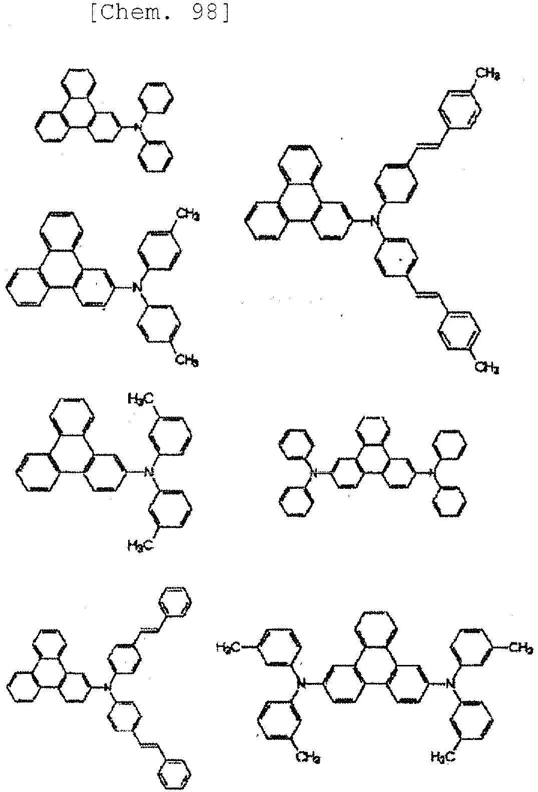

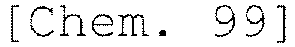

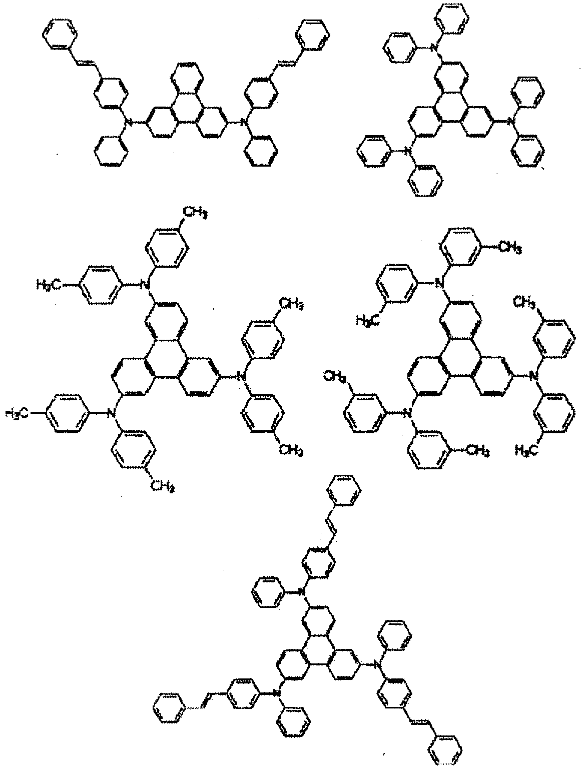

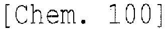

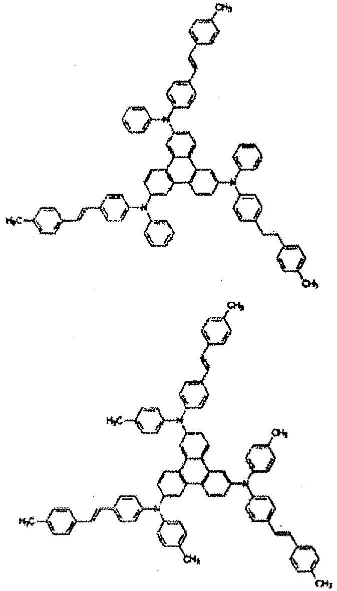

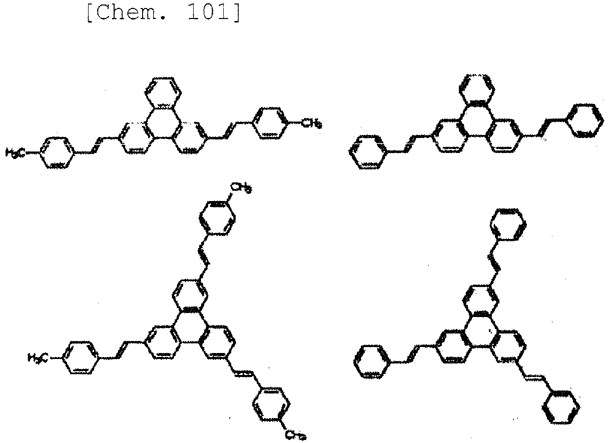

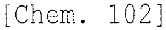

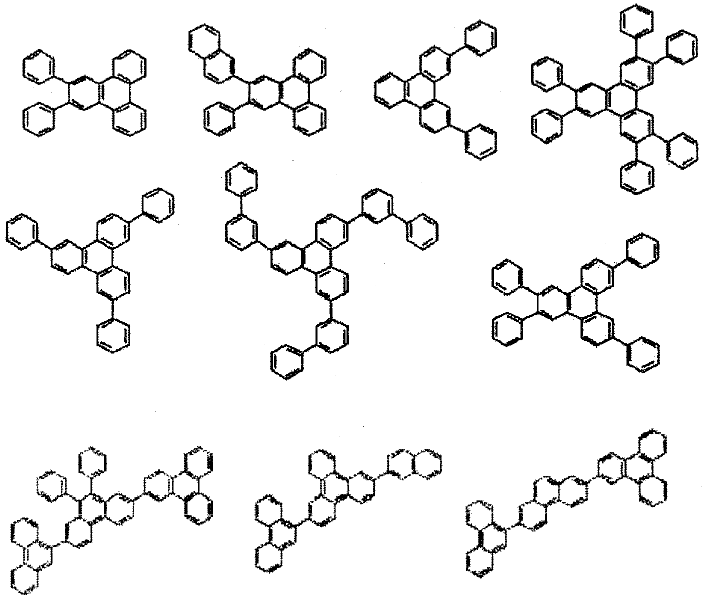

In the present invention, the polycyclic, fused aromatic skeleton portion is preferably a naphthalene simple substance or a derivative thereof.
Such naphthalene derivative is, for example, a derivative represented by the following formula (57A).

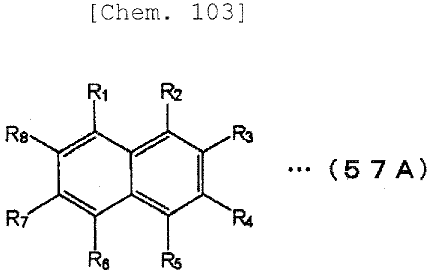

In the formula (57A), R₁ to R₈ each independently represent a hydrogen atom, or a substituent constructed of one, or a combination of two or more, of a substituted or unsubstituted aryl group having 5 to 30 ring carbon atoms, a branched or linear alkyl group having 1 to 30 carbon atoms, and a substituted or unsubstituted cycloalkyl group having 3 to 20 carbon atoms.
Specific examples of the naphthalene derivative include the following.

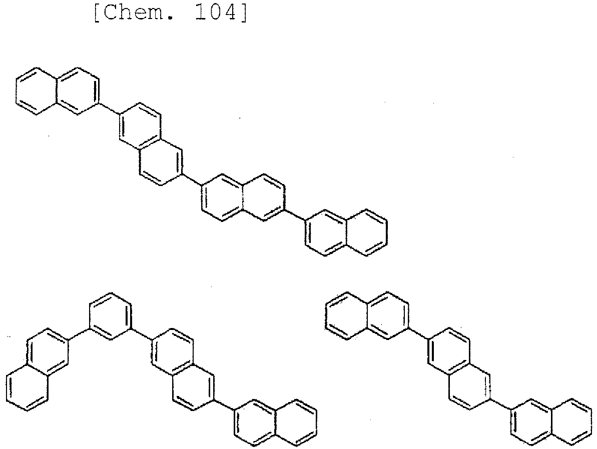

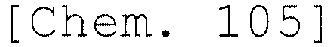

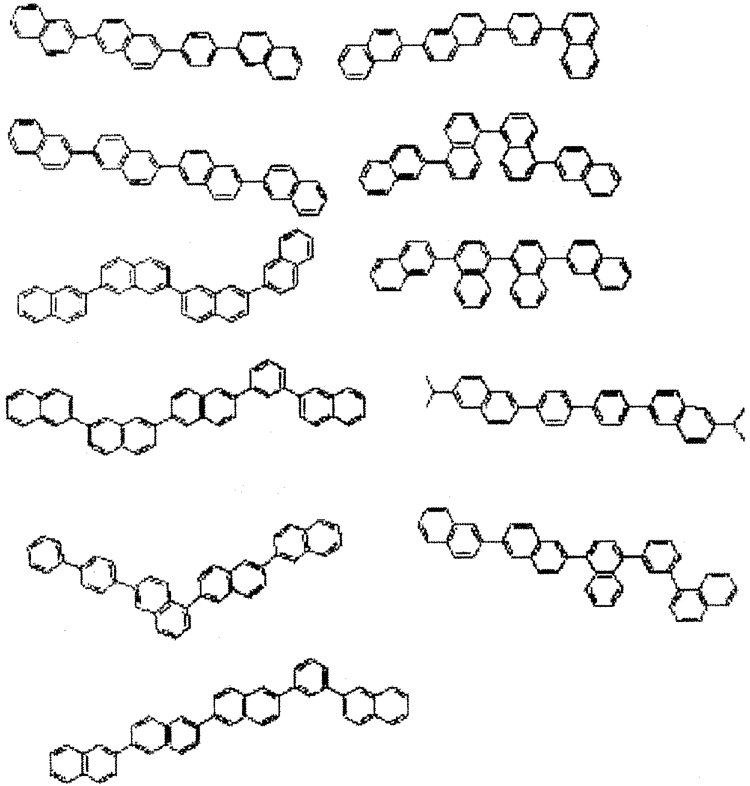

It should be noted that the polycyclic, fused aromatic skeleton portion may contain a nitrogen atom, and may be, for example, any one of the following.

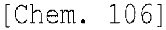

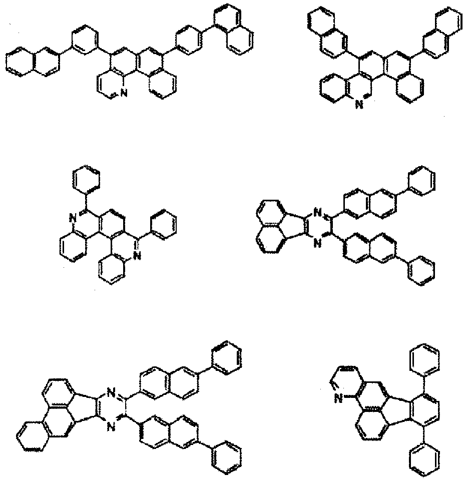

In the present invention, the polycyclic, fused aromatic skeleton portion is preferably a substituted or unsubstituted phenanthrene or chrysene.
Examples of such phenanthrene and chrysene are the same as described above.
When the polycyclic, fused aromatic skeleton portion is a substituted or unsubstituted phenanthrene or chrysene, a difference between the Eg(S) and the Eg(T) is small, and hence a reduction in the driving voltage of the organic EL device and the lengthening of its lifetime can be achieved.

### (5) Electron injecting and transporting layer

Next, the electron injecting and transporting layer is a layer which helps injection of electrons into the light emitting layer, transports the electrons to the light emitting region, and exhibits a great mobility of electrons. The adhesion improving layer is an electron injecting and transporting layer including a material exhibiting particularly improved adhesion with the cathode.
In addition, it is known that, in an organic EL device, emitted light is reflected by an electrode (cathode in this case), so emitted light directly extracted from an anode and emitted light extracted via the reflection by the electrode interfere with each other. The thickness of an electron injecting and transporting layer is appropriately selected from the range of several nanometers to several micrometers in order that the interference effect may be effectively utilized. When the thickness is particularly large, an electron mobility is preferably at least 10⁻⁵ cm²/Vs or more upon application of an electric field of 10⁴ to 10⁶ V/cm in order to avoid an increase in voltage.
A metal complex of 8-hydroxyquinoline or of a derivative of 8-hydroxyquinoline, or an oxadiazole derivative is suitable as a material to be used in an electron injecting and transporting layer. Specific examples of the metal complex of 8-hydroxyquinoline or of the derivative of 8-hydroxyquinoline that can be used as an electron injecting material include metal chelate oxynoid compounds each containing a chelate of oxine (generally 8-quinolinol or 8-hydroxyquinoline), such as tris(8-quinolinol)aluminum.

In addition, in the present invention, it is preferred that: the organic EL device have an electron transporting layer and/or an electron injecting layer provided on a side closer to the cathode than the light emitting layer; and a nitrogen-containing heterocyclic derivative represented by any one of the following formulae (31) to (33), a compound represented by any one of the following formulae (34) and (35), or a compound represented by the following formula (36) be incorporated into the electron transporting layer and/or the electron injecting layer.

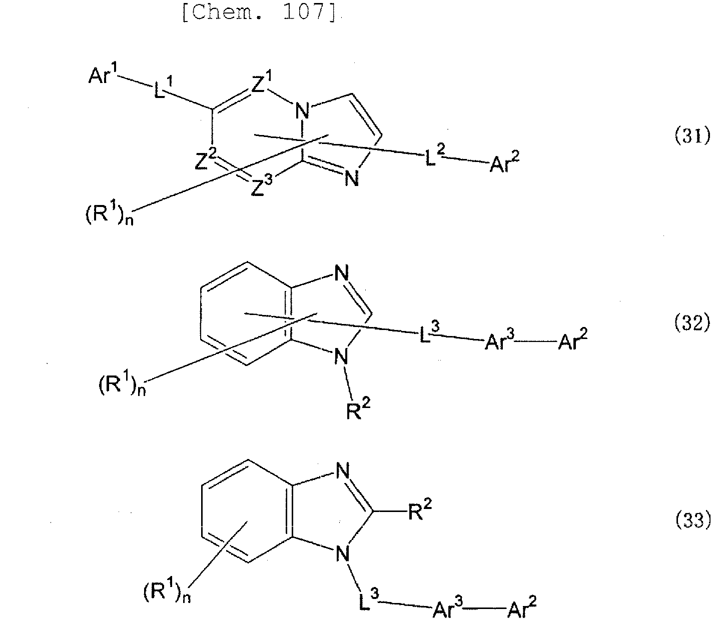

(In the formulae (31) to (33):
Z¹, Z², and Z³ each independently represent a nitrogen atom or a carbon atom;
R¹ and R² each independently represent a substituted or unsubstituted aryl group having 6 to 50 carbon atoms, a substituted or unsubstituted heteroaryl group having 3 to 50 carbon atoms, an alkyl group having 1 to 20 carbon atoms, an alkyl group having 1 to 20 carbon atoms and substituted with a halogen atom, or an alkoxy group having 1 to 20 carbon atoms;
n represents an integer of 0 to 5, and when n represents an integer of 2 or more, a plurality of R¹'s may be identical to or different from each other, and a plurality of R¹'s adjacent to each other may be bonded to each other to form a substituted or unsubstituted aromatic hydrocarbon ring;
Ar¹ represents a substituted or unsubstituted aryl group having 6 to 50 carbon atoms, or a substituted or unsubstituted heteroaryl group having 3 to 50 carbon atoms;
Ar² represents a hydrogen atom, an alkyl group having 1 to 20 carbon atoms, an alkyl group having 1 to 20 carbon atoms and substituted with a halogen atom, an alkoxy group having 1 to 20 carbon atoms, a substituted or unsubstituted aryl group having 6 to 50 carbon atoms, or a substituted or unsubstituted heteroaryl group having 3 to 50 carbon atoms,
provided that one of Ar¹ and Ar² represents a substituted or unsubstituted fused ring group having 10 to 50 carbon atoms, or a substituted or unsubstituted heterofused ring group having 9 to 50 ring atoms;
Ar³ represents a substituted or unsubstituted arylene group having 6 to 50 carbon atoms, or a substituted or unsubstituted heteroarylene group having 3 to 50 carbon atoms; and
L¹, L², and L³ each independently represent a single bond, a substituted or unsubstituted arylene group having 6 to 50 carbon atoms, a substituted or unsubstituted heterofused ring group having 9 to 50 ring atoms, or a substituted or unsubstituted fluorenylene group.)
Specific examples of the aryl group, the heteroaryl group, and the alkyl group represented by R¹, R², Ar¹, and Ar² include the same examples as those of R₃ and R₄ of the general formula (1), and examples of the alkoxy group include examples each obtained by bonding an oxygen atom to any such alkyl group. Examples of the arylene group represented by any one of Ar³, L¹, L², and L³ include examples each obtained by making any such aryl group divalent, and the heterofused ring group is, for example, a fused ring group whose carbon number is suitable out of the heteroaryl groups.

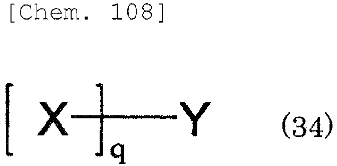

(In the formula, X represents a fused ring containing a nitrogen atom or a sulfur atom, one, or a combination of two or more, of a single bond, an alkyl chain, an alkylene chain, a cycloalkyl chain, an aryl chain, a heterocyclic chain, a silyl chain, an ether chain, and a thioether chain is selected as Y, and q represents a natural number of 2 or more.
In addition, the compound represented by the formula (34) has a molecular weight of 480 or more.)

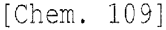

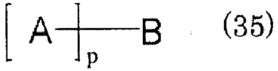

(In the formula, A represents a substituent having a phenanthroline skeleton or a benzoquinoline skeleton, B represents a p-valent organic group having a structure represented by the following formula (35A), and p represents a natural number of 2 or more.)

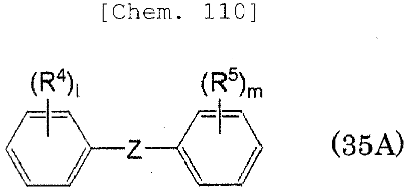

(In the formula, R⁴ and R⁵ each independently represent any one of an alkyl group and an aryl group (including an aryl group that fuses with a phenyl group), l and m each independently represent a natural number of 0 to 5, and Z represents at least one kind selected from the following formulae (35B).)

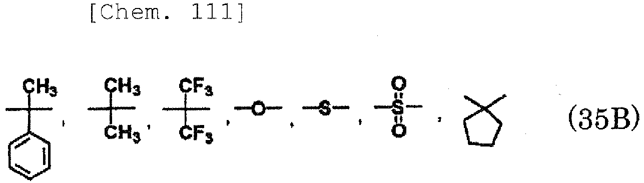

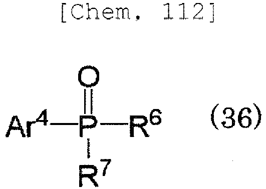

(In the formula, R⁶ and R⁷ may be identical to or different from each other, and are each selected from a hydrogen atom, an alkyl group, a cycloalkyl group, a heterocyclic group, an alkenyl group, a cycloalkenyl group, an alkynyl group, an alkoxy group, an alkylthio group, an arylether group, an arylthioether group, an aryl group, a heteroaryl group, a cyano group, a carbonyl group, an ester group, a carbamoyl group, an amino group, a silyl group, and a fused ring formed with an adj acent substituent, and Ar⁴ represents an aryl group or a heteroaryl group.)

It should be noted that an arbitrary substituent in the "substituted or unsubstituted ... group" of each of the formulae in the present invention is, for example, a substituted or unsubstituted aryl group having 6 to 50 ring carbon atoms, a substituted or unsubstituted alkyl group having 1 to 50 carbon atoms, a substituted or unsubstituted alkoxy group having 1 to 50 carbon atoms, a substituted or unsubstituted aralkyl group having 7 to 50 ring carbon atoms, a substituted or unsubstituted aryloxy group having 6 to 50 ring carbon atoms, a substituted or unsubstituted arylthio group having 6 to 50 ring carbon atoms, a substituted or unsubstituted alkoxycarbonyl group having 1 to 50 carbon atoms, an amino group, a halogen atom, a cyano group, a nitro group, a hydroxyl group, or a carboxyl group.
Of those, analkyl group having 1 to 10 carbon atoms, a cycloalkyl group having 5 to 7 carbon atoms, or an alkoxy group having 1 to 10 carbon atoms is preferred, an alkyl group having 1 to 6 carbon atoms or a cycloalkyl group having 5 to 7 carbon atoms is more preferred, and a methyl group, an ethyl group, an n-propyl group, an isopropyl group, an n-butyl group, an sec-butyl group, a tert-butyl group, an n-pentyl group, an n-hexyl group, a cyclopentyl group, or a cyclohexyl group is particularly preferred.

A preferred embodiment of the organic EL device of the present invention includes an element including a reduction-causing dopant in the region of electron transport or in the interfacial region of the cathode and the organic layer. The reduction-causing dopant is defined as a substance which can reduce a compound having the electron transporting property. Various substances can be used as the reduction-causing dopant as long as the substances have a uniform reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals, and organic complexes of rare earth metals can be preferably used.
More specifically, preferred examples of the reduction-causing dopant include at least one alkali metal selected from the group consisting of Na (the work function: 2.36 eV), K (the work function: 2.28 eV), Rb (the work function: 2.16 eV), and Cs (the work function: 1.95 eV) and at least one alkaline earth metal selected from the group consisting of Ca (the work function: 2.9 eV), Sr (the work function: 2.0 to 2.5 eV), and Ba (the work function: 2.52 eV). Particularly preferred are substances having a work function of 2.9 eV or less. Of those, at least one alkali metal selected from the group consisting of K, Rb, and Cs is more preferred, Rb and Cs are still more preferred, and Cs is most preferred as the reduction-causing dopant. In particular, those alkali metals have great reducing ability, and the emission luminance and the lifetime of the organic EL device can be increased by addition of a relatively small amount of the alkali metal into the electron injecting zone. As the reduction-causing dopant having a work function of 2. 9 eV or less, combinations of two or more alkali metals thereof are also preferred. Combinations having Cs such as the combinations of Cs and Na, Cs and K, Cs and Rb, and Cs, Na, and K are particularly preferred. The reducing ability can be efficiently exhibited by the combination having Cs. The emission luminance and the lifetime of the organic EL device can be increased by adding the combination having Cs into the electron injecting zone.

The present invention may further include an electron injecting layer which is constructed of an insulating material or a semiconductor and disposed between the cathode and the organic layer. At this time, the electron injecting property can be improved by preventing a leak of electric current effectively. As the insulating material, at least one metal compound selected from the group consisting of alkali metal chalcogenides, alkaline earth metal chalcogenide, alkali metal halides, and alkaline earth metal halides is preferred. It is preferred that the electron injecting layer be constructed of the above-mentioned substance such as the alkali metal chalcogenide since the electron injecting property can be further improved. To be specific, preferred examples of the alkali metal chalcogenide include Li₂O, K₂O, Na₂S, Na₂Se, and Na₂O. Preferred examples of the alkaline earth metal chalcogenide include CaO, BaO, SrO, BeO, BaS, and CaSe. Preferred examples of the alkali metal halide include LiF, NaF, KF, LiCl, KCl, and NaCl. Preferred examples of the alkaline earth metal halide include fluorides such as CaF₂, BaF₂, SrF₂, MgF₂, and BeF₂ and halides other than the fluorides.
Examples of the semiconductor include oxides, nitrides, and oxide nitrides of at least one element selected from Ba, Ca, Sr, Yb, Al, Ga, In, Li, Na, Cd, Mg, Si, Ta, Sb, and Zn used alone or in combination of two or more. It is preferred that the inorganic compound constructing the electron injecting layer form a crystallite or amorphous insulating thin film. When the electron injecting layer is constructed of the insulating thin film described above, a more uniform thin film can be formed, and defects of pixels such as dark spots can be decreased. Examples of the inorganic compound include alkali metal chalcogenides, alkaline earth metal chalcogenides, alkali metal halides, and alkaline earth metal halides which are described above.

### (6) Cathode

For the cathode, a material such as a metal, an alloy, an electroconductive compound, or a mixture of those materials which has a small work function (4 eV or less) is used as an electrode material because the cathode is used for injecting electrons to the electron injecting and transporting layer or the light emitting layer. Specific examples of the electrode material include sodium, sodium-potassium alloys, magnesium, lithium, magnesium-silver alloys, aluminum/aluminum oxide, aluminum-lithium alloys, indium, and rare earth metals.
The cathode can be prepared by forming a thin film of the electrode material described above in accordance with a process such as the vapor deposition process or the sputtering process.
When the light emitted from the light emitting layer is obtained through the cathode, it is preferred that the cathode have a transmittance of the emitted light higher than 10%.
It is also preferred that the sheet resistivity of the cathode be several hundred Ω□/or less. The thickness of the cathode is, in general, selected in the range of 10 nm to 1 µm and preferably in the range of 50 to 200 nm.

### (7) Insulating layer

Defects in pixels tend to be formed in organic EL device due to leak and short circuit since an electric field is applied to ultra-thin films. In order to prevent the formation of the defects, a layer of a thin film having an insulating property may be inserted between the pair of electrodes.
Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. Mixtures and laminates of the above-mentioned compounds may also be used.

### (8) Method of producing the organic EL device

In order to prepare the organic EL device of the present invention, the anode and the light emitting layer, and, where necessary, the hole inj ecting and transporting layer and the electron injecting and transporting layer are formed in accordance with the illustrated process using the illustrated materials, and the cathode is formed in the last step. The organic EL device may also be prepared by forming the above-mentioned layers in the order reverse to the order described above, i.e., the cathode being formed in the first step and the anode in the last step.
Hereinafter, an embodiment of the process for preparing an organic EL device having a construction in which an anode, a hole injecting layer, a light emitting layer, an electron injecting layer, and a cathode are disposed successively on a light-transmissive substrate will be described.
On a suitable light-transmissive substrate, a thin film made of a material for the anode is formed in accordance with the vapor deposition process or the sputtering process so that the thickness of the formed thin film is 1 µm or less and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process, or the LB process, as described above. The vacuum vapor deposition process is preferred since a uniform film can be easily obtained and the possibility of formation of pin holes is small. When the hole injecting layer is formed in accordance with the vacuum vapor deposition process, in general, it is preferred that the conditions be suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C; and the thickness of the film: 5 nm to 5 µm although the conditions of the vacuum vapor deposition are different depending on the compound to be used (i.e., material for the hole injecting layer) and the crystal structure and the recombination structure of the target hole injecting layer.

Then, the light emitting layer is formed on the hole injecting layer formed above. A thin film of the organic light emitting material can be formed by using a desired organic light emitting material in accordance with a process such as the vacuum vapor deposition process, the sputtering process, the spin coating process, or the casting process, and the formed thin film is used as the light emitting layer. The vacuum vapor deposition process is preferred since a uniform film can be easily obtained and the possibility of formation of pin holes is small. When the light emitting layer is formed in accordance with the vacuum vapor deposition process, in general, the conditions of the vacuum vapor deposition process can be selected in the same ranges as the conditions described for the vacuum vapor deposition of the hole injecting layer, although the conditions are different depending on the compound to be used.
Next, an electron injecting layer is formed on the light emitting layer formed above. Similarly to the hole injecting layer and the light emitting layer, it is preferred that the electron injecting layer be formed in accordance with the vacuum vapor deposition process since a uniform film must be obtained. The conditions of the vacuum vapor deposition can be selected in the same ranges as the condition described for the vacuum vapor deposition of the hole injecting layer and the light emitting layer.
When the vacuum vapor deposition process is used, the aromatic amine derivative of the present invention can be deposited by vapor in combination with other materials, although the situation may be different depending on which layer in the light emitting zone or in the hole transporting zone includes the compound. When the spin coating process is used, the compound can be incorporated into the formed layer by using a mixture of the compound with other materials.
A cathode is laminated in the last step, and an organic EL device can be obtained.
The cathode is formed of a metal and can be formed in accordance with the vapor deposition process or the sputtering process. It is preferred that the vacuum vapor deposition process be used in order to prevent formation of damages on the lower organic layers during the formation of the film.
In the above-mentioned preparation of the organic EL device, it is preferred that the above-mentioned layers from the anode to the cathode be formed successively while the preparation system is kept in a vacuum after being evacuated once.

The method of forming the layers in the organic EL device of the present invention is not particularly limited. A conventionally known process such as the vacuum vapor deposition process or the spin coating process can be used. The organic thin-film layer which is used in the organic EL device of the present invention and includes the compound represented by the general formula (1) described above can be formed in accordance with a known process such as the vacuum vapor deposition process or the molecular beam epitaxy process (MBE process) or, using a solution prepared by dissolving the compounds into a solvent, in accordance with a coating process such as the dipping process, the spin coating process, the casting process, the bar coating process, or the roll coating process.
The thickness of each layer in the organic layer in the organic EL device of the present invention is not particularly limited. In general, an excessively thin layer tends to have defects such as pin holes, whereas an excessively thick layer requires a high applied voltage to decrease the efficiency. Therefore, a thickness in the range of several nanometers to 1 µm is preferred.
The organic EL device emits light when a direct voltage of 5 to 40 V is applied in the condition that the polarity of the anode is positive (+) and the polarity of the cathode is negative (-). When a voltage is applied in the condition that the polarity is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### Examples

Hereinafter, the present invention is more specifically described by way of examples. However, the present invention is not limited by the following examples without departing from the gist of the present invention.

### <Synthesis Example 1 (synthesis of intermediate 1)>

In a stream of argon, 47 g of 4-bromobiphenyl, 23 g of iodine, 9.4 g of periodic acid dihydrate, 42 mL of water, 360 mL of acetic acid, and 11 mL of sulfuric acid were loaded into a 1,000-mL three-necked flask, and the mixture was stirred at 65 °C for 30 minutes and was then subjected to a reaction at 90 °C for 6 hours. The reactant was poured into ice water, followed by filtering. The resultant was washed with water, and then washed with methanol, whereby 67 g of a white powder were obtained. Main peaks having ratios m/z of 358 and 360 were obtained with respect to C₁₂H₈BrI=359 by a field desorption mass spectrometry (hereinafter, referred to as FD-MS) analysis, so the powder was identified as the intermediate 1.

### <Synthesis Example 2 (synthesis of intermediate 2)>

In a stream of argon, 10 g of di-4-biphenylylamine, 9.7 g of 4,4'-dibromobiphenyl, 3 g of t-butoxysodium, 0.5 g of bis(triphenylphosphine)palladium(II) chloride, and 500 mL of xylene were loaded, and then the mixture was reacted at 130°C for 24 hours.
After the resultant had been cooled, 1,000 mL of water were added to the resultant, and then the mixture was filtered through celite. The filtrate was extracted with toluene and dried with anhydrous magnesium sulfate. The dried product was concentrated under reduced pressure, and then the resultant coarse product was subjected to column purification and recrystallized with toluene. The recrystallized product was taken by filtration, and was then dried. Thus, 9.1 g of a white powder were obtained. The white powder was identified as the intermediate 2 by FD-MS analysis.

### <Synthesis Example 3 (synthesis of intermediate 3)>

Under an argon atmosphere, 600 mL of tetrahydrofuran anhydrous were added to 78.0 g of dibenzofuran, and then the mixture was cooled to -30°C. 300 mL of a solution of n-butyllithium in hexane (1.65 M) were dropped to the mixture, and then the temperature of the resultant mixture was increased to room temperature over 1 hour while the mixture was stirred. After having been stirred at room temperature for 5 hours, the mixture was cooled to -60°C, and then 60 mL of 1,2-dibromoethane were dropped to the mixture over 1 hour.
After having been stirred at room temperature for 15 hours, the resultant mixture was poured into 1,000 mL of ice water, and then the organic layer was extracted with dichloromethane. After having been washed with a saturated salt solution, the organic layer was dried with anhydrous magnesium sulfate, separated by filtration, and then concentrated. The resultant solid was purified by silica gel chromatography (toluene), washed with tetrahydrofuran and methanol, and dried under reduced pressure. Thus, 70 g of a solid were obtained. The solid was identified as the intermediate 3 by FD-MS analysis.

### <Synthesis Example 4 (synthesis of intermediate 4)>

Under a nitrogen atmosphere, 1,000 mL of acetic acid were added to 150 g of dibenzofuran, and then the contents were dissolved under heat. 188 g of bromine were dropped to the solution while the solution was occasionally cooled with water. After that, the mixture was stirred for 20 hours under air cooling. The precipitated crystal was separated by filtration, sequentially washed with acetic acid and water, and dried under reduced pressure. The resultant crystal was purified by distillation under reduced pressure, and was then repeatedly recrystallized with methanol several times. Thus, 66.8 g of a solid were obtained. The solid was identified as the intermediate 4 by FD-MS analysis.

### <Synthesis Example 5 (synthesis of intermediate 5)>

Under an argon atmosphere, 1,000 mL of toluene and 500 mL of an 2M aqueous solution of sodium carbonate were added to 120.0 g of 1-bromo-3-fluoro-4-iodobenzene, 72.7 g of 2-methoxyphenylboronic acid, and 9.2 g of tetrakis(triphenylphosphine)palladium(0), and then the reaction mixture was heated at reflux for 10 hours.
Immediately after the completion of the reaction, the resultant was filtrated, and then the aqueous layer was removed. The organic layer was dried with sodium sulfate, and was then concentrated. The residue was purified by silica gel column chromatography. Thus, 89.6 g of the white crystal of 4-bromo-2-fluoro-2'-methoxybiphenyl were obtained (in 80% yield).
Under an argon atmosphere, 900 mL of dichloromethane were added to 89.6 g of 4-bromo-2-fluoro-2'-methoxybiphenyl, and then the mixture was stirred under ice cooling. 95.9 g of boron tribromide were added dropwise to the mixture, and then the whole was stirred at room temperature for 12 hours. After the completion of the reaction, 200 mL of water were added to the resultant, and then the mixture was stirred for 1 hour. After that, the aqueous layer was removed. The organic layer was dried with magnesium sulfate, and was then concentrated. The residue was purified by silica gel column chromatography. Thus, 68.1 g of the white crystal of 4-bromo-2-fluoro-2'-hydroxybiphenyl were obtained (in 70% yield).
1,500 mL of N-methylpyrrolidone were added to 68.1 g of 4-bromo-2-fluoro-2'-hydroxybiphenyl and 70.4 g of potassium carbonate, and then the mixture was stirred at 180 °C for 3 hours. After the completion of the reaction, water was added to the resultant, and then the mixture was extracted with toluene. The organic layer wasdriedwithsodiumsulfate, and was then concentrated. The residue was recrystallized from toluene and purified. Thus, 44.2 g of the white crystal of 3-bromodibenzofuran were obtained (in 60% yield) . The white crystal was identified as the intermediate 5 by FD-MS analysis.

### <Synthesis Example 6 (synthesis of intermediate 6)>

In a stream of argon, 24.7 g of the intermediate 3, 14.0 g of aniline, 28.8 g of t-butoxysodium, 1.4 g of tris(dibenzylideneacetone)dipalladium(0), 1.9 g of 2,2'-bis(diphenylphosphino)-1,1'-binaphthyl, and 350 mL of toluene were loaded, and then the mixture was reacted at 130°C for 24 hours.
After cooling, the mixture was filtered through celite, and then the filtrate was concentrated under reduced pressure. The resultant coarse product was subjected to column purification and recrystallized with toluene. The recrystallized product was taken by filtration, and was then dried. Thus, 20.7 g of a white powder were obtained. The white powder was identified as the intermediate 6 by FD-MS analysis.

### <Synthesis Example 7 (synthesis of intermediate 7)>

A reaction was performed in the same manner as in Synthesis Example 6 except that 24.7 g of the intermediate 4 were used instead of the intermediate 3. Thus, 22.6 g of a white powder were obtained. The powder was identified as the intermediate 7 by FD-MS analysis.

### <Synthesis Example 8 (synthesis of intermediate 8)>

A reaction was performed in the same manner as in Synthesis Example 6 except that 24.7 g of the intermediate 5 were used instead of the intermediate 3. Thus, 23.8 g of a white powder were obtained. The powder was identified as the intermediate 8 by FD-MS analysis.

### <Synthesis Example 9 (synthesis of intermediate 9)>

5.90 mL of sulfuric acid and ethanol were added to 17.7 g of 9-phenylcarbazole, 6.03g of potassium iodide, and 7.78 g of potassium iodate, and then the mixture was reacted at 75°C for 2 hours.
After the resultant had been cooled, water and ethyl acetate were added and extracted the resultant. After that, the organic layer was washed with baking soda water and water, and was then concentrated. The resultant coarse product was purified by silica gel chromatography (developing solvent: toluene), and then the resultant solid was dried under reduced pressure. Thus, 21.8 g of a white solid were obtained. The white solid was identified as the intermediate 9 by FD-MS analysis.

### <Synthesis Example 10 (synthesis of intermediate 10)>

A reaction was performed in the same manner as in Synthesis Example 6 except that 36.9 g of the intermediate 9 were used instead of the intermediate 3. Thus, 20.8 g of a white powder were obtained. The powder was identified as the intermediate 10 by FD-MS analysis.

### <Synthesis Example 11 (synthesis of intermediate 11)>

A reaction was performed in the same manner as in Synthesis Example 6 except that: 35.9 g of the intermediate 1 were used instead of the intermediate 3; and 33.4 g of the intermediate 10 were used instead of aniline. Thus, 43.8 g of a white powder were obtained. The powder was identified as the intermediate 11 by FD-MS analysis.

### <Synthesis Example 12 (synthesis of intermediate 12)>

Under an argon atmosphere, 37.0 g of 1-acetamide, 49.4 g of the intermediate 3, 109 g of potassium carbonate and 2.5 g of a copper powder in 400 mL of decalin were reacted at 190°C for 4 days. After the reaction, the resultant was cooled, 200 mL of toluene were added to the resultant, and insoluble matter was taken by filtration. The product taken by filtration was dissolved in 500 mL of chloroform, and then insoluble matter was removed. After that, the residue was treated with activated carbon and concentrated. 300 mL of acetone were added to the concentrate, and then 27.6 g of the precipitated crystal were taken by filtration. The crystal was identified as the intermediate 12 by FD-MS analysis.

### <Synthesis Example 13 (synthesis of intermediate 13)>

A reaction was performed in the same manner as in Synthesis Example 12 except that: 22.5 g of the intermediate 12 were used instead of 1-acetamide; and 20.7 g of 1-bromonaphthalene were used instead of the intermediate 3. Thus, 21.0 g of a pale yellow powder were obtained. The pale yellow powder was identified as the intermediate 13 by FD-MS analysis.

### <Synthesis Example 14 (synthesis of intermediate 14)>

In a streamof argon, 21.0 g of the intermediate 13 were suspended in 500 mL of xylene and 50 mL of water, and then 21 g of an 85% aqueous solution of potassium hydroxide were added to the suspension. After that, the mixture was reacted at 120°C for 8 hours. After the reaction, the reaction liquid was poured into 1 L of water. The precipitated crystal was taken by filtration, and was then washed with water and methanol. The resultant crystal was dissolved in 300 mL of tetrahydrofuran under heat. The solution was treated with activated carbon, and was then concentrated. Acetone was added to the concentrate to precipitate a crystal. The crystal was taken by filtration. Thus, 15.1 g of a white powder were obtained. The white powder was identified as the intermediate 14 by FD-MS analysis.

### <Synthesis Example 15 (synthesis of intermediate 15) >

A reaction was performed in the same manner as in Synthesis Example 13 except that 23.3 g of 4-bromophenyl were used instead of the intermediate 3. Thus, 22.6 g of a pale yellow powder were obtained. The powder was identified as the intermediate 15 by FD-MS analysis.

### <Synthesis Example 16 (synthesis of intermediate 16)>

A reaction was performed in the same manner as in Synthesis Example 14 except that 22.6 g of the intermediate 15 were used instead of the intermediate 13. Thus, 16.0 g of a pale yellow powder were obtained. The powder was identified as the intermediate 16 by FD-MS analysis.

### <Synthesis Example 17 (synthesis of intermediate 17)>

A reaction was performed in the same manner as in Synthesis Example 12 except that 49.4 g of the intermediate 4 were used instead of the intermediate 3. Thus, 25.3 g of a pale yellow powder were obtained. The powder was identified as the intermediate 17 by FD-MS analysis.

### <Synthesis Example 18 (synthesis of intermediate 18) >

A reaction was performed in the same manner as in Synthesis Example 13 except that 22. 5 g of the intermediate 17 were used instead of the intermediate 12. Thus, 23.2 g of a pale yellow powder were obtained. The powder was identified as the intermediate 18 by FD-MS analysis.

### <Synthesis Example 19 (synthesis of intermediate 19)>

A reaction was performed in the same manner as in Synthesis Example 14 except that 23.2 g of the intermediate 18 were used instead of the intermediate 13. Thus, 16.7 g of a pale yellow powder were obtained. The powder was identified as the intermediate 19 by FD-MS analysis.

### <Synthesis Example 20 (synthesis of intermediate 20)>

A reaction was performed in the same manner as in Synthesis Example 12 except that 49.4 g of the intermediate 5 were used instead of the intermediate 3. Thus, 24.7 g of a pale yellow powder were obtained. The powder was identified as the intermediate 20 by FD-MS analysis.

### <Synthesis Example 21 (synthesis of intermediate 21)>

A reaction was performed in the same manner as in Synthesis Example 13 except that 22. 5 g of the intermediate 20 were used instead of the intermediate 12. Thus, 21.8 g of a pale yellow powder were obtained. The powder was identified as the intermediate 21 by FD-MS analysis.

### <Synthesis Example 22 (synthesis of intermediate 22)>

A reaction was performed in the same manner as in Synthesis Example 14 except that 21.8 g of the intermediate 21 were used instead of the intermediate 13. Thus, 13.5 g of a pale yellow powder were obtained. The powder was identified as the intermediate 22 by FD-MS analysis.

### <Synthesis Example 23 (synthesis of intermediate 23)>

A reaction was performed in the same manner as in Synthesis Example 13 except that: 22.5 g of the intermediate 20 were used instead of the intermediate 12; and 23.3 g of 4-bromophenyl were used instead of the intermediate 3. Thus, 21.4 g of a pale yellow powder were obtained. The powder was identified as the intermediate 23 by FD-MS analysis.

### <Synthesis Example 24 (synthesis of intermediate 24)>

A reaction was performed in the same manner as in Synthesis Example 14 except that 21.4 g of the intermediate 23 were used instead of the intermediate 13. Thus, 13.8 g of a pale yellow powder were obtained. The powder was identified as the intermediate 24 by FD-MS analysis.

The structural formulae of the intermediates synthesized in Synthesis Examples 1 to 24 are as shown below.

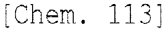

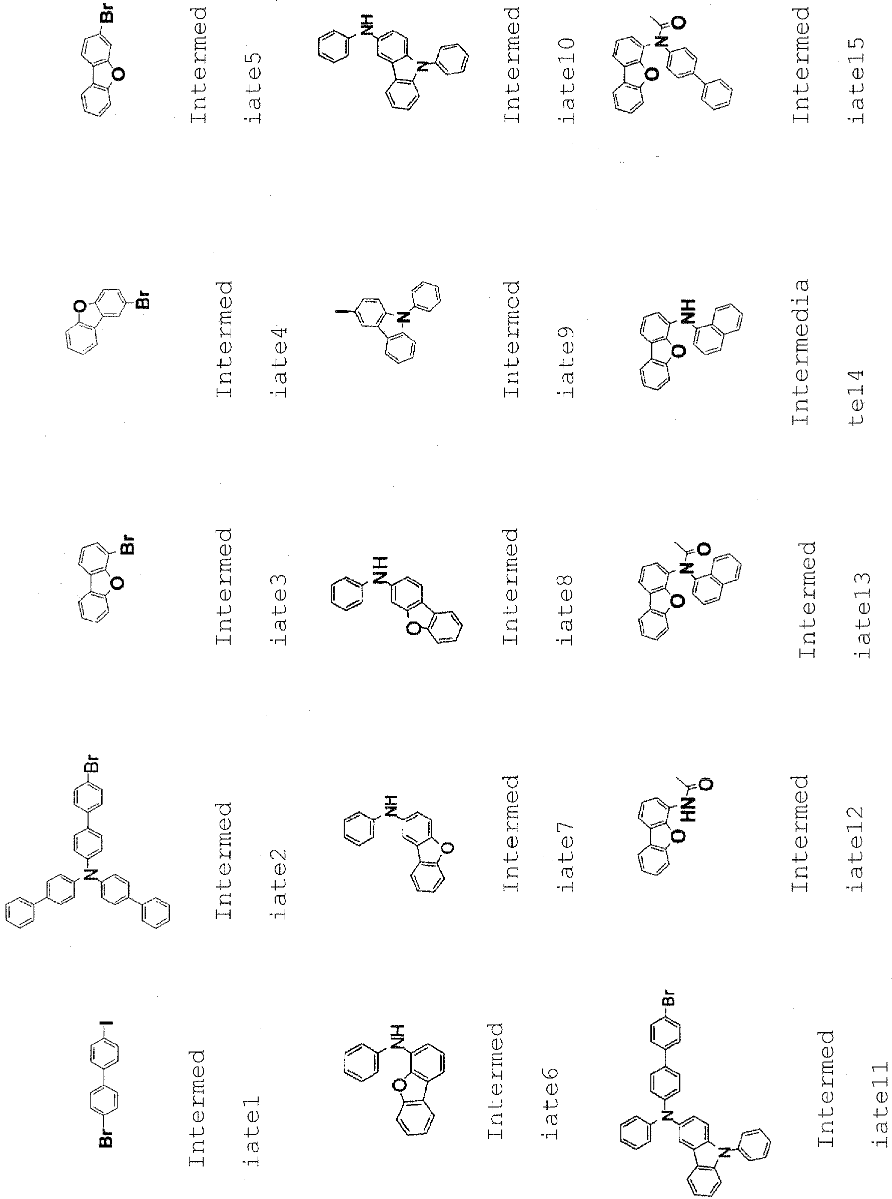

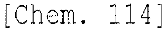

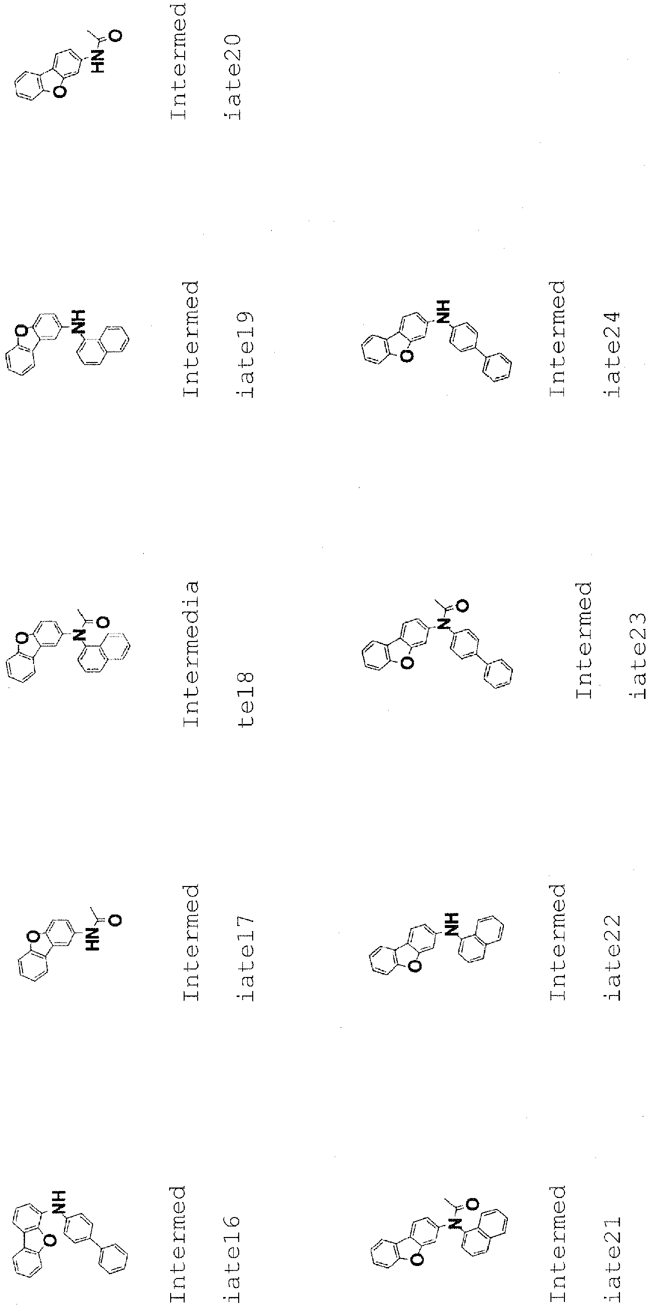

### <Synthesis Embodiment 1 (production of aromatic amine derivative (H1))>

In a stream of argon, 5.2 g of the intermediate 6, 4.1 g of 4,4'-diiodobiphenyl, 1.3 g of t-butoxy sodium, 46 mg of tris(dibenzylideneacetone)dipalladium, 21 mg of tri-t-butylphosphine, and 50 mL of toluene anhydrous were, and then the mixture was reacted at 80°C for 2 hours.
After cooling, 500 mL of water were added, and then the mixture was filtered through celite filtration. The filtrate was extracted with toluene and dried with anhydrous magnesium sulfate. The dried product was concentrated under reduced pressure, and then the resultant coarse product was subjected to column purification. The purified product was recrystallized with toluene, and then the recrystallized product was taken by filtration. After that, the resultant was dried. Thus, 4.7 g of a pale yellow powder were obtained. The pale yellow powder was identified as the aromatic amine derivative (H1) by FD-MS analysis.

### <Synthesis Embodiment 2 (production of aromatic amine derivative (H2))>

A reaction was performed in the same manner as in Synthesis Embodiment 1 except that 5.2 g of the intermediate 8 were used instead of the intermediate 6. Thus, 4.3 g of a pale yellow powder were obtained. The pale yellow powder was identified as the aromatic amine derivative (H2) by FD-MS analysis.

### <Synthesis Embodiment 3 (production of aromatic amine derivative (H3))>

A reaction was performed in the same manner as in Synthesis Embodiment 1 except that: 2.6 g of the intermediate 6 were used; and 5.5 g of the intermediate 2 were used instead of 4,4'-diiodobiphenyl. Thus, 5.0 g of a pale yellow powder were obtained. The pale yellow powder was identified as the aromatic amine derivative (H3) by FD-MS analysis.

### <Synthesis Embodiment 4 (production of aromatic amine derivative (H4))>

A reaction was performed in the same manner as in Synthesis Embodiment 1 except that: 2.6 g of the intermediate 7 were used instead of the intermediate 6; and 5.5 g of the intermediate 2 were used instead of 4, 4'-diiodobiphenyl. Thus, 5.1 g of a pale yellow powder were obtained. The pale yellow powder was identified as the aromatic amine derivative (H4) by FD-MS analysis.

### <Synthesis Embodiment 5 (production of aromatic amine derivative (H5))>

A reaction was performed in the same manner as in Synthesis Embodiment 1 except that: 2.6 g of the intermediate 8 were used instead of the intermediate 6; and 5.5 g of the intermediate 2 were used instead of 4,4'-diiodobiphenyl. Thus, 4.9 g of a pale yellow powder were obtained. The pale yellow powder was identified as the aromatic amine derivative (H5) by FD-MS analysis.

### <Synthesis Embodiment 6 (production of aromatic amine derivative (H6))>

A reaction was performed in the same manner as in Synthesis Embodiment 1 except that 6.7 g of the intermediate 16 were used instead of the intermediate 6. Thus, 5.7 g of a pale yellow powder were obtained. The pale yellow powder was identified as the aromatic amine derivative (H6) by FD-MS analysis.

### <Synthesis Embodiment 7 (production of aromatic amine derivative (H7))>

A reaction was performed in the same manner as in Synthesis Embodiment 1 except that 6.7 g of the intermediate 24 were used instead of the intermediate 6. Thus, 5.5 g of a pale yellow powder were obtained. The pale yellow powder was identified as the aromatic amine derivative (H7) by FD-MS analysis.

### <Synthesis Embodiment 8 (production of aromatic amine derivative (H8))>

A reaction was performed in the same manner as in Synthesis Embodiment 1 except that 6.2 g of the intermediate 14 were used instead of the intermediate 6. Thus, 5.3 g of a pale yellow powder were obtained. The pale yellow powder was identified as the aromatic amine derivative (H8) by FD-MS analysis.

### <Synthesis Embodiment 9 (production of aromatic amine derivative (H9))>

A reaction was performed in the same manner as in Synthesis Embodiment 1 except that 6.2 g of the intermediate 19 were used instead of the intermediate 6. Thus, 5.2 g of a pale yellow powder were obtained. The pale yellow powder was identified as the aromatic amine derivative (H9) by FD-MS analysis.

### <Synthesis Embodiment 10 (production of aromatic amine derivative (H10))>

A reaction was performed in the same manner as in Synthesis Embodiment 1 except that 6.2 g of the intermediate 22 were used instead of the intermediate 6. Thus, 4. 8 g of a pale yellow powder were obtained. The pale yellow powder was identified as the aromatic amine derivative (H10) by FD-MS analysis.

### <Synthesis Embodiment 11 (production of aromatic amine derivative (H11))>

A reaction was performed in the same manner as in Synthesis Embodiment 1 except that 4.8 g of 4, 4'-diiodo-p-terphenyl were used instead of 4,4'-diiodobiphenyl. Thus, 5.5 g of a pale yellow powder were obtained. The pale yellow powder was identified as the aromatic amine derivative (H11) by FD-MS analysis.

### <Synthesis Embodiment 12 (production of aromatic amine derivative (H12))>

A reaction was performed in the same manner as in Synthesis Embodiment 1 except that: 5.2 g of the intermediate 7 were used instead of the intermediate 6; and 4.8 g of 4,4'-diiodo-p-terphenyl were used instead of 4,4'-diiodobiphenyl. Thus, 6.0 g of a pale yellow powder were obtained. The pale yellow powder was identified as the aromatic amine derivative (H12) by FD-MS analysis.

### <Synthesis Embodiment 13 (production of aromatic amine derivative (H13))>

A reaction was performed in the same manner as in Synthesis Embodiment 1 except that: 2.6 g of the intermediate 6 were used; and 5.7 g of the intermediate 11 were used instead of 4,4'-diiodobiphenyl. Thus, 5.0 g of a pale yellow powder were obtained. The pale yellow powder was identified as the aromatic amine derivative (H13) by FD-MS analysis.

### <Synthesis Embodiment 14 (production of aromatic amine derivative (H14))>

A reaction was performed in the same manner as in Synthesis Embodiment 1 except that: 2.6 g of the intermediate 7 were used instead of the intermediate 6; and 5.7 g of the intermediate 11 were used instead of 4,4'-diiodobiphenyl. Thus, 4.8 g of a pale yellow powder were obtained. The pale yellow powder was identified as the aromatic amine derivative (H14) by FD-MS analysis.

### <Synthesis Embodiment 15 (production of aromatic amine derivative (H15))>

A reaction was performed in the same manner as in Synthesis Embodiment 1 except that: 2.6 g of the intermediate 8 were used instead of the intermediate 6; and 5.7 g of the intermediate 11 were used instead of 4,4'-diiodobiphenyl. Thus, 5.3 g of a pale yellow powder were obtained. The pale yellow powder was identified as the aromatic amine derivative (H15) by FD-MS analysis.

The structural formulae of the aromatic amine derivatives synthesized in Synthesis Embodiments 1 to 15 are as shown below.

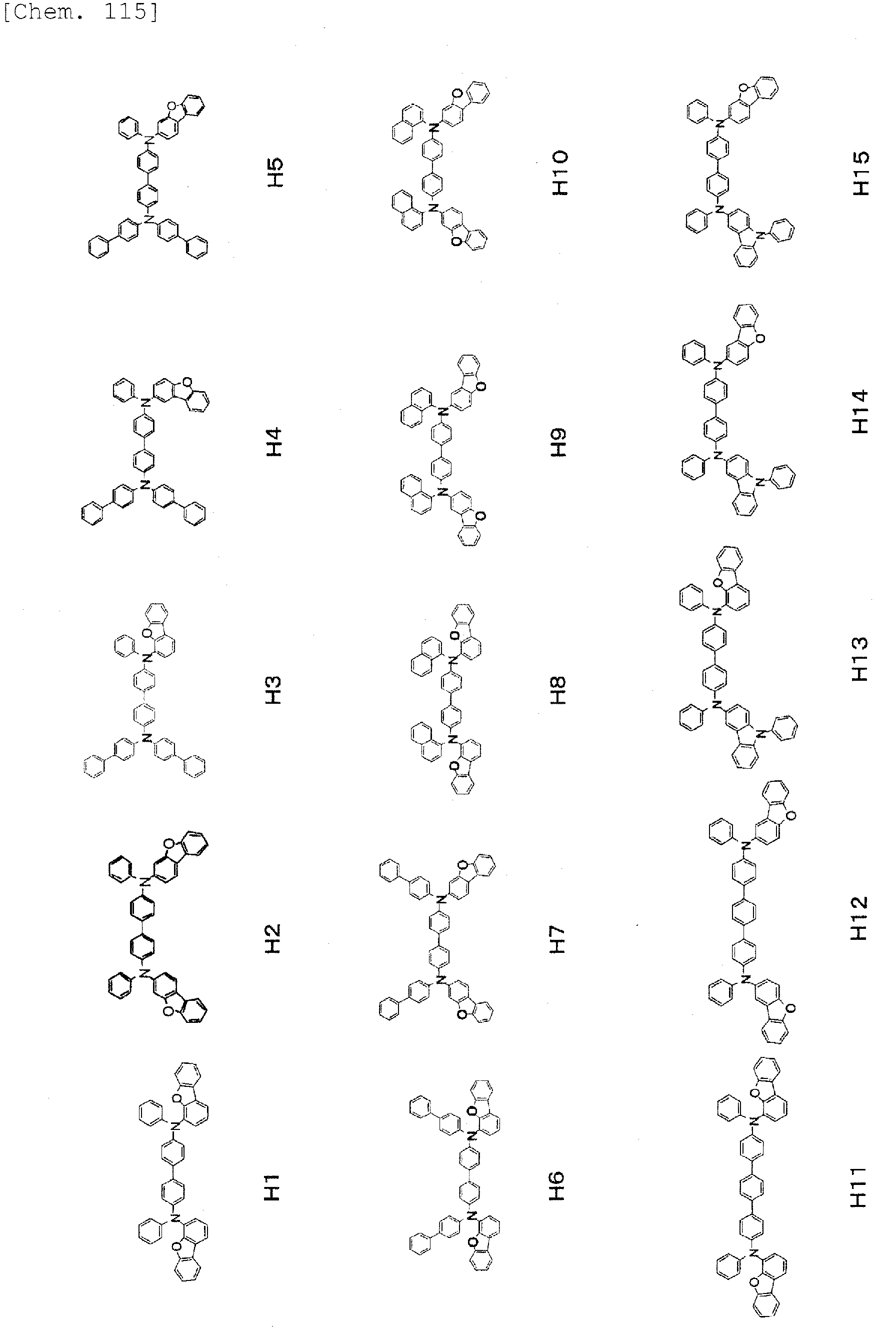

### [Example 1-1 (production of organic EL device)]

A glass substrate with an ITO transparent electrode measuring 25 mm wide by 75 mm long by 1.1 mm thick (manufactured by GEOMATEC Co. , Ltd.) was subj ected to ultrasonic cleaning in isopropyl alcohol for 5 minutes. After that, the substrate was subjected to UV ozone cleaning for 30 minutes.
The glass substrate with the transparent electrode line after the cleaning was mounted on a substrate holder of a vacuum vapor deposition device. First, the following electron-accepting compound (C-1) was deposited from vapor on the surface on the side where the transparent electrode line was formed so as to cover the transparent electrode. Thus, the C-1 film having a thickness of 10 nm was formed. The aromatic amine derivative (H1) obtained in Synthesis Embodiment 1 was deposited from vapor as a hole transporting material and formed into a hole transporting layer having a thickness of 70 nm on the C-1 film. Further, the following compound EM1 was deposited from vapor and formed into a light emitting layer having a thickness of 40 nm. Simultaneously with this formation, the following styrylamine derivative (D1), as a light emitting molecule, was deposited from vapor in such a manner that a weight ratio between the EM1 and the D1 (EM1:D1) was 40:2.
The following organic metal complex (Alq) was formed into a film having a thickness of 10 nm on the resultant film. The film functions as an electron injecting layer. After that, Li serving as a reduction-causing dopant (Li source: manufactured by SAES Getters) and the Alq were subjected to co-vapor deposition. Thus, an Alq: Li film (having a thickness of 10 nm) was formed as an electron injecting layer (cathode). Metal Al was deposited from vapor onto the Alq:Li film to form a metal cathode. Thus, an organic EL device was formed.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 1 shows the results.

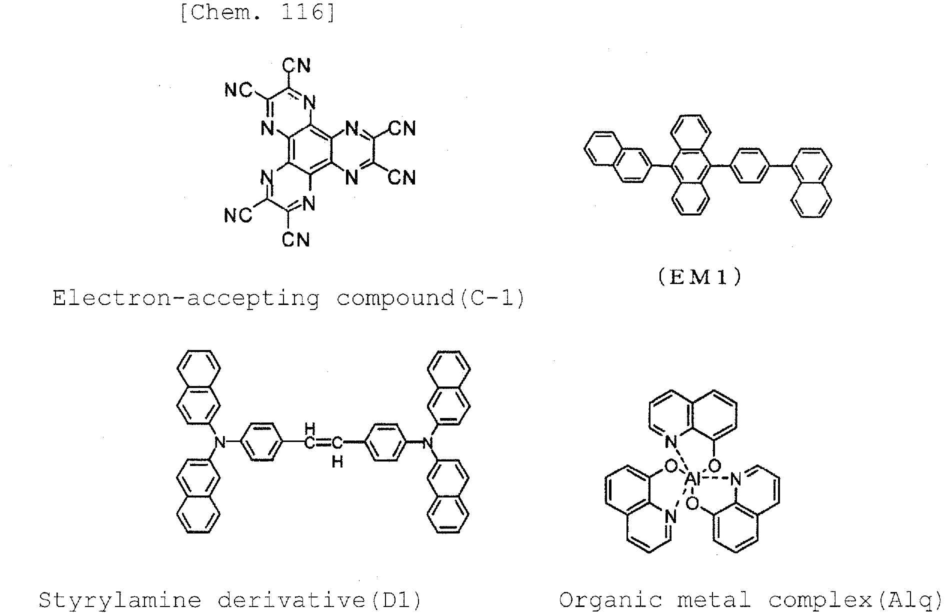

### [Examples 1-2 to 1-10 (production of organic EL devices)]

Organic EL devices were each produced in the same manner as in Example 1-1 except that each of the aromatic amine derivatives shown in Table 1 was used instead of the aromatic amine derivative (H1) as a hole transporting material.
The luminescent colors of the resultant organic EL devices were observed. Further, their current efficiencies, driving voltages, and light emission half lifetimes when the devices were each driven with a DC constant current at an initial luminance of 5,000 cd/m² and room temperature were measured. Table 1 shows the results.

### [Example 1-11 (production of organic EL device)]

An organic EL devices was produced in the same manner as in Example 1-1 except that the following arylamine derivative (D2) was used instead of the styrylamine derivative (D1).
The luminescent color of the resultant organic EL device was observed. Further, its current efficiency, driving voltage, and light emission half lifetime when the device was driven with a DC constant current at an initial luminance of 5,000 cd/m² and room temperature were measured. Table 1 shows the results.

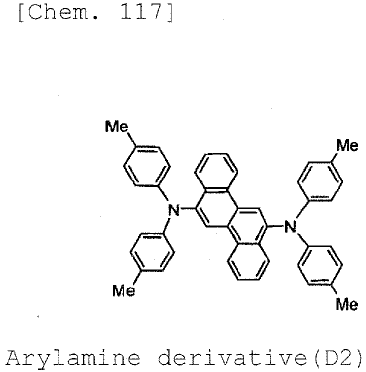

### [Example 1-12 (production of organic EL device)]

An organic EL device was produced in the same manner as in Example 1-1 except that the following benzimidazole derivative (ET1) was used instead of the organic metal complex (Alq) as an electron transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 1 shows the results.

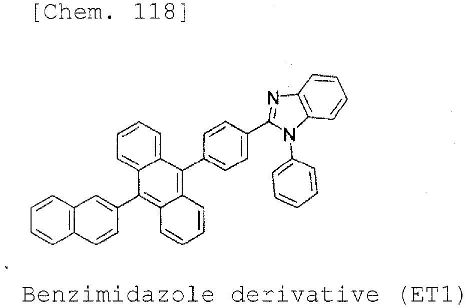

### [Example 1-13 (production of organic EL device)]

An organic EL device was produced in the same manner as in Example 1-1 except that the following phenanthroline derivative (ET2) was used instead of the organic metal complex (Alq) as an electron transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 1 shows the results.

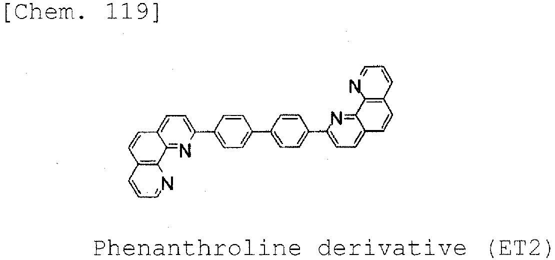

### [Example 1-14 (production of organic EL device)]

An organic EL device was produced in the same manner as in Example 1-1 except that the following phosphine oxide derivative (ET3) was used instead of the organic metal complex (Alq) as an electron transporting material. The luminescent color of the resultant organic EL device was observed. Further, its current efficiency, driving voltage, and light emission half lifetime when the device was driven with a DC constant current at an initial luminance of 5, 000 cd/m² and room temperature were measured. Table 1 shows the results.

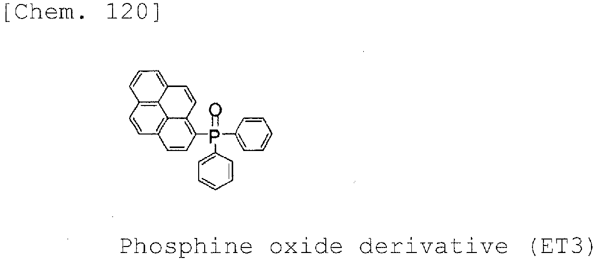

### [Comparative Examples 1-1 to 1-3]

Organic EL devices were each produced in the same manner as in Example 1-1 except that any one of the following comparative compounds 1 to 3 shown in Table 1 was used instead of the aromatic amine derivative (H1) as a hole transporting material.
The luminescent colors of the resultant organic EL devices were observed. Further, their current efficiencies, driving voltages, and light emission half lifetimes when the devices were each driven with a DC constant current at an initial luminance of 5,000 cd/m² and room temperature were measured. Table 1 shows the results.

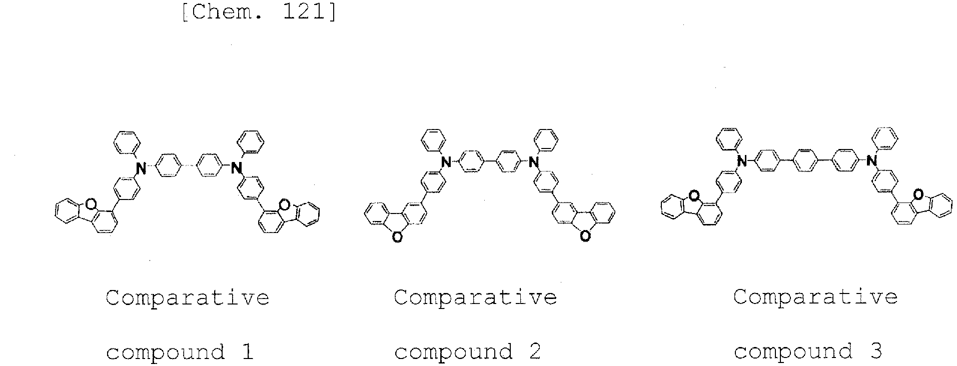

### [Comparative Example 1-4]

An organic EL device was produced in the same manner as in Example 1-11 except that the comparative compound 1 was used instead of the aromatic amine derivative (H1) as a hole transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 1 shows the results.

### [Comparative Example 1-5]

An organic EL device was produced in the same manner as in Example 1-12 except that the comparative compound 1 was used instead of the aromatic amine derivative (H1) as a hole transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 1 shows the results.

[Table 1]

**Table 1**

| | | Hole transporting material | Luminescent color | Results of measurement | | |
|---|---|---|---|---|---|---|
| | | | | Current efficiency (cd/A) | Driving voltage (V) | Half lifetime (hour(s)) |
| Example | 1-1 | H1 | Blue | 7.2 | 7.2 | 410 |
| | 1-2 | H2 | Blue | 7.0 | 6.8 | 390 |
| | 1-3 | H3 | Blue | 7.2 | 7.2 | 390 |
| | 1-4 | H4 | Blue | 7.2 | 7.0 | 400 |
| | 1-5 | H5 | Blue | 7.1 | 6.8 | 370 |
| | 1-6 | H6 | Blue | 7.2 | 7.2 | 400 |
| | 1-7 | H7 | Blue | 7.0 | 6.6 | 370 |
| | 1-8 | H8 | Blue | 7.2 | 7.2 | 390 |
| | 1-9 | H9 | Blue | 7.0 | 7.0 | 390 |
| | 1-10 | H10 | Blue | 6.9 | 6.5 | 360 |
| | 1-11 | H1 | Blue | 7.2 | 6.9 | 390 |
| | 1-12 | H1 | Blue | 7.1 | 6.6 | 380 |
| | 1-13 | H1 | Blue | 7.1 | 6.5 | 380 |
| | 1-14 | H1 | Blue | 7.0 | 6.5 | 370 |
| Comparative Example | 1-1 | Comparative compound 1 | Blue | 6.3 | 7.8 | 260 |
| | 1-2 | Comparative compound 2 | Blue | 6.0 | 7.9 | 250 |
| | 1-3 | Comparative compound 3 | Blue | 6.0 | 7.8 | 240 |
| | 1-4 | Comparative compound 1 | Blue | 6.5 | 7.9 | 250 |
| | 1-5 | Comparative compound 1 | Blue | 6.4 | 7.5 | 250 |

As can be seen from Table 1, an organic EL device using the aromatic amine derivative of the present invention can be driven at a low voltage, can obtain high current efficiency, and has a device lifetime extended as compared with a known organic EL device using an aromatic amine derivative.

### [Example 2-1 (production of organic EL device)]

A glass substrate with an ITO transparent electrode measuring 25 mm wide by 75 mm long by 1.1 mm thick (manufactured by GEOMATEC Co. , Ltd.) was subjected to ultrasonic cleaning in isopropyl alcohol for 5 minutes. After that, the substrate was subjected to UV ozone cleaning for 30 minutes.
The glass substrate with the transparent electrode line after the cleaning was mounted on a substrate holder of a vacuum vapor deposition device. First, the following electron-accepting compound (C-2) was deposited from vapor on the surface on the side where the transparent electrode line was formed so as to cover the transparent electrode. Thus, the C-2 film having a thickness of 10 nm was formed. The aromatic amine derivative (H1) obtained in Synthesis Embodiment 1 was deposited from vapor as a hole transporting material and formed into a hole transporting layer having a thickness of 70 nm on the C-2 film. Further, the compound EM1 was deposited from vapor and formed into a light emitting layer having a thickness of 40 nm. Simultaneously with this formation, the styrylamine derivative (D1), as a light emitting molecule, was deposited from vapor in such a manner that a weight ratio between the EM1 and the D1 (EM1:D1) was 40:2.
The organic metal complex (Alq) was formed into a film having a thickness of 10 nm on the resultant film. The film functions as an electron injecting layer. After that, Li serving as a reduction-causing dopant (Li source: manufactured by SAES Getters) and the Alq were subjected to co-vapor deposition. Thus, an Alq:Li film (having a thickness of 10 nm) was formed as an electron injecting layer (cathode). Metal Al was deposited from vapor onto the Alq:Li film to form a metal cathode. Thus, an organic EL device was formed.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 2 shows the results.

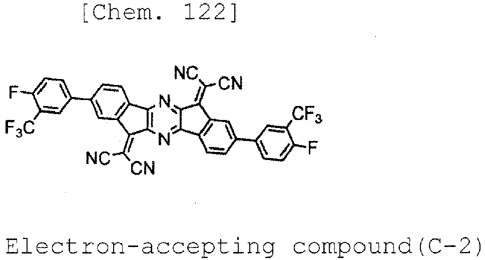

### [Examples 2-2 to 2-10 (production of organic EL devices)]

Organic EL devices were each produced in the same manner as in Example 2-1 except that each of the aromatic amine derivatives shown in Table 2 was used instead of the aromatic amine derivative (H1) as a hole transporting material.
The luminescent colors of the resultant organic EL devices were observed. Further, their current efficiencies, driving voltages, and light emission half lifetimes when the devices were each driven with a DC constant current at an initial luminance of 5,000 cd/m² and room temperature were measured. Table 2 shows the results.

### [Example 2-11 (production of organic EL device)]

An organic EL device was produced in the same manner as in Example 2-1 except that the arylamine derivative (D2) was used instead of the styrylamine derivative (D1) as a hole transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 2 shows the results.

### [Example 2-12 (production of organic EL device)]

An organic EL device was produced in the same manner as in Example 2-1 except that the benzimidazole derivative (ET1) was used instead of the organicmetal complex (Alq) as an electron transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 2 shows the results.

### [Example 2-13 (production of organic EL device)]

An organic EL device was produced in the same manner as in Example 2-1 except that the phenanthroline derivative (ET2) was used instead of the organic metal complex (Alq) as an electron transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 2 shows the results.

### [Example 2-14 (production of organic EL device)]

An organic EL device was produced in the same manner as in Example 2-1 except that the phosphine oxide derivative (ET3) was used instead of the organic metal complex (Alq) as an electron transporting material. The luminescent color of the resultant organic EL device was observed. Further, its current efficiency, driving voltage, and light emission half lifetime when the device was driven with a DC constant current at an initial luminance of 5,000 cd/m² and room temperature were measured. Table 2 shows the results.

### [Comparative Examples 2-1 to 2-3]

Organic EL devices were each produced in the same manner as in Example 2-1 except that any one of the comparative compounds 1 to 3 as shown in Table 2 was used instead of the aromatic amine derivative (H1) as a hole transporting material.
The luminescent colors of the resultant organic EL devices were observed. Further, their current efficiencies, driving voltages, and light emission half lifetimes when the devices were each driven with a DC constant current at an initial luminance of 5,000 cd/m² and room temperature were measured. Table 2 shows the results.

### [Comparative Example 2-4]

An organic EL device was produced in the same manner as in Example 2-11 except that the comparative compound 1 was used instead of the aromatic amine derivative (H1) as a hole transportingmaterial.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 2 shows the results.

### [Comparative Example 2-5]

An organic EL device was produced in the same manner as in Example 2-12 except that the comparative compound 1 was used instead of the aromatic amine derivative (H1) as a hole transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 2 shows the results.

[Table 2]

**Table 2**

| | | Hole transporting material | Luminescent color | Results of measurement | | |
|---|---|---|---|---|---|---|
| | | | | Current efficiency (cd/A) | Driving voltage (V) | Half lifetime (hour(s)) |
| Example | 2-1 | H1 | Blue | 7.2 | 7.3 | 400 |
| | 2-2 | H2 | Blue | 6.9 | 6.8 | 380 |
| | 2-3 | H3 | Blue | 7.2 | 7.3 | 390 |
| | 2-4 | H4 | Blue | 7.2 | 7.0 | 400 |
| | 2-5 | H5 | Blue | 7.0 | 6.8 | 370 |
| | 2-6 | H6 | Blue | 7.0 | 7.2 | 400 |
| | 2-7 | H7 | Blue | 6.8 | 6.6 | 370 |
| | 2-8 | H8 | Blue | 7.1 | 7.2 | 400 |
| | 2-9 | F9 | Blue | 7.1 | 7.0 | 400 |
| | 2-10 | H10 | Blue | 6.8 | 6.5 | 370 |
| | 2-11 | H1 | Blue | 7.2 | 6.9 | 390 |
| | 2-12 | H1 | Blue | 7.0 | 6.6 | 380 |
| | 2-13 | H1 | Blue | 7.1 | 6.5 | 370 |
| | 2-14 | H1 | Blue | 7.0 | 6.6 | 370 |
| Comparative Example | 2-1 | Comparative compound 1 | Blue | 6.3 | 7.8 | 260 |
| | 2-2 | Comparative compound 2 | Blue | 6.0 | 8.0 | 260 |
| | 2-3 | Comparative compound 3 | Blue | 6.0 | 7.8 | 240 |
| | 2-4 | Comparative compound 1 | Blue | 6.5 | 7.9 | 250 |
| | 2-5 | Comparative compound 1 | Blue | 6.3 | 7.5 | 260 |

As can be seen from Table 2, an organic EL device using the aromatic amine derivative of the present invention can be driven at a low voltage, can obtain high current efficiency, and has a device lifetime extended as compared with a known organic EL device using an aromatic amine derivative.

### [Example 3-1 (production of organic EL device)]

A glass substrate with an ITO transparent electrode measuring 25 mm wide by 75 mm long by 1.1 mm thick (manufactured by GEOMATEC Co. , Ltd.) was subj ected to ultrasonic cleaning in isopropyl alcohol for 5 minutes. After that, the substrate was subjected to UV ozone cleaning for 30 minutes.
The glass substrate with the transparent electrode line after the cleaning was mounted on a substrate holder of a vacuum vapor deposition device. First, the following aromatic tertiary amine derivative (H232) was deposited from vapor on the surface on the side where the transparent electrode line was formed so as to cover the transparent electrode. Thus, the H232 film having a thickness of 60 nm was formed as the hole injecting layer. The aromatic amine derivative (H1) obtained in Synthesis Embodiment 1 was deposited from vapor as a hole transporting material and formed into a hole transporting layer having a thickness of 20 nm on the H232 film. Further, the compound EM1 was deposited from vapor and formed into a light emitting layer having a thickness of 40 nm. Simultaneously with this formation, the styrylamine derivative (D1), as a light emitting molecule, was deposited from vapor in such a manner that a weight ratio between the EM1 and the D1 (EM1:D1) was 40:2.
The organic metal complex (Alq) was formed into a film having a thickness of 10 nm on the resultant film. The film functions as an electron injecting layer. After that, Li serving as a reduction-causing dopant (Li source: manufactured by SAES Getters) and the Alq were subjected to co-vapor deposition. Thus, an Alq:Li film (having a thickness of 10 nm) was formed as an electron injecting layer (cathode). Metal A1 was deposited from vapor onto the Alq:Li film to form a metal cathode. Thus, an organic EL device was formed.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 3 shows the results.

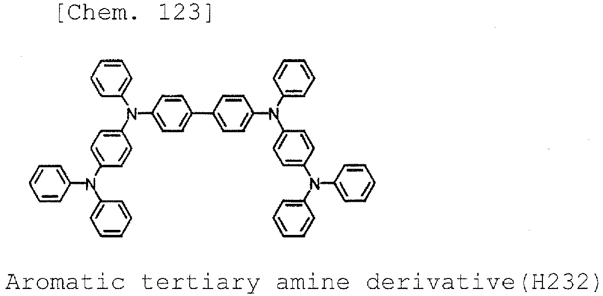

### [Examples 3-2 to 3-10 (production of organic EL devices) ]

Organic EL devices were each produced in the same manner as in Example 3-1 except that each of the aromatic amine derivatives shown in Table 3 was used instead of the aromatic amine derivative (H1) as a hole transporting material.
The luminescent colors of the resultant organic EL devices were observed. Further, their current efficiencies, driving voltages, and light emission half lifetimes when the devices were each driven with a DC constant current at an initial luminance of 5,000 cd/m² and room temperature were measured. Table 3 shows the results.

### [Example 3-11 (production of organic EL device)]

An organic EL device was produced in the same manner as in Example 3-1 except that the aryl amine derivative (D2) was used instead of the styrylamine derivative (D1) as a hole transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 3 shows the results.

### [Example 3-12 (production of organic EL device)]

An organic EL device was produced in the same manner as in Example 3-1 except that the benzimidazole derivative (ET1) was used instead of the organic metal complex (Alq) as an electron transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 3 shows the results.

### [Example 3-13 (production of organic EL device)]

An organic EL device was produced in the same manner as in Example 3-1 except that the following compound H16 as shown in Table 3 was used instead of the aromatic amine derivative (H1) as a hole transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 3 shows the results.

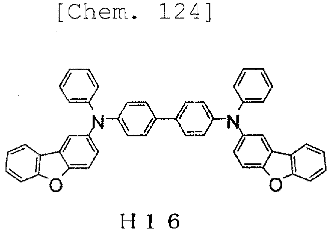

### [Comparative Examples 3-1 to 3-3]

Organic EL devices were each produced in the same manner as in Example 3-1 except that any one of the comparative compounds 1 to 3 as shown in Table 3 was used instead of the aromatic amine derivative (H1) as a hole transporting material.
The luminescent colors of the resultant organic EL devices were observed. Further, their current efficiencies, driving voltages, and light emission half lifetimes when the devices were each driven with a DC constant current at an initial luminance of 5,000 cd/m² and room temperature were measured. Table 3 shows the results.

### [Comparative Example 3-4]

An organic EL device was produced in the same manner as in Example 3-11 except that the comparative compound 1 was used instead of the aromatic amine derivative (H1) as a hole transportingmaterial .
The luminescent color of the resultant organic EL device was observed. Further, its current efficiency, driving voltage, and light emission half lifetime when the device was driven with a DC constant current at an initial luminance of 5,000 cd/m² and room temperature were measured. Table 3 shows the results.

### [Comparative Example 3-5]

An organic EL device was produced in the same manner as in Example 3-12 except that the comparative compound 1 was used instead of the aromatic amine derivative (H1) as a hole transporting material.
The luminescent color of the resultant organic EL device was observed. Further, its current efficiency, driving voltage, and light emission half lifetime when the device was driven with a DC constant current at an initial luminance of 5,000 cd/m² and room temperature were measured. Table 3 shows the results.

[Table 3]

**Table 3**

| | | Hole transporting material | Luminescent color | Results of measurement | | |
|---|---|---|---|---|---|---|
| | | | | Current efficiency (cd/A) | Driving voltage (V) | Half lifetime (hour (s)) |
| Example | 3-1 | H1 | Blue | 7.2 | 7.3 | 400 |
| | 3-2 | H2 | Blue | 6.8 | 7.0 | 370 |
| | 3-3 | H3 | Blue | 7.1 | 7.2 | 400 |
| | 3-4 | H4 | Blue | 7.1 | 7.0 | 400 |
| | 3-5 | H5 | Blue | 6.8 | 6.8 | 360 |
| | 3-6 | H6 | Blue | 7.1 | 7.0 | 400 |
| | 3-7 | H7 | Blue | 6.7 | 6.8 | 380 |
| | 3-8 | H8 | Blue | 7.0 | 7.2 | 400 |
| | 3-9 | H9 | Blue | 7.0 | 7.1 | 400 |
| | 3-10 | H10 | Blue | 6.9 | 6.8 | 370 |
| | 3-11 | 31 | Blue | 7.2 | 7.1 | 370 |
| | 3-12 | H1 | Blue | 6.9 | 6.6 | 360 |
| | 3-13 | H16 | Blue | 7.1 | 7.2 | 390 |
| Comparative Example | 3-1 | Comparative compound 1 | Blue | 6.2 | 8.0 | 250 |
| | 3-2 | Comparative compound 2 | Blue | 6.2 | 8.0 | 250 |
| | 3-3 | Comparative compound 3 | Blue | 5.9 | 7.9 | 260 |
| | 3-4 | Comparative compound 1 | Blue | 6.2 | 7.8 | 250 |
| | 3-5 | Comparative compound 1 | Blue | 6.3 | 7.6 | 260 |

### [Example 4-1 (production of organic EL device)]

A glass substrate with an ITO transparent electrode measuring 25 mm wide by 75 mm long by 1.1 mm thick (manufactured by GEOMATEC Co., Ltd.) was subjected to ultrasonic cleaning in isopropyl alcohol for 5 minutes. After that, the substrate was subjected to UV ozone cleaning for 30 minutes.
The glass substrate with the transparent electrode line after the cleaning was mounted on a substrate holder of a vacuum vapor deposition device. First, the electron-accepting compound (C-1) was deposited from vapor on the surface on the side where the transparent electrode line was formed so as to cover the transparent electrode. Thus, the C-1 film having a thickness of 5 nm was formed. The following aromatic amine derivative (X1) was deposited from vapor as a first hole transporting material and formed into a first hole transporting layer having a thickness of 50 nm on the C-1 film. Following the formation of the first hole transporting layer, the aromatic amine derivative (H1) obtained in Synthesis Embodiment 1 was deposited from vapor as a second hole transporting material and formed into a second hole transporting layer having a thickness of 60 nm.
Further, the following compound (EM2) was deposited from vapor and formed into a light emitting layer having a thickness of 45 nm on the second hole transporting layer. Simultaneously with this formation, the following compound (D3) was co-deposited as a phosphorescent material. The concentration of the compound D3 was 7.5 mass%. The co-deposited film functions as a light emitting layer.
Then, subsequent to the formation of the light emitting layer, the following compound (ET4) was formed into a film having a thickness of 30 nm. The ET1 film functions as an electron transporting layer.
Next, LiF was formed into a film having a thickness of 1 nm at a film formation rate of 0.1Å/min to serve as an electron injectable electrode (cathode). Metal Al was deposited from the vapor onto the LiF film so that a metal cathode having a thickness of 80 nm was formed. Thus, an organic EL device was produced.
The current efficiency of the resultant organic EL device when the device was driven with a DC constant current at an initial luminance of 2, 000 cd/m² and room temperature was measured. Table 4 shows the result.

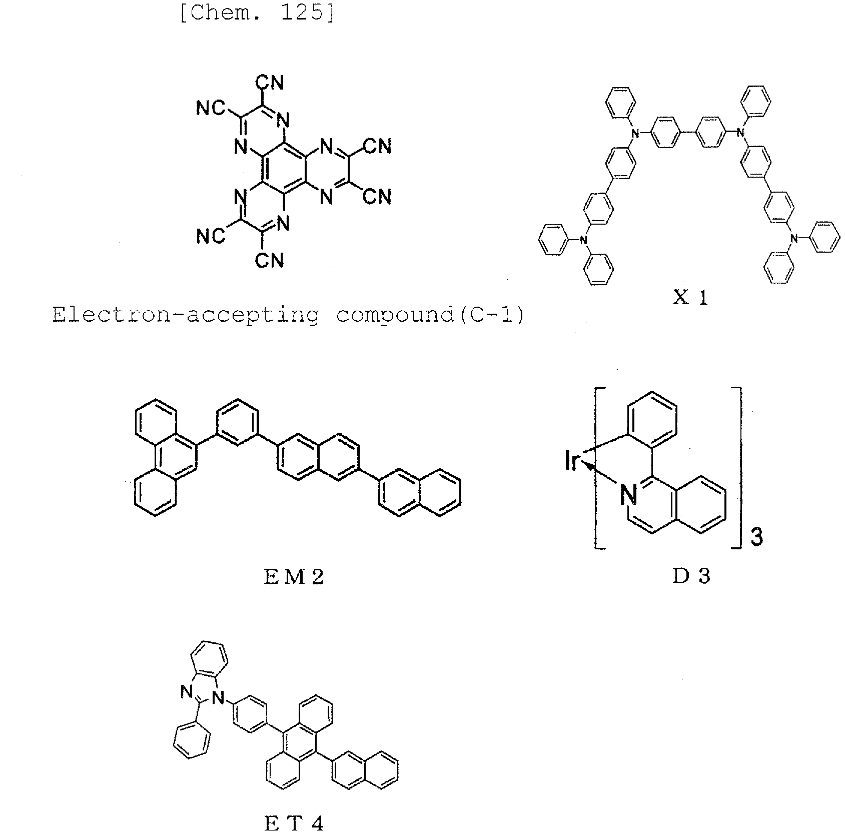

In Example 4-1, the Eg (T) of the aromatic amine derivative (H1) was measured as described below.

### Eg(T) (triplet energy gap):

The measurement was performed on the basis of a phosphorescence spectrum.
The material was dissolved in an EPA solvent (containing diethyl ether, isopentane, and ethanol at a volume ratio of 5:5:2) at 10 µ mol/L so that a sample for phosphorescence measurement was prepared. The sample for phosphorescence measurement was charged into a quartz cell, cooled to 77 K, and irradiated with excited light, and then the wavelength of phosphorescence to be emitted was measured.
A tangent was drawn to the rising edge of the resultant phosphorescence spectrum on shorter wavelengths, and then a value obtained by converting a wavelength value for the point of intersection of the tangent and a baseline into energy was defined as the excited triplet energy gap Eg(T).
It should be noted that a commercially available measuring apparatus F-4500 (manufactured by Hitachi, Ltd.) was used in the measurement.

In Example 4-1, the Tg of the aromatic amine derivative (H1) was measured as described below.
Used as the Tg was a value in second heating measured with a DSC "Pyris 1" manufactured by PerkinElmer under the following measurement conditions.

### [Measurement conditions]

(i) Heating from 30°C to the maximum temperature (10°C/min)
(ii) Holding at the maximum temperature for 3 minutes
(iii) Cooling from the maximum temperature to -50°C (200°C/min)
(iv) Holding at -50°C for 10 minutes
(v) Heating from -50°C to the maximum temperature (10°C/min)
The maximum temperature was set to the melting point in Tg-DTA plus about 30°C, and was modified in accordance with a decomposition temperature when the decomposition temperature was close to the maximum temperature.

### [Examples 4-2 to 4-4 (production of organic EL devices)]

Organic EL devices were each produced in the same manner as in Example 4-1 except that an aromatic amine derivative shown in Table 4 was used instead of the aromatic amine derivative (H1) as a second hole transporting material in Example 4-1. The current efficiencies of the resultant organic EL devices when the devices were each driven with a DC constant current at an initial luminance of 2,000 cd/m² and room temperature were measured. Table 4 shows the results.

### [Example 4-5 (production of organic EL device)]

An organic EL device was produced in the same manner as in Example 4-1 except that the phenanthroline derivative (ET2) was used instead of the compound (ET4)as an electron transporting material. The current efficiency of the resultant organic EL device when the device was driven with a DC constant current at an initial luminance of 2, 000 cd/m² and room temperature was measured. Table 4 shows the result.

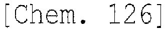

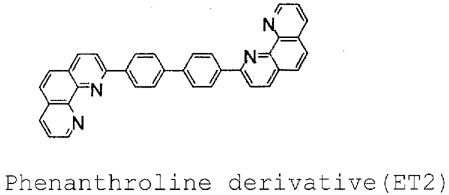

### [Example 4-6 (production of organic EL device)]

An organic EL device was produced in the same manner as in Example 4-1 except that the phosphine oxide derivative (ET3) was used instead of the compound (ET4) as an electron transporting material. The current efficiency of the resultant organic EL device when the device was driven with a DC constant current at an initial luminance of 2,000 cd/m² and room temperature was measured. Table 4 shows the result.

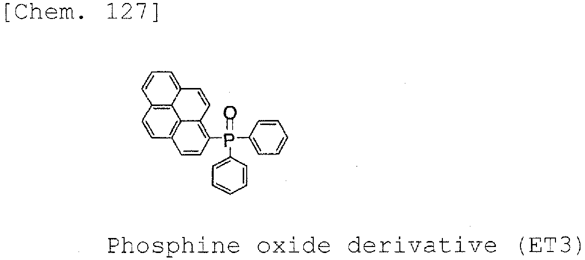

### [Example 4-7 (production of organic EL device)]

An organic EL device was produced in the same manner as in Example 4-1 except that the following aromatic amine derivative (X2) was used instead of the aromatic amine derivative (X1) as a first hole transporting material in Example 4-1. The current efficiency of the resultant organic EL device when the device was driven with a DC constant current at an initial luminance of 2, 000 cd/m² and room temperature was measured. Table 4 shows the result.

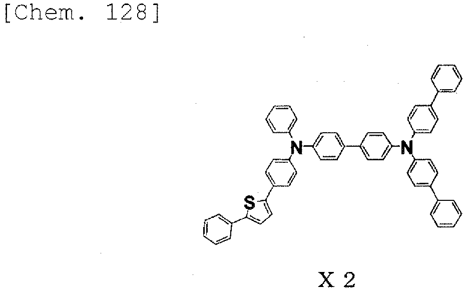

### [Examples 4-8 to 4-10 (production of organic EL devices) ]

Organic EL devices were each produced in the same manner as in Example 4-7 except that an aromatic amine derivative shown in Table 4 was used instead of the aromatic amine derivative (H1) as a second hole transporting material in Example 4-7. The current efficiencies of the resultant organic EL devices when the devices were each driven with a DC constant current at an initial luminance of 2,000 cd/m² and room temperature were measured. Table 4 shows the results.

### [Example 4-11 (production of organic EL device)]

An organic EL device was produced in the same manner as in Example 4-5 except that the aromatic amine derivative (X2) was used instead of the aromatic amine derivative (X1) as a first hole transporting material in Example 4-5. The current efficiency of the resultant organic EL device when the device was driven with a DC constant current at an initial luminance of 2,000 cd/m² and room temperature was measured. Table 4 shows the result.

### [Example 4-12 (production of organic EL device)]

An organic EL device was produced in the same manner as in Example 4-6 except that the aromatic amine derivative (X2) was used instead of the aromatic amine derivative (X1) as a first hole transporting material in Example 4-6. The current efficiency of the resultant organic EL device when the device was driven with a DC constant current at an initial luminance of 2,000 cd/m² and room temperature was measured. Table 4 shows the result.

### [Examples 4-13 and 4-14 (production of organic EL devices)]

Organic EL devices were each produced in the same manner as in Example 4-1 except that an aromatic amine derivative shown in Table 4 was used instead of the aromatic amine derivative (H1) as a second hole transporting material in Example 4-1. The current efficiencies of the resultant organic EL devices when the devices were each driven with a DC constant current at an initial luminance of 2,000 cd/m² and room temperature were measured. Table 4 shows the results.

### [Examples 4-15 and 4-16 (production of organic EL devices)]

Organic EL devices were each produced in the same manner as in Example 4-7 except that an aromatic amine derivative shown in Table 4 was used instead of the aromatic amine derivative (H1) as a second hole transporting material in Example 4-7. The current efficiencies of the resultant organic EL devices when the devices were each driven with a DC constant current at an initial luminance of 2,000 cd/m² and room temperature were measured. Table 4 shows the results.

**[Table 4]**

| | | First hole transporting layer | Second hole transporting layer | Current efficiency (cd/A) | Eg(T) (eV) | Tg (°C) |
|---|---|---|---|---|---|---|
| Example | 4-1 | X1 | H1 | 25.5 | 2.61 | 107 |
| | 4-2 | X1 | H3 | 24.8 | 2.59 | 127 |
| | 4-3 | X1 | H4 | 24.8 | 2.59 | 129 |
| | 4-4 | X1 | H6 | 24.5 | 2.59 | 145 |
| | 4-5 | X1 | H1 | 25.6 | 2.61 | 107 |
| | 4-6 | X1 | H1 | 25.5 | 2.61 | 107 |
| | 4-7 | X2 | H1 | 22.8 | 2.61 | 107 |
| | 4-8 | X2 | H3 | 22.5 | 2.59 | 127 |
| | 4-9 | X2 | H4 | 22.4 | 2.59 | 129 |
| | 4-10 | X2 | H6 | 22.6 | 2.59 | 145 |
| | 4-11 | X2 | H1 | 22.9 | 2.61 | 107 |
| | 4-12 | X2 | H1 | 22.8 | 2.61 | 107 |
| | 4-13 | X1 | H2 | 22.2 | 2.56 | 109 |
| | 4-14 | X1 | H7 | 22.3 | 2.54 | 146 |
| | 4-15 | X2 | H2 | 20.5 | 2.56 | 109 |
| | 4-16 | X2 | H7 | 20.0 | 2.54 | 146 |

### Industrial Applicability

As specifically described above, an organic EL device using the aromatic amine derivative of the present invention has a high luminous efficiency and hardly deteriorates even when used for a long time period, in other words, has a long lifetime. Accordingly, the organic EL device is useful for flat luminous bodies of a wall television and light sources for the backlights of a display.

## Claims

1. An aromatic amine derivative represented by the following formula (1) :
A-L-B (1)
in the formula (1), L is represented by the following formula (2): 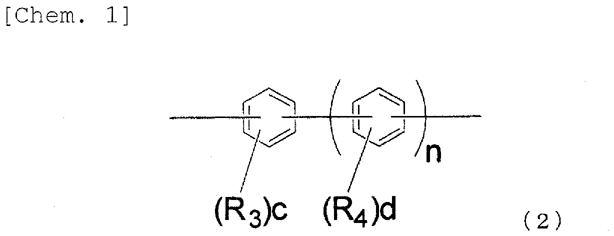 in the formula (2):
n represents an integer of 0 to 3;
R₃ and R₄ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group;
a plurality of R₃'s or R₄'s adjacent or close to each other may be bonded to each other to form a saturated or unsaturated, divalent group that forms a ring;
R₃ and R₄ adjacent to each other may be bonded to each other so that L forms a substituted or unsubstituted fluorenylene group; and
c and d each independently represent an integer of 0 to 4,
in the formula (1), A is represented by the following formula (3) : 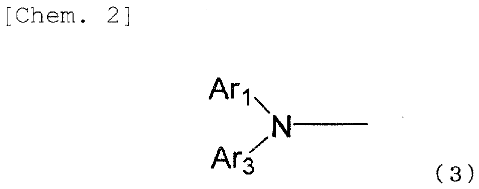 in the formula (3), Ar₁ represents a substituted or unsubstituted aryl group having 6 to 25 ring carbon atoms, or a substituted or unsubstituted heteroaryl group having 5 to 25 ring atoms, and Ar₃ is represented by the following formula (4): 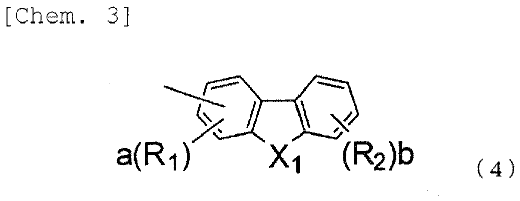 in the formula (4):
X₁ represents O (oxygen atom), S (sulfur atom), NRa, or CRbRc, Ra represents a group selected from the group consisting of an aryl group having 6 to 25 ring carbon atoms and a heteroaryl group having 5 to 25 ring atoms, and Rb or Rc each independently represent a group selected from the group consisting of an aryl group having 6 to 25 ring carbon atoms and a heteroaryl group having 5 to 25 ring atoms;
R₁ and R₂ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group, and a plurality of R₁'s or R₂'s adjacent to each other, or R₁ and R₂ may be bonded to each other to form a saturated or unsaturated, divalent group that forms a ring;
a represents an integer of 0 to 3; and
b represents an integer of 0 to 4, and
in the formula (1), B is represented by the following formula (5) : 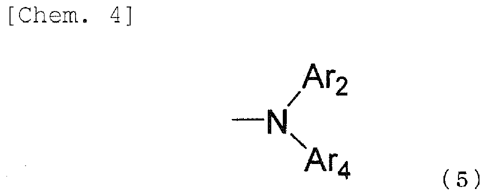 in the formula (5), Ar₂ or Ar₄ represents a substituted or unsubstituted aryl group having 6 to 25 ring carbon atoms, or a substituted or unsubstituted heteroaryl group having 5 to 25 ring atoms.

2. The aromatic amine derivative according to claim 1, wherein the Ar₃ is represented by any one of the following formulae (6) to (8) : 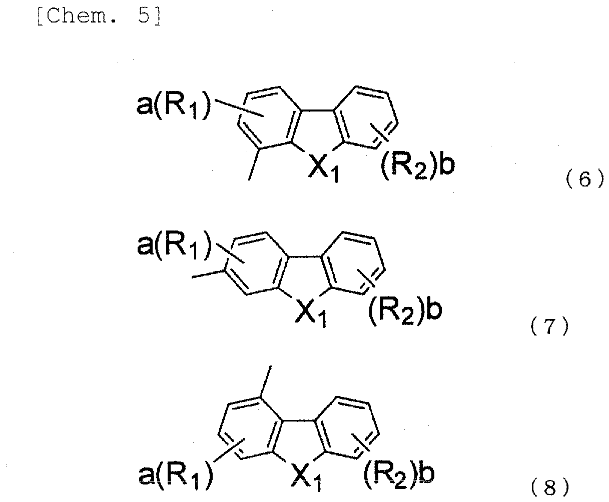 in the formulae (6) to (8):
X₁ represents O (oxygen atom), S (sulfur atom), NRa, or CRbRc, Ra represents a group selected from the group consisting of an aryl group having 6 to 25 ring carbon atoms and a heteroaryl group having 5 to 25 ring atoms, and Rb or Rc each independently represent a group selected from the group consisting of an aryl group having 6 to 25 ring carbon atoms and a heteroaryl group having 5 to 25 ring atoms;
R₁ and R₂ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group, and a plurality of R₁'s or R₂'s adjacent to each other, or R₁ and R₂ may be bonded to each other to form a saturated or unsaturated, divalent group that forms a ring;
a's each independently represent an integer of 0 to 3; and
b's each independently represent an integer of 0 to 4.

3. The aromatic amine derivative according to claim 1, wherein the X₁ represents O (oxygen atom) or S (sulfur atom).

4. The aromatic amine derivative according to claim 1, wherein the X₁ represents O (oxygen atom).

5. The aromatic amine derivative according to claim 1, wherein the Ar₃ is represented by the formula (6) or (8).

6. The aromatic amine derivative according to claim 1, wherein the Ar₃ is represented by the formula (6).

7. The aromatic amine derivative according to claim 4, wherein the Ar₃ is represented by the formula (6).

8. The aromatic amine derivative according to any one of claims 1 to 4, wherein the Ar₁ or the Ar₄ is represented by the following formula (9): 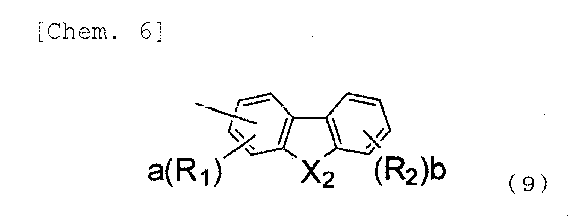 in the formula (9):
X₂ represents O (oxygen atom), S (sulfur atom), NRa, or CRbRc, Ra represents a group selected from the group consisting of an aryl group having 6 to 25 ring carbon atoms and a heteroaryl group having 5 to 25 ring atoms, and Rb or Rc each independently represent a group selected from the group consisting of an aryl group having 6 to 25 ring carbon atoms and a heteroaryl group having 5 to 25 ring atoms;
R₁ and R₂ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group, and a plurality of R₁'s or R₂'s adjacent to each other, or R₁ and R₂ may be bonded to each other to form a saturated or unsaturated, divalent group that forms a ring;
a represents an integer of 0 to 3; and
b represents an integer of 0 to 4.

9. The aromatic amine derivative according to claim 1, wherein the Ar₁ or the Ar₄ is represented by any one of the formulae (6) to (8).

10. The aromatic amine derivative according to claim 1, wherein any one of the Ar₁, the Ar₂, and the Ar₄ is represented by the following formula (10): 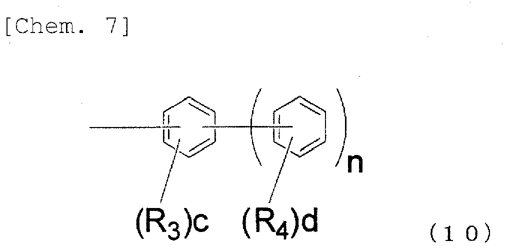 in the formula (10):
n represents an integer of 0 to 3;
R₃ and R₄ each independently represent a group selected from the group consisting of a linear or branched alkyl group having 1 to 15 carbon atoms, a linear or branched alkenyl group having 2 to 15 carbon atoms, a cycloalkyl group having 3 to 15 carbon atoms, a trialkylsilyl group having alkyl groups each having 1 to 15 carbon atoms, a triarylsilyl group having aryl groups each having 6 to 25 ring carbon atoms, an alkylarylsilyl group having an alkyl group having 1 to 15 carbon atoms and an aryl group having 6 to 25 ring carbon atoms, an aryl group having 6 to 25 ring carbon atoms, a heteroaryl group having 5 to 25 ring atoms, a halogen atom, and a cyano group;
a plurality of R₃'s or R₄'s adjacent or close to each other may be bonded to each other to form a saturated or unsaturated, divalent group that forms a ring;
R₃ and R₄ adjacent to each other may be bonded to each other so that L forms a substituted or unsubstituted fluorenylene group; and
c and d each independently represent an integer of 0 to 4.

11. The aromatic amine derivative according to claim 1, wherein any one of the Ar₁, the Ar₂, and the Ar₄ represents a phenyl group, a biphenyl group, or an m-terphenyl group.

12. The aromatic amine derivative according to claim 1, wherein A and B are identical to each other.

13. The aromatic amine derivative according to claim 1, wherein A and B are different from each other.

14. An organic electroluminescence device, comprising an organic thin-film layer formed of one or more layers including at least a light emitting layer, the organic thin-film layer being interposed between a cathode and an anode, wherein at least one layer of the organic thin-film layer contains the aromatic amine derivative according to any one of claims 1 to 13 alone or as a component of a mixture.

15. The organic electroluminescence device according to claim 14, comprising at least a hole transporting layer and/or a hole injecting layer as the organic thin-film layer, wherein the aromatic amine derivative is incorporated into the hole transporting layer and/or the hole injecting layer.

16. The organic electroluminescence device according to claim 14, wherein a layer containing an electron-accepting compound is joined to the layer containing the aromatic amine derivative.

17. The organic electroluminescence device according to claim 16, wherein the electron-accepting compound is represented by the following formula (A): 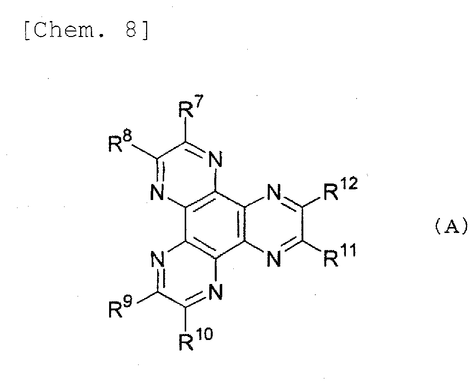 in the formula (A), R⁷ to R¹² each independently represent a cyano group, -CONH₂, a carboxyl group, or -COOR¹³ where R¹³ represents an alkyl group having 1 to 20 carbon atoms, or R⁷ and R⁸, R⁹ and R¹⁰, or R¹¹ and R¹² combine with each other to represent a group represented by -CO-O-CO-.

18. The organic electroluminescence device according to claim 16, wherein the electron-accepting compound is represented by the following formula (B): 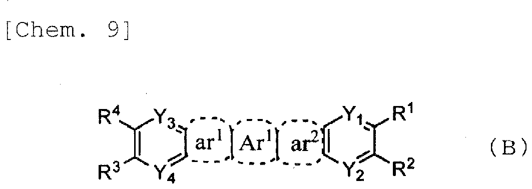 in the formula (B):
Ar¹ represents a fused ring having 6 to 24 ring carbon atoms or a heterocycle having 6 to 24 ring atoms, and ar¹ and ar² may be identical to or different from each other, and are each represented by the following formula (i) or (ii): 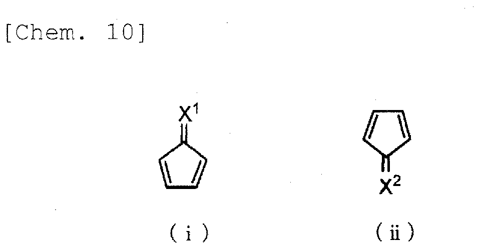
in the formulae, X¹ and X² may be identical to or different from each other, and each represent any one of divalent groups represented by the following formulae (a) to (g): 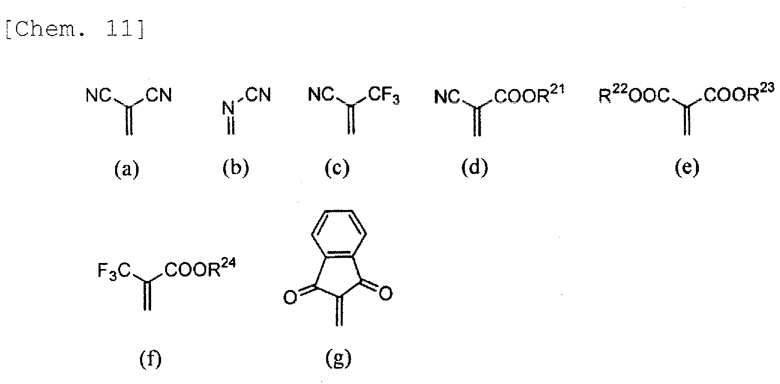 in the formulae, R²¹ to R²⁴ may be identical to or different from one another, and each represent a hydrogen atom, a substituted or unsubstituted fluoroalkyl group having 1 to 20 carbon atoms, a substituted or unsubstituted alkyl group having 1 to 20 carbon atoms, a substituted or unsubstituted aryl group having 6 to 50 carbon atoms, or a substituted or unsubstituted heterocyclic group having 3 to 50 ring atoms, and R²² and R²³ may be bonded to each other to form a ring; and
R¹ to R⁴ in the formula (B) may be identical to or different from one another, and each represent a hydrogen atom, a substituted or unsubstituted alkyl group having 1 to 20 carbon atoms, a substituted or unsubstituted aryl group having 6 to 50 carbon atoms, a substituted or unsubstituted heterocyclic group having 3 to 50 ring atoms, a halogen atom, a substituted or unsubstituted fluoroalkyl group having 1 to 20 carbon atoms, a substituted or unsubstituted alkoxy group having 1 to 20 carbon atoms, a substituted or unsubstituted fluoroalkoxy group having 1 to 20 carbon atoms, a substituted or unsubstituted aryloxy group having 6 to 50 carbon atoms, or a cyano group, substituents adjacent to each other out of R¹ to R⁴ may be bonded to each other to form a ring, Y¹ to Y⁴ may be identical to or different from one another, and each represent -N=, -CH=, or C(R⁵) =, and R⁵ represents a substituted or unsubstituted alkyl group having 1 to 20 carbon atoms, a substituted or unsubstituted aryl group having 6 to 50 carbon atoms, a substituted or unsubstituted heterocyclic group having 3 to 50 ring atoms, a halogen atom, a substituted or unsubstituted fluoroalkyl group having 1 to 20 carbon atoms, a substituted or unsubstituted alkoxy group having 1 to 20 carbon atoms, a substituted or unsubstituted fluoroalkoxy group having 1 to 20 carbon atoms, a substituted or unsubstituted aryloxy group having 6 to 50 carbon atoms, or a cyano group.

19. The organic electroluminescence device according to claim 14, wherein the light emitting layer containing a host material and a dopant material that shows light emission is joined to the layer containing the aromatic amine derivative.

20. The organic electroluminescence device according to claim 16, wherein the light emitting layer containing a host material and a dopant material that shows light emission is joined to the layer containing the aromatic amine derivative on a surface opposite to the layer containing the electron-accepting compound.

21. The organic electroluminescence device according to claim 19, wherein the dopant material comprises a metal complex compound containing a metal selected from Ir, Pt, Os, Cu, Ru, Re, and Au.

22. The organic electroluminescence device according to claim 19, wherein the dopant material is formed of a metal complex compound having a partial structure represented by the following formula (21) or (22), or a tautomer thereof: 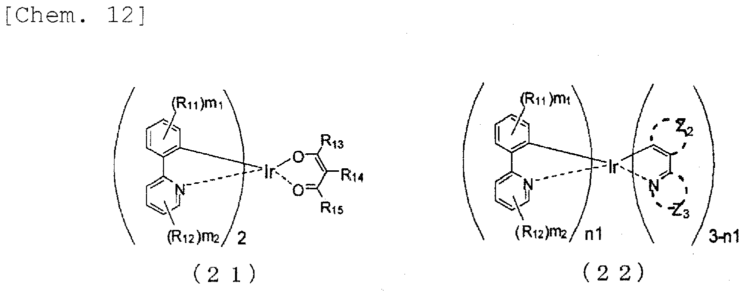 in the formulae:
R₁₁ and R₁₂ each independently represent an alkyl group having 1 to 20 carbon atoms, an alkenyl group having 1 to 20 carbon atoms, an aryl group having 6 to 25 ring carbon atoms, a heteroaryl group having 5 to 25 ring atoms, a halogen atom, or a cyano group, and a plurality of R₁₁'s or R₁₂'s adjacent to each other may each independently form a saturated or unsaturated, divalent group;
R₁₃ to R₁₅ each independently represent a hydrogen atom or an alkyl group having 1 to 20 carbon atoms;
Z₂ represents an atomic group that forms an aryl ring having 6 to 25 ring carbon atoms or a heteroaryl ring having 5 to 25 ring atoms, and Z₃ represents an atomic group that forms a nitrogen-containing heteroaryl ring having 5 to 25 ring atoms;
m₁ and m₂ each independently represent an integer of 0 to 4; and
n₁ represents an integer of 1 to 3.

23. The organic electroluminescence device according to claim 19, wherein the dopant material is formed of a metal complex compound having a partial structure represented by the following formula (23) or (24), or a tautomer thereof: 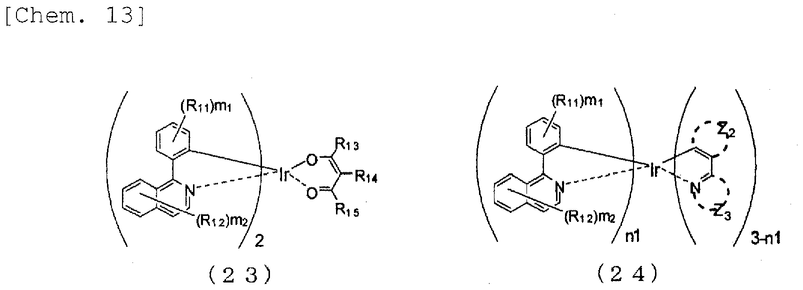 in the formulae (23) and (24), R₁₁ to R₁₅, Z₂, Z₃, m₁, m₂, and n₁ each have the same meaning as that used in each of the formulae (21) and (22).

24. The organic electroluminescence device according to claim 19, wherein the dopant material is formed of a metal complex compound having a partial structure represented by the following formula (25) or (26), or a tautomer thereof: 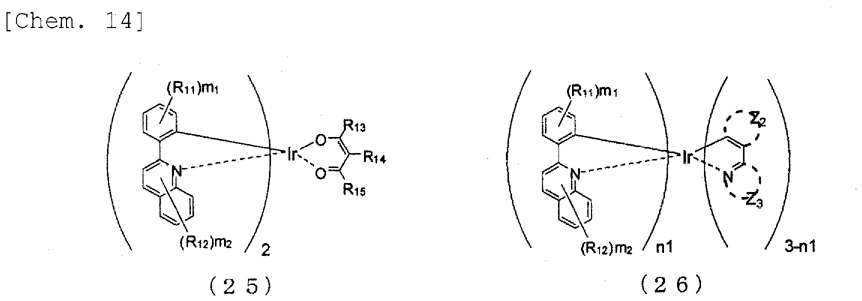 in the formulae (25) and (26), R₁₁ to R₁₅, Z₂, Z₃, m₁, m₂, and n₁ each have the same meaning as that used in each of the formulae (21) and (22).

25. The organic electroluminescence device according to claim 19, wherein the dopant material is formed of a metal complex compound having a partial structure represented by the following formula (27) or (28), or a tautomer thereof: 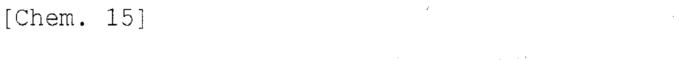 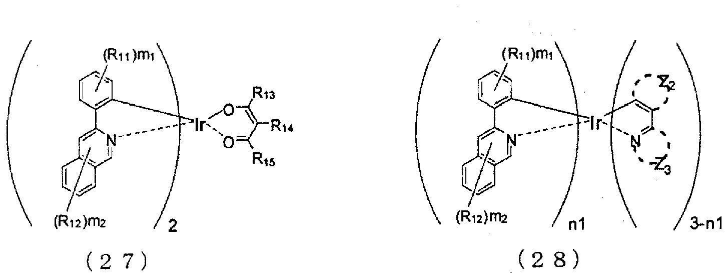 in the formulae (27) and (28), R₁₁ to R₁₅, Z₂, Z₃, m₁, m₂, and n₁ each have the same meaning as that used in each of the formulae (21) and (22).

26. The organic electroluminescence device according to claim 19, wherein the dopant material is formed of a metal complex compound having a partial structure represented by the following formula (29) or a tautomer thereof: 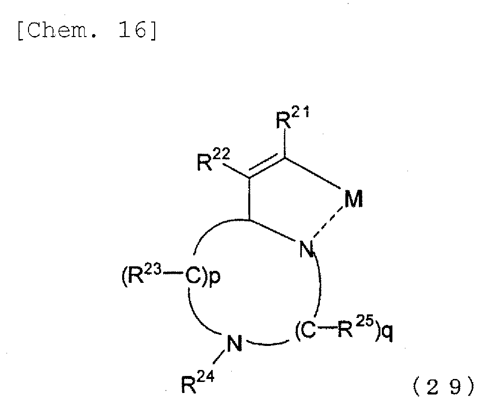 in the formula:
R²¹ to R²⁵ each independently represent a hydrogen atom, a cyano group, a nitro group, a halogen atom, a substituted or unsubstituted alkyl group having 1 to 20 carbon atoms, a substituted or unsubstituted amino group, a substituted or unsubstituted alkoxyl group having 1 to 20 carbon atoms, a substituted or unsubstituted alkylsilyl group having 1 to 20 carbon atoms, a substituted or unsubstituted acyl group having 1 to 20 carbon atoms, or a substituted or unsubstituted aromatic group having 1 to 30 carbon atoms, and R²¹ and R²², R²³ and R²⁴, or R²⁴ and R²⁵ may be bonded to each other to form a ring structure;
p and q each represent an integer of 0 to 3, p+q represents 2 or 3, when p represents an integer of 2 or more, a plurality of R²³'s may be bonded to each other to form a ring structure, and when q represents an integer of 2 or more, a plurality of R²⁵'s may be bonded to each other to form a ring structure; and
M represents a metal atom selected from iridium (Ir), rhodium (Rh), platinum (Pt), and palladium (Pd).

27. The organic electroluminescence device according to claim 19, wherein the host material comprises a compound having a substituted or unsubstituted, polycyclic, fused aromatic skeleton portion.

28. The organic electroluminescence device according to claim 27, wherein the polycyclic, fused aromatic skeleton portion of the compound having the polycyclic, fused aromatic skeleton portion is selected from the group consisting of substituted or unsubstituted phenanthrenediyl, chrysenediyl, fluoranthenediyl, and triphenylenediyl groups.

29. The organic electroluminescence device according to claim 27, wherein the polycyclic, fused aromatic skeleton portion is represented by any one of the following formulae (12) to (15): 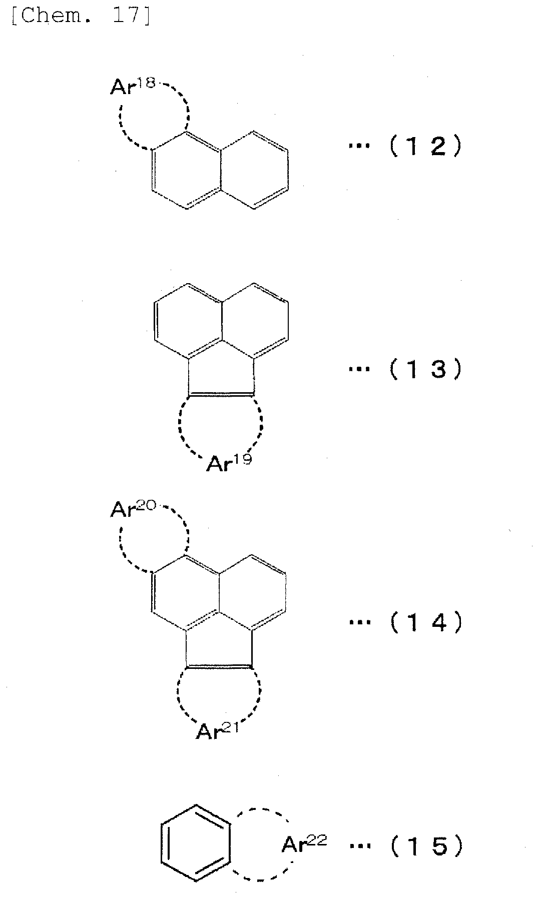 in the formulae, Ar¹⁸ to Ar²² each represent a substituted or unsubstituted, fused ring structure having 4 to 10 ring carbon atoms.

30. The organic electroluminescence device according to claim 27, wherein the polycyclic, fused aromatic skeleton portion of the compound having the polycyclic, fused aromatic skeleton portion is substituted with a group having phenanthrene, chrysene, fluoranthene, or triphenylene.

31. The organic electroluminescence device according to claim 14, wherein:
the organic electroluminescence device has, as the organic thin-film layer, an electron transporting layer and/or an electron injecting layer provided on a side closer to the cathode than the light emitting layer; and
a nitrogen-containing heterocyclic derivative represented by any one of the following formulae (31) to (33) is incorporated into the electron transporting layer and/or the electron injecting layer: 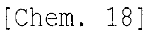 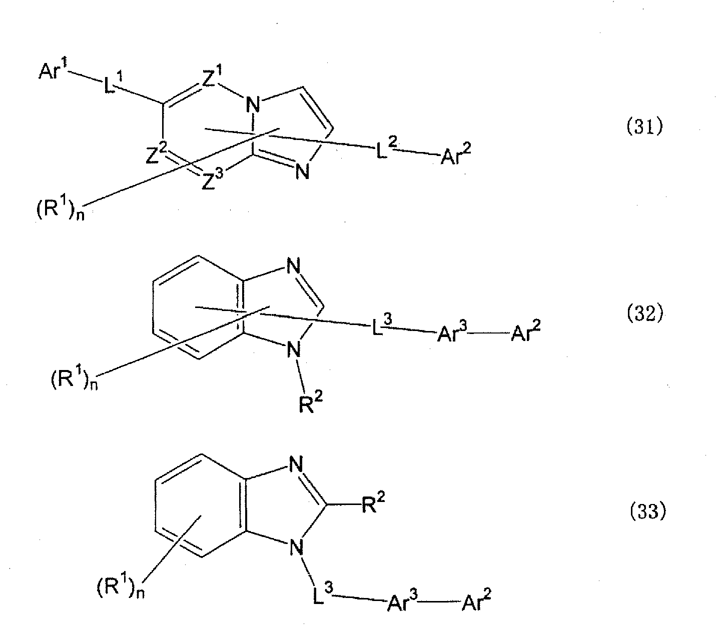
in the formulae (31) to (33):
Z¹, Z², and Z³ each independently represent a nitrogen atom or a carbon atom;
R¹ and R² each independently represent a substituted or unsubstituted aryl group having 6 to 50 carbon atoms, a substituted or unsubstituted heteroaryl group having 3 to 50 carbon atoms, an alkyl group having 1 to 20 carbon atoms, an alkyl group having 1 to 20 carbon atoms and substituted with a halogen atom, or an alkoxy group having 1 to 20 carbon atoms;
n represents an integer of 0 to 5, and when n represents an integer of 2 or more, a plurality of R¹'s may be identical to or different from each other, and a plurality of R¹'s adjacent to each other may be bonded to each other to form a substituted or unsubstituted aromatic hydrocarbon ring;
Ar¹ represents a substituted or unsubstituted aryl group having 6 to 50 carbon atoms, or a substituted or unsubstituted heteroaryl group having 3 to 50 carbon atoms;
Ar² represents a hydrogen atom, an alkyl group having 1 to 20 carbon atoms, an alkyl group having 1 to 20 carbon atoms and substituted with a halogen atom, an alkoxy group having 1 to 20 carbon atoms, a substituted or unsubstituted aryl group having 6 to 50 carbon atoms, or a substituted or unsubstituted heteroaryl group having 3 to 50 carbon atoms,
provided that one of Ar¹ and Ar² represents a substituted or unsubstituted fused ring group having 10 to 50 carbon atoms, or a substituted or unsubstituted heterofused ring group having 9 to 50 ring atoms;
Ar³ represents a substituted or unsubstituted arylene group having 6 to 50 carbon atoms, or a substituted or unsubstituted heteroarylene group having 3 to 50 carbon atoms; and
L¹, L², and L³ each independently represent a single bond, a substituted or unsubstituted arylene group having 6 to 50 carbon atoms, a substituted or unsubstituted heterofused ring group having 9 to 50 ring atoms, or a substituted or unsubstituted fluorenylene group.

32. The organic electroluminescence device according to claim 14, wherein:
the organic electroluminescence device has, as the organic thin-film layer, an electron transporting layer and/or an electron injecting layer provided on a side closer to the cathode than the light emitting layer; and
a compound represented by any one of the following formulae (34) and (35) is incorporated into the electron transporting layer and/or the electron injecting layer: 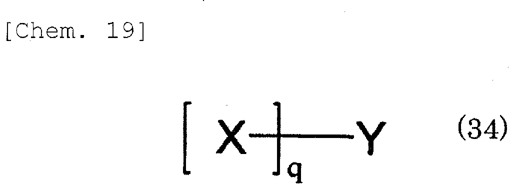 in the formula (34), X represents a fused ring containing a nitrogen atom or a sulfur atom, one, or a combination of two or more, of a single bond, an alkyl chain, an alkylene chain, a cycloalkyl chain, an aryl chain, a heterocyclic chain, a silyl chain, an ether chain, and a thioether chain is selected as Y, and q represents a natural number of 2 or more, and
the compound represented by the formula (34) has a molecular weight of 480 or more; 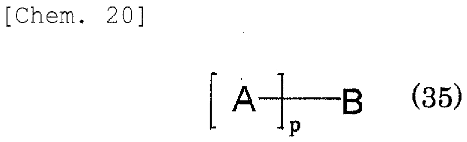 in the formula (35), A represents a substituent having a phenanthroline skeleton or a benzoquinoline skeleton, B represents a p-valent organic group having a structure represented by the following formula (35A), and p represents a natural number of 2 or more: 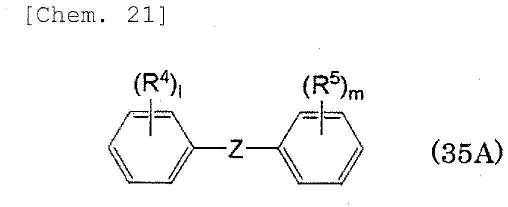 in the formula (35A)_{;} R⁴ and R⁵ each independently represent any one of an alkyl group and an aryl group including an aryl group that fuses with a phenyl group, 1 and m each independently represent a natural number of 0 to 5, and Z represents at least one kind selected from the following formulae (35B).

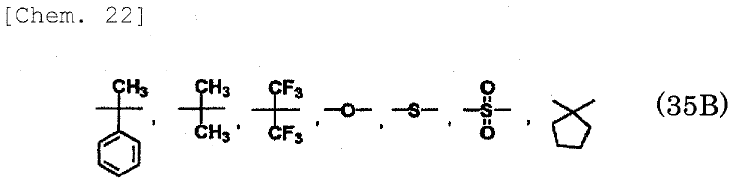

33. The organic electroluminescence device according to claim 14, wherein:
the organic electroluminescence device has, as the organic thin-film layer, an electron transporting layer and/or an electron injecting layer provided on a side closer to the cathode than the light emitting layer; and
compound represented by the following formula (36) is incorporated into the electron transporting layer and/or the electron injecting layer: 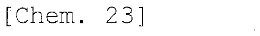 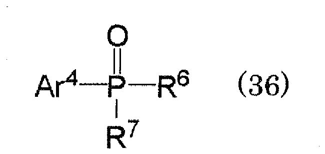
in the formula (36) , R⁶ and R⁷ may be identical to or different from each other, and each selected from a hydrogen atom, an alkyl group, a cycloalkyl group, a heterocyclic group, an alkenyl group, a cycloalkenyl group, an alkynyl group, an alkoxy group, an alkylthio group, an arylether group, an arylthioether group, an aryl group, a heteroaryl group, a cyano group, a carbonyl group, an ester group, a carbamoyl group, an amino group, a silyl group, and a fused ring formed with an adj acent substituent, and Ar⁴ represents an aryl group or a heteroaryl group.
